(19) Europäisches Patentamt
European Patent Office
Office européen des brevets

(11) EP 2 045 245 B1

(12) **EUROPEAN PATENT SPECIFICATION**

(45) Date of publication and mention of the grant of the patent:
**23.10.2013 Bulletin 2013/43**

(21) Application number: **07721493.0**

(22) Date of filing: **19.06.2007**

(51) Int Cl.:
***C07D 239/94*** (2006.01) ***C07D 215/54*** (2006.01)
***A61K 31/517*** (2006.01) ***A61K 31/4706*** (2006.01)
***A61P 35/00*** (2006.01) ***A61P 17/06*** (2006.01)
***A61P 11/00*** (2006.01) ***A61P 1/16*** (2006.01)
***A61P 13/12*** (2006.01) ***A61P 1/18*** (2006.01)
***A61P 3/10*** (2006.01) ***C07D 401/12*** (2006.01)
***C07D 405/12*** (2006.01) ***C07D 409/12*** (2006.01)

(86) International application number:
**PCT/CN2007/001920**

(87) International publication number:
**WO 2008/006287 (17.01.2008 Gazette 2008/03)**

(54) **INREVERSIBLE PROTEIN TYROSINE KINASES INHIBITORS AND THE PREPARATION METHODS AND USES THEREOF**

IRREVERSIBLE PROTEINTYROSINKINASEINHIBITOREN UND VERFAHREN ZUR HERSTELLUNG UND ANWENDUNG DAVON

INHIBITEURS DES TYROSINE KYNASES IRRÉVERSIBLES ET LEURS PROCÉDÉS DE PRÉPARATION ET UTILISATIONS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **05.07.2006 CN 200610014690**
**10.11.2006 CN 200610138377**

(43) Date of publication of application:
**08.04.2009 Bulletin 2009/15**

(73) Proprietor: **Tian Jin Hemay Bio-Tech Co., Ltd.**
**Tianjin 300457 (CN)**

(72) Inventor: **ZHANG, Hesheng**
**Tianjin 300457 (CN)**

(74) Representative: **Hryszkiewicz, Danuta**
**Kancelaria Patentowa**
**Ul. Jana z Kolna 38**
**75-204 Koszalin (PL)**

(56) References cited:
**EP-A- 0 787 722** **EP-A1- 0 787 722**
**WO-A1-00/51991** **US-B1- 6 344 459**

- **LAIRD A D ET AL: "SMALL MOLECULE TYROSINE KINASE INHIBITORS: CLINICAL DEVELOPMENT OF ANTICANCER AGENTS" EXPERT OPINION ON INVESTIGATIONAL DRUGS, ASHLEY PUBLICATIONS LTD., LONDON, GB, vol. 12, no. 1, 1 January 2003 (2003-01-01), pages 51-64, XP009032480 ISSN: 1354-3784**
- **TSOU H-R ET AL: "6-SUBSTITUTED-4-(3-BROMOPHENYLAMINO)QUINA ZOLINES AS PUTATIVE IRREVERSIBLE INHIBITORS OF THE EPIDERMAL GROWTH FACTOR RECEPTOR (EGFR) AND HUMAN EPIDERMAL GROWTH FACTOR RECEPTOR (HER-2) TYROSINE KINASES WITH ENHANCED ANTITUMOR ACTIVITY" JOURNAL OF MEDICINAL CHEMISTRY, AMERICAN CHEMICAL SOCIETY, WASHINGTON, US, vol. 44, no. 17, 1 January 2001 (2001-01-01), pages 2719-2734, XP001026039 ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 045 245 B1

## Description

The invention relates to quinazoline or quinoline derivatives, a method of their preparation, and a method of using the same as pharmaceutical agents.

As a cell surface receptor, protein tyrosine kinase (PTK) can transmit signals into cells. However, due to mutation, PTK can be activated even without ligand binding. Subsequently, false signals of cell growth and reproduction are transferred. Additionally, an overexpression of PTK in cells can cause a certain weak signal to be amplified inappropriately. Furthermore, during many steps of cellular signal transfer chain, the occurrence of mutation or overexpression of PTK can cause false signals. These false signals further make cell reproduction no more regulated effectively, and may cause cell carcinogenesis.

EGFR is one of the typical examples. EGFR belongs to cell surface receptors of epidermal growth factor receptor tyrosine kinase (EGFR-TK). The receptor family comprises EGF receptor (a protein product of oncogene erbB-1), erbB-2 (Neu or HER2) receptor, tumor protein mutant erbB-3 receptor and erbB-4 receptor. EGF and transforming growth factor alpha (TGF$\alpha$) are the two most important ligands of EGFR. Though the receptor plays a minor role in healthy adults, it is closely related to the pathological process of most cancers, particularly to colon cancer and breast cancer. Therefore, an EGFR-TK inhibitor can block the transfer of these receptor signals, and be used to treat cancers caused by EGFR overexpression, such as colorectal cancer, breast cancer, kidney cancer, lung cancer and head and neck cancer.

Furthermore, an EGFR- TK inhibitor can be used to treat other diseases caused by EGFR overexpression, such as psoriasis, nephritis and pancreatitis which are described below.

Nowadays, there is no effective way to treat proliferative skin diseases such as psoriasis. Conventionally, anti-cancer medicine such as methotrexate is used, however, the medicine has strong side effects and response is poor within the necessary limited dosage. In psoriasis tissues, TGF$\alpha$ is the main growth factor that is overexpressed. In animal experiments, 50% of transgenic mice with TGF$\alpha$ overexpression produce psoriasis, which suggests a good inhibitor of EGFR signal transfer mechanism may inhibit psoriasis, i.e., the EGFR- TK inhibitor can relieve psoriasis symptoms.

EGF is an effective mitogen of renal tubular cells. In streptozotocin-induced diabetic mice, the secretion of urine and mRNA of EGF are increased fourfold. Additionally, the expression of EGFR is enhanced in patients with proliferative glomerulonephritis (Roychaudhury et al Pathology). These findings indicate blocking EGF singal transfer can be used to treat and prevent renal injury. Therefore, it is postulated that an EGFR-TK inhibitor can be used to treat proliferative glomerulonephritis and renal disease induced by diabetes.

It has been reported that in chronic pancreatitis patients the expression of EGFR and TGF$\alpha$ is much higher than in healthy adults (Korc et al Gut 1994, 35, 1468). In patients with severe chronic pancreatitis, the overexpression of erbB-2 receptor has been confirmed (Friess et al Ann.Surg. 1994, 220, 183). Therefore, it is postulated that an EGFR-TK inhibitor could potentially be used to treat pancreatitis.

During the process of embryonic cell maturation, embryonic cell implantation in endometrium and other peripheral implantation, EGF and TGF$\alpha$ are synthesized in uterine tissues (Taga Nippon Sanka Fujinka Gakkai Zasshi 1992, 44, 939) and EGFR has increased (Brown et al Endocrinology 1989, 124, 2882). Meanwhile, heparin- binding EGF is induced due to the approach of embryonic cells which are developing but not yet caputred (Das et al Development 1994, 120, 1071). The expression of TGF$\alpha$ and EGFR have a high level in embryonic cells (Adamson, Mol. reprod. Dev. 1990, 27, 16). Surgically removing submandibular gland, a main organ of internal secretion, and treating with monoclonal antibody against EGFR can greatly reduce the fertility of mice by decreasing the success of embryonic cell implantation (Tsutsumi et al J.Endocrinlogy 1993, 138, 437). These results indicate that an EGFR-TK inhibitor may function as a contraceptive.

WO 92/078844 dated on May 14, 1992 and WO 92/14716 dated on Sep. 03, 1992 disclose 2,4-diamino quinazoline which are used as a potentiators of chemotheropeutic agents in cancer treatment.

WO 92/20642 dated on Nov. 26, 1992 discloses bis mono- and/or bicyclic aryl and/or heteroaryl compounds which can inhibit EGF and/or PDGF receptor tyrosine kinase.

European Patent 520722A1, 566226A1, 635498A1, 602851A1, WO 95/19774 and WO 95/15758 relate to reversible EGF receptor tyrosine kinase inhibitors. These inhibitors belong to 4-anilino quinazoline derivatives and some exhibit a high inhibitory activity against EGF receptor tyrosine kinase, but in animal pathological model these inhibitors exhibit a low activity. The reason for this lies in that PTK is a catalyst catalyzing a phosphate group to transfer from ATP to a protein tyrosine residue, and the above-mentioned reversible EGF receptor tyrosine kinase inhibitors compete with ATP to bind EGF receptor tyrosine kinase, but in cells the ATP concentration is much higher (mM grade), thus the reversible EGF receptor tyrosine kinase inhibitors exhibiting a high activity in vitro have difficulty in exhibiting a high effect in animal pathological models. However, irreversible EGF receptor tyrosine kinase inhibitors do not compete with ATP, so they are expected to do better in vivo.

US Patent 5,760,041 and European Patent EP 0787722 disclose a novel irreversible EGF receptor tyrosine kinase inhibitor. In the inhibitor, a Michael acceptor is introduced to the sixth position of quinazoline so that a Michael

addition reaction can occur between the inhibitor and cysteine sulphydryl in the active center pocket wall of EGF receptor tyrosine kinase. Furthermore, the activity of the inhibitor has a positive correlation with the activation energy of the Michael addition reaction between the inhibitor and cysteine sulphydryl. Therefore, both American Wyeth Pharmaceutical Co., Ltd. and Pfizer Pharmaceuticals Co. Ltd. choose to develop high reactivity-high activity irreversible EGF receptor tyrosine kinase inhibitors.

The present invention discloses an irreversible EGF receptor tyrosine kinase inhibitor featuring a unique chemical structure and low reactivity-high inhibitory activity against EGF receptor tyrosine kinase. Reseaches have found that the compound exhibits a good inhibitory activity against A431 cell self-phosphorylation stimulated by EGF, and exhibits a certain growth inhibitory activity against the cell strain, have a good anti-tumor effect in A431 tumor animal pathological model.

In view of the above-described problems, it is an objective of the invention to provide a compound or pharmaceutically acceptable salts or hydrates thereof which can inhibit protein tyrosine kinase activity.

It is another objective of the invention to provide a preparation at least comprising a compound or pharmaceutically acceptable salts or hydrates thereof which can inhibit protein tyrosine kinase activity.

It is still another objective of the invention to provide a method of preparing a compound which can inhibit protein tyrosine kinase activity.

To achieve the above objectives, in accordance with one embodiment of the invention, provided is a compound of formula (**I**),

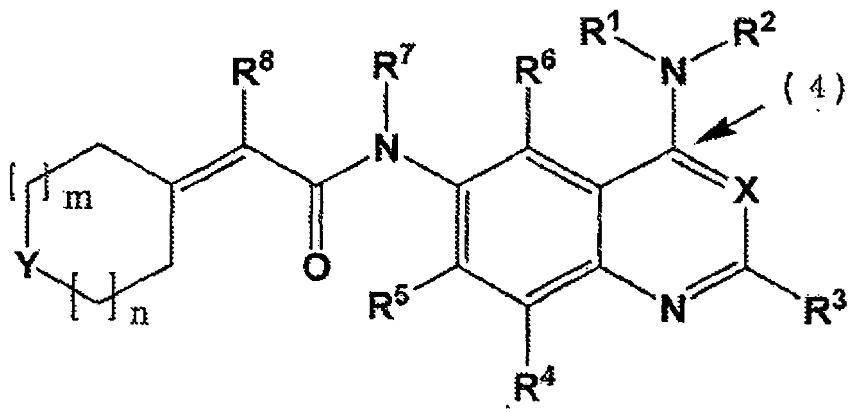

**(I)**

wherein X represents N, C-CN or CH; Y represents $CH_2$, S, O or N-$R^9$; $R^1$, $R^3$, $R^7$ and $R^8$ independently represent H, $CF_3$ or $C_{1-6}$alkyl; $R^2$ represents a group selected from formula (**II**), (**III**), (**IV**), (**V**), (**VI**), (**VII**) or (**VIII**);

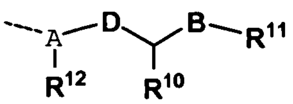

**(II)**

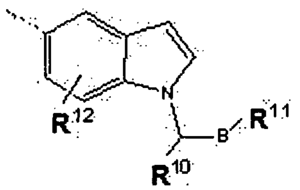

**(III)**

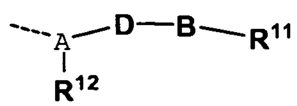

**(IV)**

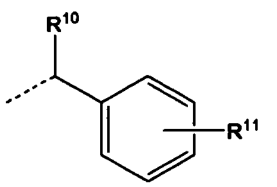

**(V)**

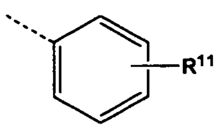

**(VI)**

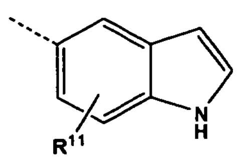

**(VII)**

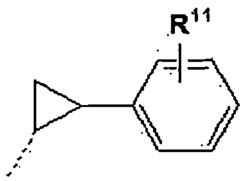

**(VIII)**

$R^4$, $R^6$ independently represent H, $C_{1-6}$alkyl, $OC_{1-6}$alkyl, OH, F, Cl, Br, $OCF_3$ or trifluoromethyl;

$R^5$ at each occurrence is independently selected from H, F, $C_{1-6}$alkyl, OH, $OC_{1-6}$alkyl, $OCF_3$, $OCF_2CH_3$, $NH_2$, NH ($C_{1-6}$alkyl), N ($C_{1-6}$alkyl)$_2$, 1- pyrrolinyl, 1- piperidyl, 4- morpholin, Cl, Br, trifluoromethyl, O $(CH_2)_{2-4}OCF_3$, O $(CH_2)_{2-4}OC_{1-6}$alkyl, O $(CH_2)_{2-4}$NH ($C_{1-6}$alkyl), O $(CH_2)_{2-4}$N ($C_{1-6}$alkyl)$_2$, (1- pyrrolinyl) $(CH_2)_{2-4}$O, (1- piperidyl) $(CH_2)_{2-4}$O, (4- morpholin) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) O) $C_{1-6}$alkyl), OC (O) NH ($C_{1-6}$alkyl), OC (O) N ($C_{1-6}$alkyl)$_2$, (1- piperidyl) $(CH_2)_{2-4}$OC (O), (4- morpholin) $(CH_2)_{2-4}$OC (O), (1- pyrrolinyl) $(CH_2)_{2-4}$OC (O), (1- imidazolyl) $(CH_2)_{2-4}$O, (4- imidazolyl) $(CH_2)_{2-4}$OC (O), (pyrazolyl) $(CH_2)_{2-4}$O, (triazolyl) $(CH_2)_{2-4}$OC (O) or Ar $(CH_2)_{1-4}$) O;

$R^9$ at each occurrence is independently selected from H, $C_{1-6}$alkyl, $CF_3$, $CF_2CH_3$, $(CH_2)_{2-4}OH$, $(CH_2)_{1-4}OC_{1-6}$alkyl, $(CH_2)_{1-4}$NH ($C_{1-6}$alkyl), $(CH_2)_{1-4}$N ($C_{1-6}$alkyl)$_2$, (1- pyrrolinyl) $(CH_2)_{1-4}$, (1- piperidyl) $(CH_2)_{1-4}$, (4- morpholin) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N ($C_{1-6}$alkyl)$_2$, (1- pyrrolinyl) $(CH_2)_{1-6}$C (O), (1- piperidyl) $(CH_2)_{1-6}$C (O), (4- morpholin) $(CH_2)_{1-4}$C (O), C (O) $OC_{1-6}$alkyl, C (O) O $(CH_2)_{2-4}OC_{1-6}$alkyl, C (O) O $(CH_2)_{2-4}$N ($C_{1-6}$alkyl)$_2$, C (O) O $(CH_2)_{2-4}$NH ($C_{1-6}$alkyl), (1- pyrrolinyl) $(CH_2)_{2-4}$OC (O), (1- piperidyl) $(CH_2)_{2-4}$OC (O), (4- morpholin) $(CH_2)_{2-4}$OC (O), $(CH_2)_{1-4}$C (O) $OC_{1-6}$alkyl, Ar $(CH_2)_{1-4}$;

$R^{10}$ represents H, $C_{1-6}$alkyl or one or two F;

$R^{11}$, $R^{12}$ independently represent H, 1 to 4 same of different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH ($C_{1-4}$alkyl), N ($C_{1-4}$alkyl)$_2$, OC (O) $C_{1-4}$alkyl, NHC (O) H, NHC (O) $C_{1-4}$alkyl, N ($C_{1-4}$alkyl) C (O) $C_{1-4}$alkyl, C (O) $OC_{1-4}$alkyl, C (O) $NHC_{1-4}$alkyl, C (O) N ($C_{1-4}$alkyl)$_2$, COOH, C (O) $C_{1-4}$alkyl, S (O) $C_{1-4}$alkyl, $SO_2C_{1-4}$alkyl, $SO_2NHC_{1-4}$alkyl or $SO_2N$ ($C_{1-4}$alkyl)$_2$;

A, B independently represent aromatic ring; Ar is phenyl, substituted phenyl or pyridyl; D represents O, S, NH or methylene; and m, n independently represent an integer of 0 to 4.

In the embodiments of the invention, $C_{1-4}$alkyl and $C_{1-6}$alkyl can be straight chain alkyl, branched chain alkyl or cyclic alkyl, saturated or unsaturated alkyl, optionally substituted with F, OH, COOH, $CO_2$ ($C_{1-4}$alkyl), C (O) $NH_2$, C (O) NH ($C_{1-4}$alkyl), C (O) N ($C_{1-4}$alkyl)$_2$, NHC (O) ($C_{1-4}$alkyl), $NH_2$, NH ($C_{1-4}$alkyl), N ($C_{1-4}$alkyl)$_2$, NHC (O) $NH_2$, NHC (NH) $NH_2$, O ($C_{1-4}$alkyl) or S ($C_{1-4}$alkyl) .

When A, B independently represent an aromatic ring, the ring can be 5 to 7 numbered and contain from 0 to 4 heteroatom such as N, O or S and so on. A, B can also independently represent a polycyclic aromatic group consisting of two or three 5 to 7 membered fused rings.

When the compound of formula (**I**) is defined as an E/Z isomer, it can be an E isomer or a Z isomer or a mixture of an E isomer and a Z isomer.

When the compound of formula (**I**) is defined as an R/S isomer, it can be an R isomer or an S isomer or a mixture of an R isomer and an S isomer.

The uniqueness of the compound of the invention can be clearly visualized by comparing the structure and property of the compound of Example **33** with the comparison compound **A** of Example **84**, **B** and **C**.

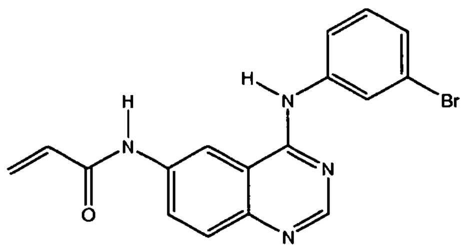

Comparison compound **C**

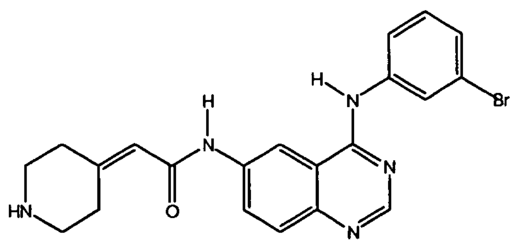

Example **33**

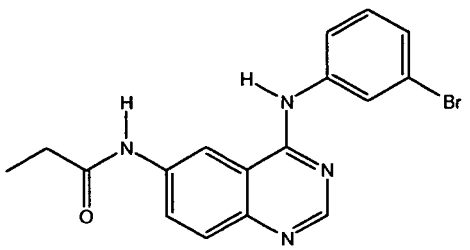

Comparison compound **A** (Example **84**)

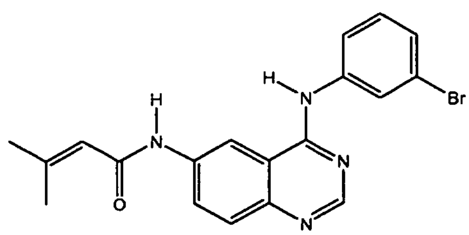

Comparison compound **B**

The comparison compound **C** is an irreversible small molecular EGFR-TK inhibitor having the highest activity among all literature-reported compounds. At a molecular level, the $EC_{50}$ value of the comparison compound **C** against EGFR-TK is $10^{-7}$ μm, while in the same the test $EC_{50}$ value of the comparison compound **B** is merely about 0.5 μm, which means the activity of the comparison compound **B** is five million times lower compared to the comparison compound **C**. The difference between the two compounds in chemical structure is merely the difference in the steric hindrance of substituted branch of the sixth position. Based on that rule, the activity of the compound of the invention is lower than that of the comparison compound **B**.

However, in a test of inhibitory activity of EGFR- TK at cellular level, the activity of the compound of the invention is several order of magnitude higher than that of the comparison compound **B**. For example, the activity of the compound in Example **33** is not only much higher than that of the comparison compound **B**, but also close to the contrast compound **A** (as shown in Tables **3**, **4**) . However, the $EC_{50}$ value of the comparison compound **A** and **C** against EGFR- TK is equivalent at the molecular level, which means the activity of the compound in Example **33** is equivalent to that of the comparison compound **C**, and it is million times higher than that of the comparison compound **B**.

It has been reported in the literatures the activity of irreversible EGFR-TK inhibitor is ten times higher that that of reversible EGFR-TK inhibitor in A431 animal tumor model, but the activity of the two is equivalent at a molecular level. The compound of the present invention is proved to be an irreversible EGFR-TK inhibitor by a cellular model test (as shown in Table **5**), while the high activity comparison compound **A** exhibits to be a reversible inhibitor in the same test, which conforms to the result by a direct test using EGFR-TK described in some literatures.

The compound of the invention has bioactivity of inhibiting Her-2 TK, and part of the results are listed in Table **6**.

The compound of the invention has a certain degree of growth inhibition against epidermal cancer cell strain A431, colorectal cancer cell strain LoVo, breast cancer cell strain BT 474 and breast cancer cell strain SK-Br-3.A part of the test results are listed in Tables **7**, **8** and **9**. Some compounds have 30 times higher growth-inhibition activities against A431 than Tarceva (as shown in Table **7**). Some have an equivalent growth-inhibition activities against breast cancer with Lapatinib (as shown in Table **8**). Some have better growth-inhibition activities against colorectal cancer cell LoVo than adriamycin (as shown in Table **9**).

Accordingly, also disclosed is a method of inhibiting cancer cell growth in mammals comprising administering to a mammal in need thereof the compound of formula (**I**) or pharmaceutically acceptable salt or hydrate thereof, wherein the cancer includes but is not limited to breast cancer, skin cancer, colorectal cancer, head and neck cancer, lung cancer, kidney cancer, bladder cancer, ovarian cancer, oral cancer, laryngeal cancer, esophagus cancer, gastric cancer, cervical cancer and liver cancer.

The compound of formula (**I**) suitable for being used as medical active ingredient comprises the compounds wherein $R^1$ represents H, $CH_3$ or $CH_2CH_3$, more particularly, $R^1$ represents H.

The compound of formula (**I**) suitable for being used as medical active ingredient comprises the compounds wherein $R^3$ represents H, $CH_3$ or $CH_2CH_3$, more particularly, $R^3$ represents H.

The compound of formula (**I**) suitable for being used as medical active ingredient comprises the compounds wherein $R^7$ represents H, $CH_3$ or $CH_2CH_3$, more particularly, $R^7$ represents H.

The compound of formula (**I**) suitable for being used as medical active ingredient comprises the compounds wherein $R^8$ represents H, $CH_3$ or $CH_2CH_3$, more particularly, $R^8$ represents H.

The compound of formula (**I**) suitable for being used as medical active ingredient comprises the compounds wherein $R^4$ represents H, F, $CH_3$, $CH_2CH_3$, $CH_2CH_2CH_3$, $OCH_3$ or $OCH_2CH_3$, more particularly, $R^4$ represents H, F, $CH_3$, $CH_2CH_3$ or $OCH_3$, and most particluarly, $R^4$ represents H, F, $CH_3$ or $OCH_3$.

The compound of formula (**I**) suitable for being used as medical active ingredient comprises the compounds wherein $R^6$ represents H, F, $CH_3$, $CH_2CH_3$, $CH_2CH_2CH_3$, $OCH_3$, $OCF_3$ or $OCH_2CH_3$, more particularly, $R^6$ represents H, F, $CH_3$, $CH_2CH_3$ or $OCH_3$, and most particularly, $R^6$ represents H, F, $CH_3$ or $OCH_3$.

The compound of formula (**I**) suitable for being used as medical active ingredient comprises the compounds wherein A represents benzene ring, pyridine, pyrimidine, pyrazine, 4-nigrogen heterocyclic pyridine, pyrrole, furan, thiophene, pyrazole, thiazole, indole, benzofuran, benzothiophene or naphthalene ring, more particularly, A represents benzene ring, pyridine, thiophene, pyrazole, thiazole, indole or naphthalene ring.

The compound of formula (**I**) suitable for being used as medical active ingredient comprises the compounds wherein B represents benzene ring, pyridine, pyrimidine, pyrazine, 4-nigrogen heterocyclic pyridine, pyrrole, furan, thiophene, pyrazole, thiazole, indole, benzofuran, benzothiophene or naphthalene ring, more particularly, B represents benzene ring, pyridine, thiophene, pyrazole, thiazole, indole or naphthalene ring.

The compound of formula (**I**) suitable for being used as medical active ingredient comprises the compounds wherein D represents O, S or NH, more particularly, D represents O.

The compound of formula (**I**) suitable for being used as medical active ingredient comprises the compounds wherein $R^{10}$ represents H, one or two F, $CH_3$, $CF_3$ or Et, more particularly, $R^{10}$ represents H, two F, $CH_3$ or $CF_3$, and most particularly, $R^{10}$ represents H.

The compound of formula (**I**) suitable for being used as medical active ingredient comprises the compounds

wherein $R^{11}$ represents H, 1 to 4 same or different F, Cl, Br, CN, $NO_2$, $CF_3$, OH, $CH_3$, $CH_2CH_3$, $CH_2CH_2CH_3$, alkynyl, $OCH_3$, $OCH_2CH_3$, $OCF_3$, $CO_2CH_3$, $CO_2CH_2CH_3$, $NH_2$, $NHCH_3$, $NHCH_2CH_3$, N $(CH_3)_2$, NHC (O) H, NHC (O) $CH_3$, NHC (O) $CH_2CH_3$, N $(CH_3)$ C (O) $CH_3$, C (O) $NHCH_3$, C (O) $NHCH_2CH_3$ or C (O) N $(CH_3)_2$, more particularly, $R^{11}$ represents H, one or two same or different F, Cl, Br, CN, $CF_3$, OH, $CH_3$, $CH_2CH_3$, ethynyl, $OCH_3$, $OCH_2CH_3$, $OCF_3$, $CO_2CH_3$, $CO_2CH_2CH_3$, $NH_2$, $NHCH_3$, $NHCH_2CH_3$, N $(CH_3)_2$ or NHC (O) $CH_3$, most particularly, $R^{11}$ represents H, one or two same or different F, Cl, Br, CN, $CF_3$, $CH_3$, $CH_2CH_3$, ethynyl, $OCH_3$, $OCH_2CH_3$, $OCF_3$, $CO_2CH_2CH_3$, $NH_2$, $NHCH_3$, $NHCH_2CH_3$, N $(CH_3)_2$ or NHC (O) $CH_3$.

The compound of formula (**I**) suitable for being used as medical active ingredient comprises the compounds wherein $R^{12}$ represents H, 1 to 4 same or different F, Cl, Br, CN, $NO_2$, $CF_3$, OH, $CH_3$, $CH_2CH_3$, $CH_2CH_2CH_3$, alkynyl, $OCH_3$, $OCH_2CH_3$, $OCF_3$, $CO_2CH_3$, $CO_2CH_2CH_3$, $NH_2$, $NHCH_3$, $NHCH_2CH_3$, N $(CH_3)_2$, NHC (O) H, NHC (O) $CH_3$, NHC (O) $CH_2CH_3$, N $(CH_3)$ C (O) $CH_3$, C (O) $NHCH_3$, C (O) $NHCH_2CH_3$ or C (O) N $(CH_3)_2$, more particularly, $R^{12}$ represents H, one or two same or different F, Cl, Br, CN, $CF_3$, OH, $CH_3$, $CH_2CH_3$, ethynyl, $OCH_3$, $OCH_2CH_3$, $OCF_3$, $CO_2CH_3$, $CO_2CH_2CH_3$, $NH_2$, $NHCH_3$, $NHCH_2CH_3$, N $(CH_3)_2$ or NHC (O) $CH_3$.

The compound of formula (**I**) suitable for being used as medical active ingredient comprises the compounds wherein $R^5$ represents H, F, OH, $CH_3$, $CH_2CH_3$, $CH_2CH_2CH_3$, $OCH_3$, $OCH_2CH_3$, $OCF_3$, $OCF_2CH_3$, $NH_2$, $NHCH_3$, N $(CH_3)_2$, 1- pyrrolinyl, 1- piperidyl, 4- morpholin, trifluoromethyl, O $(CH_2)_2OH$, O $(CH_2)_3OH$, O $(CH_2)_4OH$, O $(CH_2)_2OCH_3$, O $(CH_2)_3OCH_3$, O $(CH_2)_4OCH_3$, O $(CH_2)_2OCH_2CH_3$, O $(CH_2)_3OCH_2CH_3$, O $(CH_2)_4OCH_2CH_3$, O $(CH_2)_2N$ $(CH_3)_2$, O $(CH_2)_3N$ $(CH_3)_2$, O $(CH_2)_4N$ $(CH_3)_2$, O $(CH_2)_2N$ $(CH_2CH_3)_2$, O $(CH_2)_3N$ $(CH_2CH_3)_2$, O $(CH_2)_4N$ $(CH_2CH_3)_2$, O $(CH_2)_2N$ $(CH_3)$ $CH_2CH_3$, O $(CH_2)_3N$ $(CH_3)$ $CH_2CH_3$, O $(CH_2)_4N$ $(CH_3)$ $CH_2CH_3$, O $(CH_2)_2NH$ $(CH_3)$, O $(CH_2)_3NH$ $(CH_3)$, O $(CH_2)_4NH$ $(CH_3)$, (1- pyrrolinyl) $(CH_2)_2O$, (1- pyrrolinyl) $(CH_2)_3O$, (1- pyrrolinyl) $(CH_2)_4O$, (1- piperidyl) $(CH_2)_2O$, (1- piperidyl) $(CH_2)_3O$, (1- piperidyl) $(CH_2)_4O$, (4- morpholin) $(CH_2)_2O$, (4- morpholin) $(CH_2)_3O$, (4- morpholin) $(CH_2)_4O$, NHC (O) H, NHC (O) $CH_3$, NHC (O) $CH_2CH_3$, NHC (O) $CH_2CH_2CH_3$, N $(CH_3)$ C (O) H, N $(CH_3)$ C (O) $CH_3$, N $(CH_3)$ C (O) $CH_2CH_3$, N $(CH_3)$ C (O) $CH_2CH_2CH_3$, NHC (O) $OCH_3$, NHC (O) $OCH_2CH_3$, N $(CH_3)$ C (O) $OCH_3$, N $(CH_3)$ C (O) $OCH_2CH_3$, N $(CH_3)$ C (O) $OCH_2CH_2CH_3$, OC (O) $NHCH_3$, OC (O) $NHCH_2CH_3$, OC (O) N $(CH_3)_2$ or OC (O) N $(CH_3)$ $CH_2CH_3$.

The compound of formula (**I**) suitable for being used as medical active ingredient comprises the compounds wherein $R^9$ represents H, $CH_3$, $CH_2CH_3$, $CH_2CH_2CH_3$, $CF_3$, $CF_2CH_3$, $(CH_2)_2OH$, $(CH_2)_3OH$, $(CH_2)_4OH$, $(CH_2)_2OCH_3$, $(CH_2)_3OCH_3$, $(CH_2)_4OCH_3$, $(CH_2)_2OCH_2CH_3$, $(CH_2)_3OCH_2CH_3$, $(CH_2)_4OCH_2CH_3$, $(CH_2)_2N$ $(CH_3)_2$, $(CH_2)_3N$ $(CH_3)_2$, $(CH_2)_4N$ $(CH_3)_2$, $(CH_2)_2N$ $(CH_2CH_3)_2$, $(CH_2)_3N$ $(CH_2CH_3)_2$, $(CH_2)_4N$ $(CH_2CH_3)_2$, $(CH_2)_2N$ $(CH_3)$ $CH_2CH_3$, $(CH_2)_3N$ $(CH_3)$ $CH_2CH_3$, $(CH_2)_4N$ $(CH_3)$ $CH_2CH_3$, $(CH_2)_2NH$ $(CH_3)$, $(CH_2)_3NH$ $(CH_3)$, $(CH_2)_4NH$ $(CH_3)$, (1- pyrrolinyl) $(CH_2)_2$-, (1- pyrrolinyl) $(CH_2)_3$-, (1- pyrrolinyl) $(CH_2)_4$-, (1- piperidyl) $(CH_2)_2$-, (1- piperidyl) $(CH_2)_3$, (1- piperidyl) $(CH_2)_4$, (4- morpholin) $(CH_2)_2$, (4- morpholin) $(CH_2)_3$, (4- morpholin) $(CH_2)_4$, C (O) $CH_3$, C (O) $CH_2CH_3$, C (O) $CH_2CH_2CH_3$, C (O) $(CH_2)_4OH$, C (O) $CH_2OH$, C (O) $(CH_2)_2OH$, C (O) $(CH_2)_3OH$, C (O) $CH_2OCH_3$, C (O) $(CH_2)_2OCH_3$, C (O) $(CH_2)_3OCH_3$, C (O) $(CH_2)_4OCH_3$, C (O) $CH_2OCH_2CH_3$, C (O) $CH_2N$ $(CH_3)_2$, C (O) $(CH_2)_2N$ $(CH_3)_2$, C (O) $(CH_2)_3N$ $(CH_3)_2$, C (O) $(CH_2)_4N$ $(CH_3)_2$, C (O) $CH_2N$ $(CH_3)$ $CH_2CH_3$, C (O) $(CH_2)_2N$ $(CH_3)$ $CH_2CH_3$, C (O) $(CH_2)_3N$ $(CH_3)$ $CH_2CH_3$, C (O) $(CH_2)_4N$ $(CH_3)$ $CH_2CH_3$, C (O) $CH_2NHCH_2CH_3$, C (O) $(CH_2)_2NHCH_2CH_3$, C (O) $(CH_2)_3NHCH_2CH_3$, C (O) $(CH_2)_4NHCH_2CH_3$, C (O) $CH_2NHCH_3$, C (O) $(CH_2)_2NHCH_3$, C (O) $(CH_2)_3NHCH_3$, C (O) $(CH_2)_4NHCH_3$, C (O) $CH_2NHPr$, C (O) $(CH_2)_2NHPr$, C (O) $(CH_2)_3NHPr$, C (O) $(CH_2)_4NHPr$ , C (O) $CH_2N$ $(CH_2CH_3)_2$, C (O) $(CH_2)_2N$ $(CH_2CH_3)_2$, C (O) $(CH_2)_3N$ $(CH_2CH_3)_2$, C (O) $(CH_2)_4N$ $(CH_2CH_3)_2$, (1- pyrrolinyl) $CH_2C$ (O), (1- pyrrolinyl) $(CH_2)_2C$ (O), (1- pyrrolinyl) $(CH_2)_3C$ (O), (1- pyrrolinyl) $(CH_2)_4C$ (O), (1- piperidyl) $CH_2C$ (O), (1- piperidyl) $(CH_2)_2C$ (O), (1- piperidyl) $(CH_2)_3C$ (O), (1- piperidyl) $(CH_2)_4C$ (O), (4- morpholin) $(CH_2)_4C$ (O), (4- morpholin) $(CH_2)_3C$ (O), (4- morpholin) $CH_2C$ (O), (4- morpholin) $(CH_2)_2C$ (O), C (O) $OCH_3$, C (O) $OCH_2CH_3$, C (O) OPr, C (O) OPr- i, C (O) O $(CH_2)_2OCH_3$, C (O) O $(CH_2)_3OCH_3$, C (O) O $(CH_2)_4OCH_3$, C (O) O $(CH_2)_2OCH_2CH_3$, C (O) O $(CH_2)_2N$ $(CH_3)_2$, C (O) O $(CH_2)_3N$ $(CH_3)_2$, C (O) O $(CH_2)_4N$ $(CH_3)_2$, (1- pyrrolinyl) $(CH_2)_2OC$ (O), (1- pyrrolinyl) $(CH_2)_3OC$ (O), (1- pyrrolinyl) $(CH_2)_4OC$ (O), (1- piperidyl) $(CH_2)_2OC$ (O), (1- piperidyl) $(CH_2)_3OC$ (O), (1- piperidyl) $(CH_2)_4OC$ (O), (4- morpholin) $(CH_2)_2OC$ (O), (4- morpholin) $(CH_2)_3OC$ (O), (4- morpholin) $(CH_2)_4OC$ (O), $CH_2C$ (O) $OCH_3$, $(CH_2)_2C$ (O) $OCH_3$, $(CH_2)_3C$ (O) $OCH_3$, $CH_2C$ (O) $OCH_2CH_3$, $(CH_2)_2C$ (O) $OCH_2CH_3$ or $(CH_2)_3C$ (O) $OCH_2CH_3$.

The compound of formula (**I**) suitable for being used as medical active ingredient comprises the compounds wherein m, n independently represent 0, 1 or 2.

**Table 1**

| Examples of $R^9$, $R^{11}$ in the compound of formula (**I**) suitable for being used as medical active ingredient. | | | |
|---|---|---|---|
| $R^9$ | $R^{11}$ | $R^9$ | $R^{11}$ |
| H | H | H | 3-Cl |
| H | 3-Br | H | 3-F |
| H | 3-$CH_3$ | H | 3-$CF_3$ |

(continued)

| Examples of $R^9$, $R^{11}$ in the compound of formula (**I**) suitable for being used as medical active ingredient. | | | |
|---|---|---|---|
| $R^9$ | $R^{11}$ | $R^9$ | $R^{11}$ |
| H | 3-alkynyl | H | 3-CN |
| H | 3-$NO_2$ | H | 3-$OCH_3$ |
| H | 3-C(O)$CH_3$ | H | 3-$SO_2CH_3$ |
| H | 3-S(O)$CH_3$ | H | 3-$CO_2CH_3$ |
| H | 3-$SO_2NHCH_3$ | H | 3-$CO_2CH_2CH_3$ |
| H | 3-$SO_2N(CH_3)_2$ | H | 3-$CONHCH_3$ |
| H | 3-$CON(CH_3)_2$ | H | 4-$CON(CH_3)_2$ |
| H | 3-I | H | 4-Cl |
| H | 4-Br | H | 4-F |
| H | 4-$CH_3$ | H | 4-$CF_3$ |
| H | 4-alkynyl | H | 4-CN |
| H | 4-$NO_2$ | H | 4-$OCH_3$ |
| H | 4-C(O)$CH_3$ | H | 4-$SO_2CH_3$ |
| H | 4-S(O)$CH_3$ | H | 4-$CO_2CH_3$ |
| H | 4-$SO_2NHCH_3$ | H | 4-$CO_2CH_2CH_3$ |
| H | 4-$SO_2N(CH_3)_2$ | H | 4-$CONHCH_3$ |
| H | 4-F-3-Br | H | 4-F-3-I |
| H | 4-F-3-$CH_3$ | H | 4-F-3-CF3 |
| H | 4-F-3-alkynyl | H | 4-F-3-CN |
| H | 4-F-3-$NO_2$ | H | 4-F-3-OCH3 |
| H | 4-F-3-C(O)$CH_3$ | H | 4-F-3-$SO_2CH_3$ |
| H | 4-F-3-S(O)$CH_3$ | H | 4-F-3-$CO_2CH_3$ |
| H | 4-F-3-$SO_2NHCH_3$ | H | 4-F-3-$CO_2CH_2CH_3$ |
| H | 4-F-3-$SO_2N(CH_3)_2$ | H | 4-F-3-$CONHCH_3$ |
| H | 4-F-3-$CON(CH_3)_2$ | H | 3-F-4-F |
| H | 4-F-3-Cl | | |
| $CH_3$ | H | $CH_3$ | 3-Cl |
| $CH_3$ | 3-Br | $CH_3$ | 3-F |
| $CH_3$ | 3-CH3 | $CH_3$ | 3-$CF_3$ |
| $CH_3$ | 3-alkynyl | $CH_3$ | 3-CN |
| $CH_3$ | 3-N02 | $CH_3$ | 3-$OCH_3$ |
| $CH_3$ | 3-C(O)$CH_3$ | $CH_3$ | 3-$SO_2CH_3$ |
| $CH_3$ | 3-S(O)$CH_3$ | $CH_3$ | 3-$CO_2CH_3$ |
| $CH_3$ | 3-$SO_2NHCH_3$ | $CH_3$ | 3-$CO_2CH_2CH_3$ |
| $CH_3$ | 3-$SO_2N(CH_3)_2$ | $CH_3$ | 3-$CONHCH_3$ |
| $CH_3$ | 3-$CON(CH_3)_2$ | $CH_3$ | 4-$CON(CH_3)_2$ |
| $CH_3$ | 3-I | $CH_3$ | 4-Cl |
| $CH_3$ | 4-Br | $CH_3$ | 4-F |
| $CH_3$ | 4-$CH_3$ | $CH_3$ | 4-$CF_3$ |
| $CH_3$ | 4-alkynyl | $CH_3$ | 4-CN |
| $CH_3$ | 4-$SO_2N(CH_3)_2$ | $CH_3$ | 4-$CONHCH_3$ |
| $CH_3$ | 4-F-3-Br | $CH_3$ | 4-F-3-I |
| $CH_3$ | 4-F-3-$CH_3$ | $CH_3$ | 4-F-3-CF3 |
| $CH_3$ | 4-F-3-alkynyl | $CH_3$ | 4-F-3-CN |
| $CH_3$ | 4-F-3-$NO_2$ | $CH_3$ | 4-F-3-OCH3 |
| $CH_3$ | 4-F-3-C(O)$CH_3$ | $CH_3$ | 4-F-3-$SO_2CH_3$ |
| $CH_3$ | 4-F-3-S(O)$CH_3$ | $CH_3$ | 4-F-3-$CO_2CH_3$ |
| $CH_3$ | 4-F-3-$SO_2NHCH_3$ | $CH_3$ | 4-F-3-$CO_2CH_2CH_3$ |

(continued)

| Examples of $R^9$, $R^{11}$ in the compound of formula (**I**) suitable for being used as medical active ingredient. | | | |
|---|---|---|---|
| $R^9$ | $R^{11}$ | $R^9$ | $R^{11}$ |
| $CH_3$ | 4-F-3-$SO_2N(CH_3)_2$ | $CH_3$ | 4-F-3-$CONHCH_3$ |
| $CH_3$ | 4-F-3-$CON(CH_3)_2$ | $CH_3$ | 4-F-3-$CON(CH_3)_2$ |
| $CH_3$ | 4-F-3-Cl | $CH_3$ | 3-F-4-F |
| $CH_3$ | 2-$SO_2CH_3$ | $CH_3$ | 3-COOH |
| $CH_3$ | 4-COOH | $CH_3$ | 2-C(O)$OCH_3$ |
| $CH_3$ | 2-1 | $CH_3$ | 2-Cl |
| $CH_3$ | 2-Br | $CH_3$ | 2-F |
| $CH_3$ | 2-$CH_3$ | $CH_3$ | 2-$CH_3$ |
| $CH_3$ | 2-alkynyl | $CH_3$ | 2-CN |
| $CH_3$ | 2-$NO_2$ | $CH_3$ | 2-$OCH_3$ |
| $CH_3$ | 2-C(O)$CH_3$ | Et | 3-Cl |
| Et | 3-Br | Et | 3-F |
| Et | 3-CH3 | Et | 3-$CF_3$ |
| Et | 3-alkynyl | Et | 3-CN |
| Et | 3-NO2 | Et | 3-$OCH_3$ |
| Et | 3-C(O)$CH_3$ | Et | 3-$SO_2CH_3$ |
| Et | 3-S(O)$CH_3$ | Et | 3-$CO_2CH_3$ |
| Et | 3-$SO_2NHCH_3$ | Et | 3-$CO_2CH_2CH_3$ |
| Et | 3-$SO_2N(CH_3)_2$ | Et | 3-$CONHCH_3$ |
| Et | 3-$CON(CH_3)_2$ | Et | 4-$CON(CH_3)_2$ |
| Et | 3-I | Et | 4-Cl |
| Et | 4-Br | Et | 4-F |
| Et | 4-$CH_3$ | Et | 4-$CF_3$ |
| Et | 4-alkynyl | Et | 4-CN |
| Et | 4-$NO_2$ | Et | 4-$OCH_3$ |
| Et | 4-C(O)$CH_3$ | Et | 4-$SO_2CH_3$ |
| Et | 4-S(O)$CH_3$ | Et | 4-$CO_2CH_3$ |
| Et | 4-$SO_2NHCH_3$ | Et | 4-$CO_2CH_2CH_3$ |
| Et | 4-$SO_2N(CH_3)_2$ | Et | 4-$CONHCH_3$ |
| Et | 4-F-3-Br | Et | 4-F-3-I |
| Et | 4-F-3-$CH_3$ | Et | 4-F-3-CF3 |
| Et | 4-F-3-alkynyl | Et | 4-F-3-CN |
| Et | 4-F-3-$NO_2$ | Et | 4-F-3-OCH3 |
| Et | 4-F-3-C(O)$CH_3$ | Et | 4-F-3-$SO_2CH_3$ |
| Et | 4-F-3-S(O)$CH_3$ | Et | 4-F-3-$CO_2CH_3$ |
| Et | 4-F-3-$SO_2NHCH_3$ | Et | 4-F-3-$CO_2CH_2CH_3$ |
| Et | 4-F-3-$SO_2N(CH_3)_2$ | Et | 4-F-3-$CONHCH_3$ |
| Et | 4-F-3-$CON(CH_3)_2$ | Et | H |
| Et | 4-F-3-Cl | Et | 3-F-4-F |
| Et | 2-$SO_2CH_3$ | Et | 3-COOH |
| Et | 4-COOH | Et | 2-C(O)$OCH_3$ |
| Et | 2-I | Et | 2-Cl |
| Et | 2-Br | Et | 2-F |
| Et | 2-$CH_3$ | Et | 2-$CH_3$ |
| Et | 2-alkynyl | Et | 2-CN |
| Et | 2-$NO_2$ | Et | 2-$OCH_3$ |
| Et | 2-C(O)$CH_3$ | $O(CH_2)_2OMe$ | 3-$CON(CH_3)_2$ |
| $O(CH_2)_2OMe$ | H | $O(CH_2)_2OMe$ | 3-Cl |

(continued)

| Examples of $R^9$, $R^{11}$ in the compound of formula (**I**) suitable for being used as medical active ingredient. | | | |
|---|---|---|---|
| $R^9$ | $R^{11}$ | $R^9$ | $R^{11}$ |
| $O(CH_2)_2OMe$ | 3-Br | $O(CH_2)_2OMe$ | 3-F |
| $O(CH_2)_2OMe$ | 3-$CH_3$ | $O(CH_2)_2OMe$ | 3-$CF_3$ |
| $O(CH_2)_2OMe$ | 3-alkynyl | $O(CH_2)_2OMe$ | 3-CN |
| $O(CH_2)_2OMe$ | 3-$NO_2$ | $O(CH_2)_2OMe$ | 3-$OCH_3$ |
| $O(CH_2)_2OMe$ | 3-C(O)$CH_3$ | $O(CH_2)_2OMe$ | 3-$SO_2CH_3$ |
| $O(CH_2)_2OMe$ | 3-S(O)$CH_3$ | $O(CH_2)_2OMe$ | 3-$CO_2CH_3$ |
| $O(CH_2)_2OMe$ | 3-$sO_2NHCH_3$ | $O(CH_2)_2OMe$ | 3-$CO_2CH_2CH_3$ |
| $O(CH_2)_2OMe$ | 3-$SO_2N(CH_3)_2$ | $O(CH_2)_2OMe$ | 3-$CONHCH_3$ |
| $O(CH_2)_2OMe$ | 3-I | $O(CH_2)_2OMe$ | 4-Cl |
| $O(CH_2)_2OMe$ | 4-Br | $O(CH_2)_2OMe$ | 4-F |
| $O(CH_2)_2OMe$ | 4-$CH_3$ | $O(CH_2)_2OMe$ | 4-$CF_3$ |
| $O(CH_2)_2OMe$ | 4-alkynyl | $O(CH_2)_2OMe$ | 4-CN |
| $O(CH_2)_2OMe$ | 4-$NO_2$ | $O(CH_2)_2OMe$ | 4-$OCH_3$ |
| $O(CH_2)_2OMe$ | 4-C(O)$CH_3$ | $O(CH_2)_2OMe$ | 4-$SO_2CH_3$ |
| $O(CH_2)_2OMe$ | 4-S(O)$CH_3$ | $O(CH_2)_2OMe$ | 4-$CO_2CH_3$ |
| $O(CH_2)_2OMe$ | 4-$SO_2NHCH_3$ | $O(CH_2)_2OMe$ | 4-$CO_2CH_2CH_3$ |
| $O(CH_2)_2OMe$ | 4-$SO_2N(CH_3)_2$ | $O(CH_2)_2OMe$ | 4-$CONHCH_3$ |
| $O(CH_2)_2OMe$ | 4-$CON(CH_3)$ | $O(CH_2)_2OMe$ | 4-F-3-$CON(CH_3)_2$ |
| $O(CH_2)_2OMe$ | 4-F-3-Br | $O(CH_2)_2OMe$ | 4-F-3-I |
| $O(CH_2)_2OMe$ | 4-F-3-$CH_3$ | $O(CH_2)_2OMe$ | 4-F-3-CF3 |
| $O(CH_2)_2OMe$ | 4-F-3-alkynyl | $O(CH_2)_2OMe$ | 4-F-3-CN |
| $O(CH_2)_2OMe$ | 4-F-3-$NO_2$ | $O(CH_2)_2OMe$ | 4-F-3-OCH3 |
| $O(CH_2)_2OMe$ | 4-F-3-C(O)$CH_3$ | $O(CH_2)_2OMe$ | 4-F-3-$SO_2CH_3$ |
| $O(CH_2)_2OMe$ | 4-F-3-S(O)$CH_3$ | $O(CH_2)_2OMe$ | 4-F-3-$CO_2CH_3$ |
| $O(CH_2)_2OMe$ | 4-F-3-$SO_2NHCH_3$ | $O(CH_2)_2OMe$ | 4-F-3-$CO_2CH_2CH_3$ |
| $O(CH_2)_2OMe$ | 4-F-3-$SO_2N(CH_3)_2$ | $O(CH_2)_2OMe$ | 4-F-3-$CONHCH_3$ |
| $O(CH_2)_2OMe$ | 4-F-3-Cl | $O(CH_2)_2OMe$ | 3-F-4-F |
| $O(CH_2)_2OMe$ | 2-$SO_2CH_3$ | $O(CH_2)_2OMe$ | 3-COOH |
| $O(CH_2)_2OMe$ | 4-COOH | $O(CH_2)_2OMe$ | 2-C(O)$OCH_3$ |
| $O(CH_2)_2OMe$ | 2-I | $O(CH_2)_2OMe$ | 2-Cl |
| $O(CH_2)_2OMe$ | 2-Br | $O(CH_2)_2OMe$ | 2-F |
| $O(CH_2)_2OMe$ | 2-$CH_3$ | $O(CH_2)_2OMe$ | 2-$CH_3$ |
| $O(CH_2)_2OMe$ | 2-alkynyl | $O(CH_2)_2OMe$ | 2-CN |
| $O(CH_2)_2OMe$ | 2-$NO_2$ | $O(CH_2)_2OMe$ | 2-$OCH_3$ |
| $O(CH_2)_2OMe$ | 2-C(O)$CH_3$ | $O(CH_2)_2OMe$ | 3-Cl |
| $O(CH_2)_2OMe$ | 3-Br | $O(CH_2)_2OMe$ | 3-F |
| $O(CH_2)_2OMe$ | 3-$CH_3$ | $O(CH_2)_2OMe$ | 3-$CF_3$ |
| $O(CH_2)_2OMe$ | 3-alkynyl | $O(CH_2)_2OMe$ | 3-CN |
| $O(CH_2)_2OMe$ | 3-$NO_2$ | $O(CH_2)_2OMe$ | 3-$OCH_3$ |
| $O(CH_2)_2OMe$ | 3-C(O)$CH_3$ | $O(CH_2)_2OMe$ | 3-$SO_2CH_3$ |
| $O(CH_2)_2OMe$ | 3-S(O)$CH_3$ | $O(CH_2)_2OMe$ | 3-$CO_2CH_3$ |
| $O(CH_2)_2OMe$ | 3-$SO_2NHCH_3$ | $O(CH_2)_2OMe$ | 3-$CO_2CH_2CH_3$ |
| $O(CH_2)_2OMe$ | 3-$SO_2N(CH_3)_2$ | $O(CH_2)_2OMe$ | 3-$CONHCH_3$ |
| $O(CH_2)_2OMe$ | 3-$CON(CH_3)_2$ | $O(CH_2)_2OMe$ | 4-$CON(CH_3)_2$ |
| $O(CH_2)_2OMe$ | 3-I | $O(CH_2)_2OMe$ | 4-Cl |
| $O(CH_2)_2OMe$ | 4-Br | $O(CH_2)_2OMe$ | 4-F |
| $O(CH_2)_2OMe$ | 4-$CH_3$ | $O(CH_2)_2OMe$ | 4-$CF_3$ |
| $O(CH_2)_2OMe$ | 4-alkynyl | $O(CH_2)_2OMe$ | 4-CN |

(continued)

Examples of $R^9$, $R^{11}$ in the compound of formula (**I**) suitable for being used as medical active ingredient.

| $R^9$ | $R^{11}$ | $R^9$ | $R^{11}$ |
|---|---|---|---|
| $O(CH_2)_2OMe$ | $4\text{-}NO_2$ | $O(CH_2)_2OMe$ | $4\text{-}OCH_3$ |
| $O(CH_2)_2OMe$ | $4\text{-}C(O)CH_3$ | $O(CH_2)_2OMe$ | $4\text{-}SO_2CH_3$ |
| $O(CH_2)_2OMe$ | $4\text{-}S(O)CH_3$ | $O(CH_2)_2OMe$ | $4\text{-}CO_2CH_3$ |
| $O(CH_2)_2OMe$ | $4\text{-}SO_2NHCH_3$ | $O(CH_2)_2OMe$ | $4\text{-}CO_2CH_2CH_3$ |
| $O(CH_2)_2OMe$ | $4\text{-}SO_2N(CH_3)_2$ | $O(CH_2)_2OMe$ | $4\text{-}CONHCH_3$ |
| $O(CH_2)_2OMe$ | 4-F-3-alkynyl | $O(CH_2)_2OMe$ | 4-F-3-CN |
| $O(CH_2)_2OMe$ | 4-F-3-Br | $O(CH_2)_2OMe$ | 4-F-3-I |
| $O(CH_2)_2OMe$ | $4\text{-}F\text{-}3\text{-}CH_3$ | $O(CH_2)_2OMe$ | 4-F-3-CF3 |
| $O(CH_2)_2OMe$ | $4\text{-}F\text{-}3\text{-}NO_2$ | $O(CH_2)_2OMe$ | 4-F-3-OCH3 |
| $O(CH_2)_2OMe$ | $4\text{-}F\text{-}3\text{-}C(O)CH_3$ | $O(CH_2)_2OMe$ | $4\text{-}F\text{-}3\text{-}SO_2CH_3$ |
| $O(CH_2)_2OMe$ | $4\text{-}F\text{-}3\text{-}S(O)CH_3$ | $O(CH_2)_2OMe$ | $4\text{-}F\text{-}3\text{-}CO_2CH_3$ |
| $O(CH_2)_2OMe$ | $4\text{-}F\text{-}3\text{-}SO_2NHCH_3$ | $O(CH_2)_2OMe$ | $4\text{-}F\text{-}3\text{-}CO_2CH_2CH_3$ |
| $O(CH_2)_2OMe$ | $4\text{-}F\text{-}3\text{-}SO_2N(CH_3)_2$ | $O(CH_2)_2OMe$ | $4\text{-}F\text{-}3\text{-}CONHCH_3$ |
| $O(CH_2)_2OMe$ | $4\text{-}F\text{-}3\text{-}CON(CH_3)_2$ | $O(CH_2)_2OMe$ | 4-F-3-Cl |
| $O(CH_2)_2OMe$ | 3-F-4-F | $O(CH_2)_2OMe$ | $2\text{-}SO_2CH_3$ |
| $O(CH_2)_2OMe$ | H | $O(CH_2)_2OMe$ | 3-COOH |
| $O(CH_2)_2OMe$ | 4-COOH | $O(CH_2)_2OMe$ | $2\text{-}C(O)OCH_3$ |
| $O(CH_2)_2OMe$ | 2-I | $O(CH_2)_2OMe$ | 2-Cl |
| $O(CH_2)_2OMe$ | 2-Br | $O(CH_2)_2OMe$ | 2-F |
| $O(CH_2)_2OMe$ | $2\text{-}CH_3$ | $O(CH_2)_2OMe$ | $2\text{-}CF_3$ |
| $O(CH_2)_2OMe$ | 2-alkynyl | $O(CH_2)_2OMe$ | 2-CN |
| $O(CH_2)_2OMe$ | $2\text{-}NO_2$ | $O(CH_2)_2OMe$ | $2\text{-}OCH_3$ |
| $O(CH_2)_2OMe$ | $2\text{-}C(O)CH_3$ | $O(CH_2)_2OH$ | 3-I |
| $O(CH_2)_2OH$ | H | $O(CH_2)_2OH$ | 3-Cl |
| $O(CH_2)_2OH$ | 3-Br | $O(CH_2)_2OH$ | 3-F |
| $O(CH_2)_2OH$ | $3\text{-}CH_3$ | $O(CH_2)_2OH$ | $3\text{-}CF_3$ |
| $O(CH_2)_2OH$ | 3-alkynyl | $O(CH_2)_2OH$ | 3-CN |
| $O(CH_2)_2OH$ | $3\text{-}NO_2$ | $O(CH_2)_2OH$ | $3\text{-}OCH_3$ |
| $O(CH_2)_2OH$ | $3\text{-}C(O)CH_3$ | $O(CH_2)_2OH$ | $3\text{-}CO_2CH_3$ |
| $O(CH_2)_2OH$ | $3\text{-}S(O)CH_3$ | $O(CH_2)_2OH$ | $3\text{-}CO_2CH_2CH_3$ |
| $O(CH_2)_2OH$ | $3\text{-}SO_2NHCH_3$ | $O(CH_2)_2OH$ | $3\text{-}CONHCH_3$ |
| $O(CH_2)_2OH$ | $3\text{-}SO_2N(CH_3)_2$ | $O(CH_2)_2OH$ | $4\text{-}CON(CH_3)_2$ |
| $O(CH_2)_2OH$ | $3\text{-}CON(CH_3)_2$ | $O(CH_2)_2OH$ | 4-Cl |
| $O(CH_2)_2OH$ | 4-Br | $O(CH_2)_2OH$ | 4-F |
| $O(CH_2)_2OH$ | $4\text{-}CH_3$ | $O(CH_2)_2OH$ | $4\text{-}CF_3$ |
| $O(CH_2)_2OH$ | 4-alkynyl | $O(CH_2)_2OH$ | 4-CN |
| $O(CH_2)_2OH$ | $4\text{-}NO_2$ | $O(CH_2)_2OH$ | $4\text{-}OCH_3$ |
| $O(CH_2)_2OH$ | $4\text{-}C(O)CH_3$ | $O(CH_2)_2OH$ | $4\text{-}SO_2CH_3$ |
| $O(CH_2)_2OH$ | $4\text{-}S(O)CH_3$ | $O(CH_2)_2OH$ | $4\text{-}CO_2CH_3$ |
| $O(CH_2)_2OH$ | $4\text{-}SO_2NHCH_3$ | $O(CH_2)_2OH$ | $4\text{-}CO_2CH_2CH_3$ |
| $O(CH_2)_2OH$ | $4\text{-}SO_2N(CH_3)_2$ | $O(CH_2)_2OH$ | $4\text{-}CONHCH_3$ |
| $O(CH_2)_2OH$ | $4\text{-}CON(CH_3)_2$ | $O(CH_2)_2OH$ | $4\text{-}F\text{-}3\text{-}CON(CH_3)_2$ |
| $O(CH_2)_2OH$ | 4-F-3-Br | $O(CH_2)_2OH$ | 4-F-3-I |
| $O(CH_2)_2OH$ | $4\text{-}F\text{-}3\text{-}CH_3$ | $O(CH_2)_2OH$ | 4-F-3-CF3 |
| $O(CH_2)_2OH$ | 4-F-3-alkynyl | $O(CH_2)_2OH$ | 4-F-3-CN |
| $O(CH_2)_2OH$ | $4\text{-}F\text{-}3\text{-}NO_2$ | $O(CH_2)_2OH$ | 4-F-3-OCH3 |
| $O(CH_2)_2OH$ | $4\text{-}F\text{-}3\text{-}C(O)CH_3$ | $O(CH_2)_2OH$ | $4\text{-}F\text{-}3\text{-}SO_2CH_3$ |
| $O(CH_2)_2OH$ | $4\text{-}F\text{-}3\text{-}SO_2NHCH_3$ | $O(CH_2)_2OH$ | $4\text{-}F\text{-}3\text{-}CO_2CH_2CH_3$ |

(continued)

| Examples of $R^9$, $R^{11}$ in the compound of formula (**I**) suitable for being used as medical active ingredient. | | | |
|---|---|---|---|
| $R^9$ | $R^{11}$ | $R^9$ | $R^{11}$ |
| $O(CH_2)_2OH$ | $4\text{-F-3-}SO_2N(CH_3)_2$ | $O(CH_2)_2OH$ | $4\text{-F-3-}CONHCH_3$ |
| $O(CH_2)_2OH$ | 4-F-3-Cl | $O(CH_2)_2OH$ | 3-F-4-F |
| $O(CH_2)_2OH$ | H | $O(CH_2)_2OH$ | 3-COOH |
| $O(CH_2)_2OH$ | 4-COOH | $O(CH_2)_2OH$ | $2\text{-C(O)}OCH_3$ |
| $O(CH_2)_2OH$ | 2-I | $O(CH_2)_2OH$ | 2-Cl |
| $O(CH_2)_2OH$ | 2-Br | $O(CH_2)_2OH$ | 2-F |
| $O(CH_2)_2OH$ | $2\text{-}CH_3$ | $O(CH_2)_2OH$ | $2\text{-}CH_3$ |
| $O(CH_2)_2OH$ | 2-alkynyl | $O(CH_2)_2OH$ | 2-CN |
| $O(CH_2)_2OH$ | $2\text{-}NO_2$ | $O(CH_2)_2OH$ | $2\text{-}OCH_3$ |
| $O(CH_2)_2OH$ | $2\text{-C(O)}CH_3$ | $O(CH_2)_2OH$ | $2\text{-}SO_2CH_3$ |
| $CH_3$ | $4\text{-}NO_2$ | $CH_3$ | $4\text{-}OCH_3$ |
| $CH_3$ | $4\text{-C(O)}CH_3$ | $CH_3$ | $4\text{-}SO_2CH_3$ |
| $CH_3$ | $4\text{-S(O)}CH_3$ | $CH_3$ | $4\text{-}CO_2CH_3$ |
| $CH_3$ | $4\text{-}SO_2NHCH_3$ | $CH_3$ | $4\text{-}CO_2CH_2CH_3$ |

**Table 2**

Examples of $R^5$ in the compound of formula (**I**) suitable for being used as medical active ingredient

| **$R^5$** | **$R^5$** | **$R^5$** | **$R^5$** |
|---|---|---|---|
| **F** | **OH** | **$OCH_3$** | **$OCH_2CH_3$** |
| **$O(CH_2)_2OMe$** | **$O(CH_2)_3OMe$** | **$O(CH_2)_2OH$** | **OPr-n** |
| **OPr-i** | **$O(CH_2)_3OH$** | **$O(CH_2)_4OMe$** | **OBu-n** |
| **$O(CH_2)_3NMe_2$** | **$O(CH_2)_2NMe_2$** | **$O(CH_2)_3NEt_2$** | **$O(CH_2)_2$NEt2** |
| **$O(CH_2)_3$(1-morpholi n)** | **$O(CH_2)_3$(1-pyrrolinyl)** | **$O(CH_2)_2$(1-morpholin)** | **$O(CH_2)_2$(1-pyrrolinyl)** |
| **$O(CH_2)_3$(1-imidazoly l)** | **$O(CH_2)_3$(1-piperidyl)** | **$O(CH_2)_2$(1-imidazolyl)** | **$O(CH_2)_2$(1-imidazoly l)** |
| **$O(CH_2)_4$(1-morpholi n)** | **$O(CH_2)_4$(1- pyrrolyl)** | **$O(CH_2)_4$(1-imidazolyl)** | **H** |
| **$O(CH_2)_4$(1-piperidyl)** | **$CH_3$** | **$NMe_2$** | **NHC(O)Me** |
| **N(Me)C(O)Me** | **$OCF_3$** | **$OCF_2CH_3$** | |

The invention further relates to an application of a compound of formula (I),

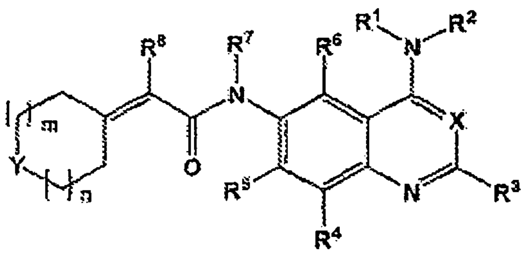

**(I)**

wherein X represents N, C-CN or CH; Y represents $CH_2$, S, O or N-$R^9$; $R^1$, $R^3$, $R^7$ and $R^8$ independently represent H, $CF_3$ or $C_{1\text{-}6}$alkyl;

$R^2$ represents a group selected from formula (**II**), (**III**), (**IV**), (**V**), (**VI**), (**VII**) or (**VIII**) ;

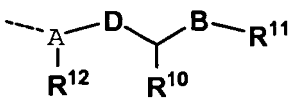
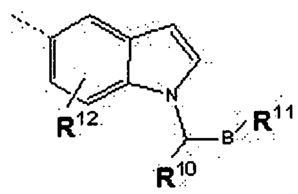
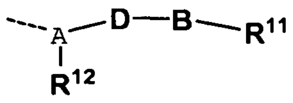

(II) (III) (IV)

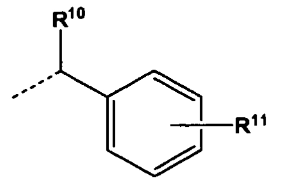
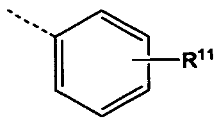
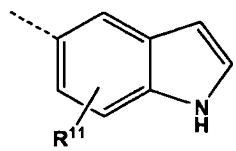
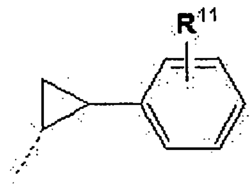

(V) (VI) (VII) (VIII)

$R^4$, $R^6$ independently represent H, $C_{1-6}$alkyl, $OC_{1-6}$alkyl, OH, F, Cl, Br, $OCF_3$ or trifluoromethyl;

$R^5$ at each occurrence is independently selected from H, F, $C_{1-6}$alkyl, OH, $OC_{1-6}$alkyl, $OCF_3$, O $(CH_2)_{2-4}OCF_3$, $OCF_2CH_3$, $NH_2$, NH ($C_{1-6}$alkyl), N $(C_{1-6}alkyl)_2$, 1- pyrrolinyl, 1- piperidyl, 4- morpholin, F, Cl, Br, trifluoromethyl, O $(CH_2)_{2-4}OC_{1-6}$alkyl, O $(CH_2)_{2-4}$NH ($C_{1-6}$alkyl), O $(CH_2)_{2-4}$N $(C_{1-6}alkyl)_2$, (1- pyrrolinyl) $(CH_2)_{2-4}$O, (1- piperidyl) $(CH_2)_{2-4}$O, (4- morpholin) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) O ($C_{1-6}$alkyl), OC (O) NH ($C_{1-6}$alkyl), OC (O) N $(C_{1-6}alkyl)_2$, (1- piperidyl) $(CH_2)_{2-4}$OC (O), (4- morpholin) $(CH_2)_{2-4}$OC (O), (1- pyrrolinyl) $(CH_2)_{2-4}$OC (O), (1- imidazolyl) $(CH_2)_{2-4}$O, (4- imidazolyl) $(CH_2)_{2-4}$OC (O), Ar $(CH_2)_{1-4}$O, (pyrazolyl) $(CH_2)_{2-4}$O or (triazolyl) $(CH_2)_{2-4}$OC (O) ;

$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-6}$alkyl), $(CH_2)_{1-4}$N $(C_{1-6}alkyl)_2$, (1- pyrrolinyl) $(CH_2)_{1-4}$, (1- piperidyl) $(CH_2)_{1-4}$, (4- morpholin) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N $(C_{1-6}alkyl)_2$, (1- pyrrolinyl) $(CH_2)_{1-6}$C (O), (1- piperidyl) $(CH_2)_{1-6}$C (O), (4- morpholin) $(CH_2)_{1-4}$C (O), C (O) $OC_{1-6}$alkyl, C (O) O $(CH_2)_{2-4}OC_{1-6}$alkyl, C (O) O $(CH_2)_{2-4}$N $(C_{1-6}alkyl)_2$, C (O) O $(CH_2)_{2-4}$NH ($C_{1-6}$alkyl), (1- pyrrolinyl) $(CH_2)_{2-4}$OC (O), (1- piperidyl) $(CH_2)_{2-4}$OC (O), (4- morpholin) $(CH_2)_{2-4}$OC (O), $(CH_2)_{1-4}$C (O) $OC_{1-6}$alkyl or Ar $(CH_2)_{1-4}$;

$R^{10}$ represents H, $C_{1-6}$ alkyl or one or two F;

$R^{11}$, $R^{12}$ independently represent H, one to four same of different groups selected from F, Cl, Br, I, CN, $NO_2$, $CF_3$, OH $NH_2$, $C_{1-4}$ alkyl, $OC_{1-4}$ alkyl, $OCF_3$, $OCF_2CH_3$, NH ($C_{1-4}$ alkyl), N $(C_{1-4}\ alkyl)_2$, OC (O) $C_{1-4}$ alkyl, NHC (O) H, NHC (O) $C_{1-4}$ alkyl, N ($C_{1-4}$ alkyl) C (O) $C_{1-4}$ alkyl, C (O) $OC_{1-4}$ alkyl, C (O) $NHC_{1-4}$ alkyl, C (O) N ($C_{1-4}$ alkyl), COOH, C (O) $C_{1-4}$ alkyl, S (O) $C_{1-4}$ alkyl, $SO_2C_{1-4}$ alkyl, $SO_2NHC_{1-4}$ alkyl or $SO_2N$ ($C_{1-4}$ alkyl) ;

A, B independently represent aromatic ring; Ar is phenyl, substituted phenyl or pyridyl; D represents O, S, NH or methylene; and m, n independently represent an integer of 0 to 4.

The application relates to treating or preventing physiological disorder caused by EGFR or Her-2 overexpression in mammals, the disorder including but not limited to breast cancer, kidney cancer, bladder cancer, oral cancer, laryngeal cancer, esophagus cancer, gastric cancer, colorectal cancer, ovarian cancer, lung cancer and head and neck cancer; besides, the application relates to treating or preventing physiological disorder by inhibiting EGFR-TK activity in mammals, the disorder including but not limited to psoriasis, pneumonia, hepatitis, nephritis, pancreatitis, diabetes.

To achieve the above objectives, in accordance with another embodiment of the invention, provided is a preparation at least comprising a compound of formula (**I**) or pharmaceutically acceptable salts or hydrates thereof,

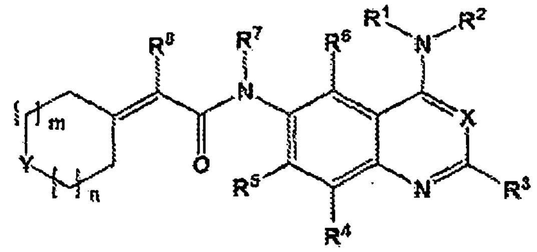

(I)

wherein X represents N, C-CN or CH; Y represents $CH_2$, S, O or N-$R^9$; $R^1$, $R^3$, $R^7$ and $R^8$ independently represent H, $CF_3$ or $C_{1-6}$ alkyl;

$R^2$ represents a group selected from formula (**II**), (**III**), (**IV**), (**V**), (**VI**), (**VII**) or (**VIII**) ;

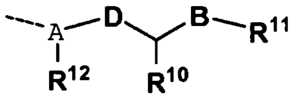

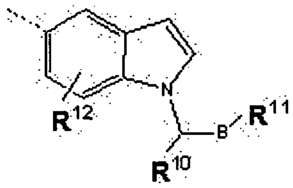

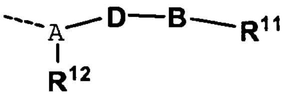

(**II**) (**III**) (**IV**)

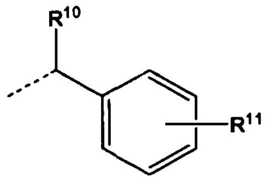

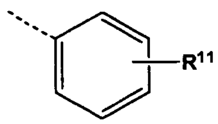

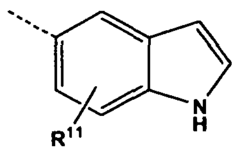

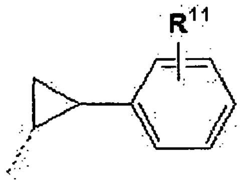

(**V**) (**VI**) (**VII**) (**VIII**)

$R^4$, $R^6$ independently represent H, $C_{1-6}$ alkyl, $OC_{1-6}$ alkyl, OH, F, Cl, Br, $OCF_3$ or trifluoromethyl;

$R^5$ at each occurrence is independently selected from H, F, $C_{1-6}$alkyl, OH, $OC_{1-6}$alkyl, $OCF_3$, O $(CH_2)_{2-4}OCF_3$, $OCF_2CH_3$, $NH_2$, NH ($C_{1-6}$alkyl), N $(C_{1-6}alkyl)_2$, 1- pyrrolinyl, 1- piperidyl, 4- morpholin, F, Cl, Br, trifluoromethyl, O $(CH_2)_{2-4}OC_{1-6}$alkyl, O $(CH_2)_{2-4}$NH ($C_{1-6}$alkyl), O $(CH_2)_{2-4}$N $(C_{1-6}alkyl)_2$, (1- pyrrolinyl) $(CH_2)_{2-4}$O, (1- piperidyl) $(CH_2)_{2-4}$O, (4- morpholin) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) O ($C_{1-6}$alkyl), OC (O) NH ($C_{1-6}$alkyl), OC (O) N $(C_{1-6}alkyl)_2$, (1- piperidyl) $(CH_2)_{2-4}$OC (O), (4- morpholin) $(CH_2)_{2-4}$OC (O), (1- pyrrolinyl) $(CH_2)_{2-4}$OC (O), (1- imidazolyl) $(CH_2)_{2-4}$O, (4- imidazolyl) $(CH_2)_{2-4}$OC (O), Ar $(CH_2)_{1-4}$O, (pyrazolyl) $(CH_2)_{2-4}$O or (triazolyl) $(CH_2)_{2-4}$OC (O) ;

$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-6}$alkyl), $(CH_2)_{1-4}$N $(C_{1-6}alkyl)_2$, (1- pyrrolinyl) $(CH_2)_{1-4}$, (1- piperidyl) $(CH_2)_{1-4}$, (4- morpholin) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N $(C_{1-6}alkyl)_2$, (1- pyrrolinyl) $(CH_2)_{1-6}$C (O), (1- piperidyl) $(CH_2)_{1-6}$C (O), (4- morpholin) $(CH_2)_{1-4}$C (O), C (O) $OC_{1-6}$alkyl, C (O) O $(CH_2)_{2-4}OC_{1-6}$alkyl, C (O) O $(CH_2)_{2-4}$N $(C_{1-6}alkyl)_2$, C (O) O $(CH_2)_{2-4}$NH ($C_{1-6}$alkyl), (1- pyrrolinyl) $(CH_2)_{2-4}$OC (O), (1- piperidyl) $(CH_2)_{2-4}$OC (O), (4- morpholin) $(CH_2)_{2-4}$OC (O), $(CH_2)_{1-4}$C (O) $OC_{1-6}$alkyl or Ar $(CH_2)_{1-4}$;

$R^{10}$ represents H, $C_{1-6}$ alkyl or one or two F;

$R^{11}$, $R^{12}$ independently represent H, 1 to 4 same of different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH ($C_{1-4}$alkyl), N $(C_{1-4}alkyl)_2$, OC (O) $C_{1-4}$alkyl, NHC (O) H, NHC (O) $C_{1-4}$alkyl, N ($C_{1-4}$alkyl) C (O) $C_{1-4}$alkyl, C (O) $OC_{1-4}$alkyl, C (O) $NHC_{1-4}$alkyl, C (O) N $(C_{1-4}alkyl)_2$, COOH, C (O) $C_{1-4}$alkyl, S (O) $C_{1-4}$alkyl, $SO_2C_{1-4}$alkyl, $SO_2NHC_{1-4}$alkyl or $SO_2$N $(C_{1-4}alkyl)_2$;

A, B independently represent aromatic ring; Ar is phenyl, substituted phenyl or pyridyl; D represents O, S, NH or methylene; and m, n independently represent an integer of 0 to 4.

The preparation at least comprises the compound of formula (**I**) or pharmaceutically acceptable salts or hydrates thereof which is used as medical active ingredient and processed with required and pharmaceutically acceptable excipients into a suitable dosage form. The administration method of the dosage form includes but is not limited to oral administration, oral containing, intravenous injection, intraperitoneal injection, subcutaneous injection, intramuscular injection, nasal drip, eye drop, inhalation, anal administration, vaginal administration or epidermal administration.

The preparation at least comprising the compound of formula (**I**) or pharmaceutically acceptable salts or hydrates thereof can treat or prevent physiological disorder caused by overexpression of EGFR or Her-2 in mammals, the disorder including but not limited to breast cancer, kidney cancer, bladder cancer, oral cancer, laryngeal cancer, esophagus cancer, gastric cancer, colorectal cancer, ovarian cancer, lung cancer, head and neck cancer, psoriasis, pneumonia, hepatitis, nephritis, pancreatitis and diabetes.

To achieve the above objectives, in accordance with still another embodiment of the invention, provided is a method of preparing a compound of formula (**I**),

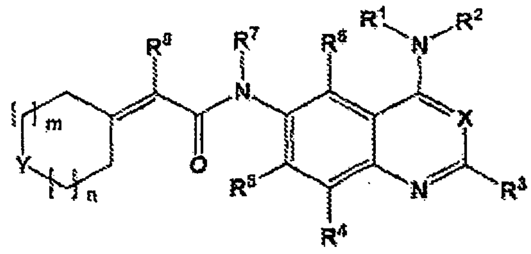

**(I)**

wherein, X represents N, C- CN or CH; Y represents $CH_2$, S, O or N- $R^9$; $R^1$, $R^3$, $R^7$ and $R^8$ independently represent H, $CF_3$ or $C_{1-6}$ alkyl;

$R^2$ represents a group selected from formula (**II**), (**III**), (**IV**), (**V**), (**VI**), (**VII**) or (**VIII**) ;

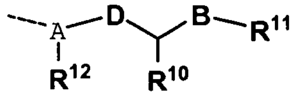

**(II)**

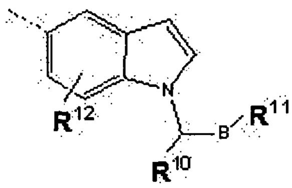

**(III)**

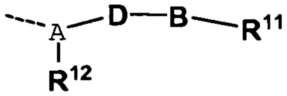

**(IV)**

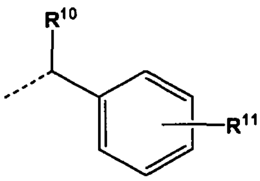

**(V)**

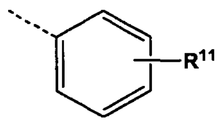

**(VI)**

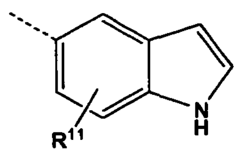

**(VII)**

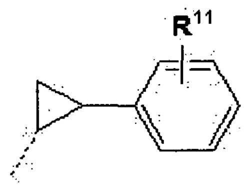

**(VIII)**

$R^4$, $R^6$ independently represent H, $C_{1-6}$ alkyl, $OC_{1-6}$ alkyl, OH, F, Cl, Br, $OCF_3$ or trifluoromethyl;

$R^5$ at each occurrence is independently selected from H, F, $C_{1-6}$alkyl, OH, $OC_{1-6}$alkyl, $OCF_3$, O $(CH_2)_{2-4}OCF_3$, $OCF_2CH_3$, $NH_2$, NH ($C_{1-6}$alkyl), N $(C_{1-6}alkyl)_2$, 1- pyrrolinyl, 1- piperidyl, 4- morpholin, F, Cl, Br, trifluoromethyl, O $(CH_2)_{2-4}OC_{1-6}$alkyl, O $(CH_2)_{2-4}$NH ($C_{1-6}$alkyl), O $(CH_2)_{2-4}$N $(C_{1-6}alkyl)_2$, (1- pyrrolinyl) $(CH_2)_{2-4}$O, (1- piperidyl) $(CH_2)_{2-4}$O, (4- morpholin) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) O ($C_{1-6}$alkyl), OC (O) NH ($C_{1-6}$alkyl), OC (O) N $(C_{1-6}alkyl)_2$, (1- piperidyl) $(CH_2)_{2-4}$OC (O), (4- morpholin) $(CH_2)_{2-4}$OC (O), (1- pyrrolinyl) $(CH_2)_{2-4}$OC (O), (1- imidazolyl) $(CH_2)_{2-4}$O, (4- imidazolyl) $(CH_2)_{2-4}$OC (O), Ar $(CH_2)_{1-4}$O, (pyrazolyl) $(CH_2)_{2-4}$O or (triazolyl) $(CH_2)_{2-4}$OC (O) ;

$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-6}$alkyl), $(CH_2)_{1-4}$N $(C_{1-6}alkyl)_2$, (1- pyrrolinyl) $(CH_2)_{1-4}$, (1- piperidyl) $(CH_2)_{1-4}$, (4- morpholin) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N $(C_{1-6}alkyl)_2$, (1- pyrrolinyl) $(CH_2)_{1-6}$C (O), (1- piperidyl) $(CH_2)_{1-6}$C (O), (4- morpholin) $(CH_2)_{1-4}$C (O), C (O) $OC_{1-6}$alkyl, C (O) O

$(CH_2)_{2-4}OC_{1-6}$alkyl, C (O) O $(CH_2)_{2-4}$N $(C_{1-6}alkyl)_2$, C (O) O $(CH_2)_{2-4}$NH $(C_{1-6}$alkyl), (1- pyrrolinyl) $(CH_2)_{2-4}$OC (O), (1-piperidyl) $(CH_2)_{2-4}$OC (O), (4- morpholin) $(CH_2)_{2-4}$OC (O), $(CH_2)_{1-4}$C (O) $OC_{1-6}$alkyl or Ar $(CH_2)_{1-4}$;

$R^{10}$ represents H, $C_{1-6}$ alkyl or one or two F;

$R^{11}$, $R^{12}$ independently represent H, 1 to 4 same of different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH $(C_{1-4}$alkyl), N $(C_{1-4}alkyl)_2$, OC (O) $C_{1-4}$alkyl, NHC (O) H, NHC (O) $C_{1-4}$alkyl, N $(C_{1-4}$alkyl) C (O) $C_{1-4}$alkyl, C (O) $OC_{1-4}$alkyl, C (O) $NHC_{1-4}$alkyl, C (O) N $(C_{1-4}alkyl)_2$, COOH, C (O) $C_{1-4}$alkyl, S (O) $C_{1-4}$alkyl, $SO_2C_{1-4}$alkyl, $SO_2NHC_{1-4}$alkyl or $SO_2N$ $(C_{1-4}alkyl)_2$;

A, B independently represent aromatic ring; Ar is phenyl, substituted phenyl or pyridyl; D represents O, S, NH or methylene; and m, n independently represent an integer of 0 to 4.

The method comprises the steps of:

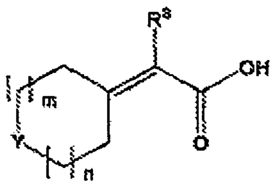

**(IX)**

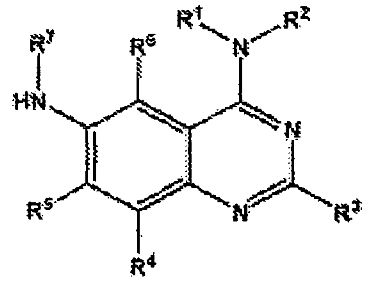

**(X)**

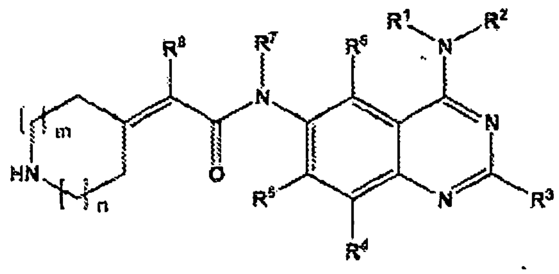

**(XII)**

1) Contacting the compound of formula (**IX**) with the compound of formula (**X**) to obtain the compound of formula (**I**) (except that Y represents NH) . The definitions of $R^1$, $R^2$, $R^3$, $R^4$, $R^5$, $R^6$, $R^7$ and $R^8$ for formula (**IX**), (**X**) are the same as those for formula (**I**) . During reaction, the compound of formula (**IX**) can be firstly transformed into active ester, acyl chloride, acyl imidazole or mixed anhydride and then contacted with the compound of formula (**X**), and tertiary amines such as triethylamine, N- methylmorpholine, trimethylamine, pyridine or substituted pyridine can be used to accelerate the reaction. When the compound of formula (**IX**) is transformed into thionyl chloride, phosphorus trichloride, phosphorus pentachloride, phosphorus oxychloride, oxalyl chloride or cyanuric chloride can be used as chlorinating agents. Optionally, the compound of formula (**IX**) can be firstly transformed into anhydride and then contacted with the compound of formula (**X**), and pyridine or substituted pyridine such as DMAP can be used as catalyst to accelerate the reaction.

2) Transforming the compound of formula (**I**) wherein Y represents $(CH_3)_3COC$ (O) N into the compound of formula (**XII**) under acidic or pyrolysis condition, wherein the definitions of $R^1$, $R^2$, $R^3$, $R^4$, $R^5$, $R^6$, $R^7$ and $R^8$ for formula (**XII**) is the same as those for formula (**I**) . The acidic condition can be trifluoroacetate acid, hydrochloric acid, sulfonic acid or an acetyl chloride plus alcohol system.

3) Contacting the compound of formula (**XII**) with $XR^9$ to obtain the compound of formula (**I**) wherein Y represents $NR^9$ and the definition of $R^9$ is the same as that for formula (I) (except that $R^9$ represents H), X represents Cl, Br, I, Oms or Ots. During reaction, organic base such as triethylamine, trimethylamine, pyridine, substituted pyridine or inorganic base such as sodium carbonate, potassium carbonate can be used to accelerate the reaction, and solvent suitable for the reaction comprises acetonitrile, dimethylformamide, dimethylacetamide, tetrahydrofuran or ethylene glycol dimethyl ether.

Inhibition experiment of cellular epidermal growth factor receptor tyrosine kinase (EGFR-TK) (following protocol described in ZD1839, a Specific epidermal growth factor receptor (EGFR) tyrosine kinase inhibitor, induces the formation of inactive EGFR/HER2 and EGFR/HER3 heterodimers and prevents heregulin signaling in HER2-overexpressing breast

cancer cells, Judit Anido, et.al. Clinical Cancer Research, 2003, 9, 1274-1283)

1) A431 cells were cultured in a medium which was prepared by adding 10% FCS into another medium comprising 50% DMEM and 50% F12.

2) The A431 cells grown in six- well plates were cultured in a serum- free medium for 24 hours, and during the 24 hour period the medium was replenished once after 12 hours.

3) A solution containing a compound to be assessed was added to the A431 cells and the cells were cultured for 2 hours, supplemented twice by a medium free of the compound, and then EGF (100 ng/ well) was added, and cultured for 5 minutes.

4) A431 cell homogenate was prepared by Laemili buffer which comprises 2% sodium dodecyl sulfonate (SDS), 5% 2- mercaptoethanol, 10% glycerol, the pH value was 6.8.

5) The A431 cell homogenate was heated for 5 minutes at 100°C.

6) Proteins in the A431 cell homogenates were separated by PAGE method and transferred to a nitrocellulose membrane, and an infrared reading was obtained.

7) Calculation formula of inhibition percent:

$$\%\ \text{inhibition} = 100 - [\text{reading by infrared reader (sample)/ reading by infrared reader (blank)}] \times 100$$

Partial one-time measurement results for a single concentration are listed in Table 3 (preliminary selection). $EC_{50}$ measurement results of some compounds are listed in Table **4.**

**Table 3**

Inhibitory activity (inhibition percent) of some compounds of Examples with concentration of 3μM against A431 cells EGFR-TK phosphorylation stimulated by EGF

| Compound | Inhibition percent(%) | Compound | Inhibition percent(% ) | Compound | Inhibition percent(% ) |
|---|---|---|---|---|---|
| Example 1 | NA | Example 3 | 85 | Example 4 | 78 |
| Example 5 | 73 | Example 6 | 24 | Example 7 | 65 |
| Example 8 | NA | Example 30 | 55 | Example 31 | 33 |
| Example 32 | 87 | Example 33 | 94 | Example 34 | 68 |
| Example 35 | 23 | Example 36 | 57 | Example 37 | 82 |
| Example 38 | 94 | Example 40 | 89 | Example 41 | 71 |
| Example 43 | 91 | Example 50 | 95 | Example 51 | 94 |
| Example 72 | 97 | Example 73 | 82 | Example 105 | 44 |
| Example 107 | 52 | Example 110 | 59 | Example 118 | 41 |
| Example 132 | 64 | Example 134 | 59 | Example 141 | 91 |
| Example 143 | 96 | Example 144 | 89 | Example 150 | 46 |

Note: NA= No activity

**Table 4**

Inhibitory activity ($EC_{50}$) of some compounds of Examples against A431 cells EGFR-TK phosphorylation stimulated by EGF

| Compound | $EC_{50}$(μM) | Compound | $EC_{50}$(μM) | Compound | $EC_{50}$(μM) |
|---|---|---|---|---|---|
| Example 3 | 0.15 | Example 4 | 0.58 | Example 7 | 0.4 |
| Example 32 | 0.09 | Example 33 | 0.038 | Example 34 | 0.76 |
| Example 36 | 0.98 | Example 41 | 0.30 | Example 65 | 0.23 |
| Example 141 | 0.28 | Example 143 | 0.16 | Example 110 | 0.9 |

Irreversible inhibition experiment of cellular epidermal growth factor receptor tyrosine kinase (EGFR-TK)

1) A431 cells were cultured in a medium which was prepared by adding 10% FCS into another medium comprising 50% DMEM and 50% F12.

2) The A431 cells grown in six- well plate were cultured in a serum- free medium for 24 hours, and during the 24 hour period the medium was replenished once after 12 hours.

3) A solution containing a compound to be assessed was added to the A431 cells and cultured for 2 hours, supplemented twice by a medium free of the compound, and then EGF (100ng/ well) was added, cultured for 5 minutes.

4) A431 cell homogenate was prepared by Laemili buffer which comprises 2% sodium dodecyl sulfonate (SDS), 5% 2- mercaptoethanol, 10% glycerol, and the pH value was 6.8.

5) The A431 cell homogenate was heated for 5 minutes at 100°C.

6) Proteins in the A431 cell homogenates were separated by PAGE method and transferred to a nitrocellulose membrane, and an infrared reading was obtained.

7) Calculation formula of inhibition percent:

$$\% \text{ inhibition} = 100 - [\text{reading by infrared reader (medicine)} / \text{reading by infrared reader (blank)}] \times 100\%$$

8) Calculation formula of recovery percent:

$$\% \text{ recovery} = 100 - \% \text{ inhibition}$$

Part of the results are listed in Table **5**.

**Table 5**

Irreversible property of some compounds of Examples against A431 cells EGFR-TK phosphorylation stimulated by EGF

| Compound | $EC_{50}$(μM) | Inhibition percent (%) | Recovery percent (%) | Property |
|---|---|---|---|---|
| **Example 32** | **0.09** | **78** | **22** | **Irreversible inhibitor** |
| **Example 33** | **0.038** | **89** | **11** | **Irreversible inhibitor** |

Following the procedure of literatures, inhibitory activity of some compounds of Examples against BT474 cells Her-2 receptor TK phosphorylation stimulated by Her-2 was measured, and part of the results are listed in Table **6**.

**Table 6**

Inhibitory activity of some compounds of Examples against BT474 cells Her-2 receptor TK phosphorylation stimulated by Her-2

| Compound | $EC_{50}$(μM) | Compound | $EC_{50}$(μM) | Compound | $EC_{50}$(μM) |
|---|---|---|---|---|---|
| **Example 43** | **0.35** | **Example 72** | **0.82** | **Example 73** | **1.7** |
| **Example 105** | **0.48** | **Example 108** | **0.18** | **Example 150** | **0.26** |

Cell growth inhibition assay(MTS Assay)

1. Cell strain and reagents

A431: human epithelial adenocarcinoma cell strain; LoVo: human colorectal cancer cell strain; BT474: breast cancer; SK-Br-3: breast cancer; SIT solution (SIGMA); RPMI1640 culture solution; phosphoric acid buffer; Dimethyl Sulphoxide (DMSO); MTS solution (Promega), 96 well cell culture plate, anti-cancer compounds.

2. Measurement

The above-mentioned cells were cultured for several days (RPMI 1640, 10% of bovine serum), collected and suspended in RPMI1640-SIT serum-free medium, implanted in 96 well cell culture plate with each well containing 20,000 cells/100 microlitre. The cells were cultured overnight under the condition of 5% $CO_2$ and 37°C. The next day, anti-cancer compounds (between 3 and 10 mM) were dissolved by Dimethyl Sulphoxide (DMSO) as mother solution, and Adriamycin was used as postive control, DMSO as negative control. According to experimental design, the mother

solution was diluted and added to the 96 hole cell culture plate, cultured for 48 hours under the condition of 5% $CO_2$ and 37°C. Subsequently, 20 mL of MTS solution was added to each hole of the 96 hole cell culture plate and cultured for another 2 to 4 hours under the condition of 5% $CO_2$ and 37°C. Absorbency was read at 490 nm wavelength, and converted into cell survival rate.

Calculation formula of inhibition percent:

$$\% \text{ inhibition} = 100 - [\text{reading of infrared reader (medicine)/ reading of infrared reader (blank)}] \times 100$$

For each concentration, there two measurements were taken and the average was obtained. Part of the results are listed in Tables **7, 8** and **9.**

**Table 7**

Growth inhibition activity ($EC_{50}$) of some compounds of Examples against A431 cells

| Compound | $EC_{50}$(μM) | Compound | $EC_{50}$(μM) | Compound | $EC_{50}$(μM) |
|---|---|---|---|---|---|
| Example 3 | 2 | Example 4 | 1.8 | Example 7 | 5 |
| Example 32 | 0.6 | Example 33 | 0.10 | Example 34 | 13 |
| Example 36 | 30 | Example 38 | 0.014 | Example 41 | 1.5 |
| Example 42 | 0.12 | Example 43 | 0.11 | Example 50 | 0.15 |
| Example 51 | 0.12 | Example 72 | 0.09 | Example 73 | 0.63 |
| Example 118 | 1.9 | Example 134 | 0.9 | Example 108 | 3.0 |
| Example 154 | 0.11 | Tarceva | 0.45 | Example 143 | 0.25 |
| | | | | Lapatinib | 1.19 |

**Table 8**

Growth inhibition activity ($EC_{50}$) (in μM) of some compounds of Examples against BT474 and SK-Br-3 cells

| Compound | BT474 | SK-Br-3 |
|---|---|---|
| Example 48 | 0.61 | 0.52 |
| Example 72 | 1.31 | 0.80 |
| Example 73 | 21.48 | 2.31 |
| Example 108 | 1.57 | 0.53 |
| Lapatinib | 0.12 | 0.07 |

**Table 9**

Growth inhibition activity ($EC_{50}$) of some compounds of Examples against colorectal cancer LoVo cells

| Compound | $EC_{50}$(μM) | Compound | $EC_{50}$(μM) | Compound | $EC_{50}$(μM) |
|---|---|---|---|---|---|
| Example 42 | 3 | Example 43 | 1.5 | Example 38 | 1.6 |
| Example 51 | 7.6 | Example 72 | 1.8 | Example 73 | 8.0 |
| Example 108 | 7.0 | Example 154 | 3.1 | Adriamycin | 1.5 |

Preparation of **1a**: tert- butyl 4- oxopiperidine- 1- carboxylate

Hydrated 4- piperidone hydrochloride (8.65 g), $BOC_2O$ (12.2 g), $NaHCO_3$ (8.8 g) and NaCl (11.2 g) were dissolved separately in a mixtureof tetrahydrofuran (80 ml) and water (80 ml), stirred at room temperature and allowed to stand overnight for layer separation. The water layer was extracted once with chloroform. The organic phases were combined, washed once with saturated salt water, dried over anhydrous magnesium sulfate and filtered. The filtrate was evaporated to give a white solid (11.35 g).

Preparation of **1b:** tert- butyl 3- oxopyrrolidine- 1- carboxylate

Following the procedure of preparing 1 a except that 4- piperidone hydrochloride was substituted with 3-oxopyrrolidine hydrochloride, the title compound was prepared.

Preparation of **1c:** 1- (2- methoxyethyl) piperidin- 4- one

Hydrated 4- piperidone hydrochloride (8.65 g), $CH_3OCH_2CH_2I$ (12.58 g) and $K_2CO_3$ (15.55 g) were dissolved separately in a mixture of tetrahydrofuran (80 ml) and water (80 ml), stirred at room temperature and allowed to stand overnight for layer separation. The water layer was extracted once with chloroform. The organic phase were combined, washed once with saturated salt water, dried over anhydrous magnesium sulfate and filtered. Finally the filtrate was evaporated in vacuo to give an oily product.

Preparation of **2a:** tert- butyl 4- (2- methoxy- 2- oxoethylidene) piperidine- 1- carboxylate

Sodium hydroxide (4.56 g, 0.114 mol) was dissolved in alcohol (210 ml), and alcohol solution containing dimethoxy phosphoryl methyl acetate (11.4g, 0.062mol) added with stirring. The mixture was stirred for 30 min at room temperature. 1- (tert- butoxy carboxyl) piperidyl- 4- one (11.35g, 0.057mol) was added with stirring at room temperature, and the reaction was allowed to stand overnight. Then, the mixture was acidized with diluted hydrochloric acid until the pH value was 4, filtered, concentrated, dissolved with water and chloroform. The chloroform phase was obtained after layer separation. The water phase was extracted once with chloroform. The chloroform phase was combined, washed once with saturated salt water, dried over anhydrous magnesium sulfate and filtered. The filtrate was evaporated in vacuo to give the title compound.

Preparation of **2b:** methyl 2- (1- (2- methoxyethyl) piperidin- 4- ylidene) acetate

Following the procedure of preparing 2a except that tert- butyl 4- oxopiperidine 1- carboxylate was substituted with (1- (2- methoxy) ethyl- 4- oxopiperidin, the title compound was prepared.

Preparation of **2c:** (E/Z)- tert- butyl 3- (2- methoxy- 2- oxoethylidene) pyrrolidine carboxylate

Following the procedure of preparing 2a except that tert-butyl 4-oxopiperidine 1-carboxylate was substituted with tert-butyl 3-oxopyrroline 1-carboxylate, the title compound was prepared.

Preparation of **3a:** 2- (1- (tert- butoxycarbonyl) piperidin- 4- ylidene) acetic acid

The above obtained tert- butyl 4- (2- methoxy- 2- oxoethylidene) piperidine- 1- carboxylate was added to tetrahydrofuran (60ml), methanol (60ml) and 1 N lithium hydroxide (60ml), with stirring at room temperature and standing overnight. Then the mixutre was extracted thrice with dichloromethane. The organic phase was removed and water phase was acidized with 1 N hydrochloric acid until the pH value was 4, extracted thrice with dichloromethane and then to combine organic phase. The organic phase was washed once with saturated salt water, dried over anhydrous magnesium sulfate and filtered. Finally the filtrate was evaporated in vacuo to give the title compound.

Preparation of **3b:** 2- (1- (2- methoxyethyl) piperidin- 4- ylidene) acetic acid

Following the procedure of preparing 3a except that tert- butyl 4- (2- methoxy- 2- oxoethylidene) piperidine- 1- carboxylate1 was substituted with methyl 2- (1- (2- methoxyethyl) piperidin- 4- ylidene) acetate, , the title compound was prepared.

Preparation of **3c:** (E)- 2- (1- (tert- butoxycarbonyl) pyrrolidin- 3- ylidene) acetic acid

Following the procedure of preparing 3a except that tert- butyl 4- (2- methoxy- 2- oxoethylidene) piperidine- 1- carboxylate was substituted with (E/Z)- tert- butyl 3- (2- methoxy- 2- oxoethylidene) pyrrolidine- 1- carboxylate, the title compound was prepared.

Preparation of **4a:** N4- (3- ethynylphenyl) quinazoline- 4, 6- diamine

2 g of iron powder was immersed in diluted hydrochloric acid for 30 min, filtered and washed with water. Subsequently, the obtained iron powder, 0.1 g of N- (3- acetylene- phenyl)- 6- nitroquinazoline- 4- amine, 25 ml of alcohol- water solution (water : alcohol = 1: 2), and 0.3 ml of acetic acid were added into a four- neck flask, with mechanical stirring and heating reflux for a hour. After reaction, the mixture was cooled to room temperature, filtered, concentrated and ethyl acetate added, washed thrice with hydrochloric acid. The hydrochloric acid layer was combined and alkalified with $Na_2CO_3$ until the pH value was 9, extracted thrice with ethyl acetate and then to combine organic phase. The organic phase was washed once with saturated salt water, dried over anhydrous magnesium sulfate and filtered. Finally the filtrate was evaporated in vacuo to give the title compound.

Following the procedure of preparing 4a, the compounds of 4b-4r were prepared.

Preparation of **4b**: $N^4$- (4- (benzyloxy)- 3- chlorophenyl) quinazoline- 4, 6- diamine

Preparation of **4c**: $N^4$- (4- (3- chlorobenzyloxy)- 3- chlorophenyl) quinazoline- 4, 6- diamine

Preparation of **4d**: $N^4$- (4- (3- bromobenzyloxy)- 3- chlorophenyl) quinazoline- 4, 6- diamine

Preparation of **4e**: $N^4$- (4- (3- methoxybenzyloxy)- 3- chlorophenyl) quinazoline- 4, 6- diamine

Preparation of **4f**: $N^4$- (4- (3- ethoxybenzyloxy)- 3- chlorophenyl) quinazoline- 4, 6- diamine

Preparation of **4g**: $N^4$- (3- chloro- 4- (pyridin- 2- ylmethoxy) phenyl) quinazoline- 4, 6- diamine

Preparation of **4h**: $N^4$- (4- (3- fluorobenzyloxy)- 3- chlorophenyl)- 7- fluoroquinazoline- 4, 6- diamine

Preparation of **4i:** $N^4$- (4- (3- fluorobenzyloxy)- 3- chlorophenyl)- 7- methoxyquinazoline- 4, 6- diamine

Preparation of **4j:** $N^4$- (4- (3- fluorobenzyloxy)- 3- chlorophenyl)- 7- ethoxyquinazoline- 4, 6- diamine

Preparation of **4k:** $N^4$- (4- (3- fluorobenzyloxy)- 3- chlorophenyl)- 7- (2- methoxyethoxy) quinazoline- 4, 6- diamine

Preparation of **4l**: $N^4$- (4- (3- chlorobenzyloxy)- 3- chlorophenyl)- 7- methoxyquinazoline- 4, 6- diamine

Preparation of **4m**: $N^4$- (3- chtoro- 4- (pyridin- 2- ylmethoxy) phenyl)- 7- methoxyquinazoline- 4, 6- diamine

Preparation of **4n**: $N^4$- (1- benzyl- 1H- indol- 5- yl) quinazoline- 4, 6- diamine

Preparation of **4o**: $N^4$- (1- (3- fluorobenzyl)- 1H- indol- 5- yl)- 7- methoxyquinazoline- 4, 6- diamine

Preparation of **4p**: $N^4$- (1- benzyl- 1H- indol- 5- yl)- 7- (2- methoxyethoxy) quinazoline- 4, 6- diamine

Preparation of **4q**: 7- ethoxy- $N^4$- (3- methoxy- 4- phenoxyphenyl) quinazoline- 4, 6- diamine

Preparation of **4r**: 7- ethoxy- $N^4$- (4- (4- fluorophenoxy)- 3- methoxyphenyl) quinazoline- 4, 6- diamine

**Example 1**

tert- butyl 4- (2- (4- (3- ethynylphenylamino) quinazolin- 6- ylamino)- 2- oxoethylidene) piperidine- 1- carboxylate

1 g of 2- (1- (tert- butyloxycarbonyl) piperidine- 4- ylidene) acetic acid was added to 20 ml of anhydrous THF in a one- neck flask (100 ml) . The solution was dissolved with stirring and cooled with salt- ice bath. Then 0.6 ml of isobutylchloroformat and 0.5 ml of N- methylmorpholine were added with stirring for 20 min. 1.046 g of $N^4$- (3- ethynyl phenyl) quinazoline- 4, 6 diamine was dissolved in 10 ml of pyridine (dried over molecular sieve) and 0.4 ml of N- methylmorpholine added. The mixture was added in the flask under ice bath with stirring. After reaction, the solvent was evaporated in vacuo and separated with chloroform and water. The chloroform layer was washed once with saturated salt water, dried over anhydrous magnesium sulfate, filtered, evaporated in vacuo to give a crude product which was recrystalled from isopropanol, MS (electron impact) $482M^+$.

**Example 2**

N- (4- (3- ethynylphenylamino) quinazolin- 6- yl)- 2- (piperidin- 4- ylidene) acetamide trifluoroacetate

Tert- buty- ((4- (3- ethynylphenylamino) quinazolin- 6- yl- aminocarbonyl)- methylene) pipe ridine- 1- carboxylic ester (92 mg, 0.38 mmol) was dissovled in 10 ml of 20% anhydrous TFA/DCM solution and stirred at room temperature for 2 hours, evaporated in vacuo, vacuum dried to give a whitish foam solid, MS: 384 (M+1) .

**Example 3**

N- (4- (3- ethynylphenylamino) quinazolin- 6- yl)- 2- (piperidin- 4- ylidene) acetamide

N- (4- (3- ethynylphenylamino) quinazolin- 6- yl)- 2- (piperidin- 4- ylidene) acetamide trifluoroacetate was dissolved in ethyl acetate. The mixture was washed once separately with saturated $Na_2CO_3$ and saturated salt water, dried over anhydrous magnesium sulfate, filtered, evaporated in vacuo to give a product, MS (electron impact) 384 (M+1) .

**Example 4**

N- (4- (3- ethynylphenylamino) quinazolin- 6- yl)- 2- (1- methylpiperidin- 4- ylidene) acetamide

N- (4- (3- ethynylphenylamino) quinazolin- 6- yl)- 2- (piperidin- 4- ylidene) acetamide (20 mg, 0.046 mmol), methyl iodide (8.0 mg, 0.056 mmol), anhydrous potassium carbonate (17 mg) and acetonitrile (5 ml) were added in a one- neck flask (50 ml), with stirring at room temperature for 24 hours. After reaction, the solution was filtered, evaporated in vacuo to give a solid. The solid was purified with TLC (silica gel plate with a thickness of 500 $\mu$m, developer: chloroform to methanol is 95: 5), MS: 398 (M+1) .

Following the procedure of Example **4**, the compounds of Example **5-8** were prepared.

**Example 5**

2- (1- ethylpiperidin- 4- ylidene)- N- (4- (3- ethynylphenylamino) quinazolin- 6- yl) acetamide MS: 412 (M+1)

**Example 6**

2- (1- benzylpiperidin- 4- ylidene)- N- (4- (3- ethynylphenylamino) quinazolin- 6- yl) acetamide MS: 474 (M+1)

**Example 7**

N- (4- (3- ethynylphenylamino) quinazolin- 6- yl)- 2- (1- (2- methoxyethyl) piperidin- 4- yliden e) acetamide MS: 440 (M- 1)

**Example 8**

Methyl 2- (4- (2- (4- (3- ethynylphenylamino) quinazolin- 6- ylamino)- 2- oxoethylidene) piperidin- 1- yl) acetate MS: 454 ($M^+$)

**Example 9**

N- (4- (3- ethynylphenylamino) quinazolin- 6- yl)- 2- (1- isopropylpiperidin- 4- ylidene) aceta mide MS: 426 (M+1)

**Example 10**

N- (4- (3- ethynylphenylamino) quinazolin- 6- yl)- 2- (1- (2- hydroxyethyl) piperidin- 4- ylidene ) acetamide MS: 428 (M+1)

Following the procedure of Example **1**, the compounds of Example **11-29** were prepared.

**Example 11**

tert- butyl 4- (2- oxo- 2- (4- (phenylamino) quinazolin- 6- ylamino) ethylidene) piperidine- 1- arboxylate MS: 459 (M+)

**Example 12**

tert- butyl 4- (2- (4- (3- chloro- 4- fluorophenylamino) quinazolin- 6- ylamino)- 2- oxoethylidene) piperidine- 1- carboxylate MS: 511 ($M^+$)

**Example 13**

tert- butyl 4- (2- (4- (3- bromophenylamino) quinazolin- 6- ylamino)- 2- oxoethylidene) piperidine- 1- carboxylate MS: 539 (M+1)

**Example 14**

(S)- tert- butyl 4- (2- oxo- 2- (4- (1- phenylethylamino) quinazolin- 6- ylamino) ethylidene) piperidine- 1- carboxylate MS: 487 (M+)

**Example 15**

(*R*)- tert- butyl 4- (2- oxo- 2- (4- (1- phenylethylamino) quinazolin- 6- ylamino) ethylidene) piperidine- 1- carboxylate MS: 487 (M+)

**Example 16**

tert- butyl 4- (2- (4- (3- chlorophenylamino) quinazolin- 6- ylamino)- 2- oxoethylidene) piperidine- 1- carboxylate MS: 493 ($M^+$)

**Example 17**

tert- butyl 4- (2- (4- (3- chlorophenylamino)- 7- fluoroquinazolin- 6- ylamino)- 2- oxoethylidene) piperidine- 1- carboxylate MS: 511 ($M^+$)

**Example 18**

tert- butyl 4- (2- (4- (3- bromophenylamino)- 7- methoxyquinazolin- 6- ylamino)- 2- oxoethylidene) piperidine- 1- carboxylate MS: 568 (M+1)

**Example 19**

tert- butyl 4- (2- (4- (3- bromophenylamino)- 7- ethoxyquinazolin- 6- ylamino)- 2- oxoethylidene) piperidine- 1-carboxylate MS: 582 (M+1)

**Example 20**

tert- butyl 4- (2- (4- (3- bromophenylamino)- 7- (2- methoxyethoxy) quinazolin- 6- ylamino)- 2- oxoethylidene) piperidine- 1- carboxylate MS: 612 (M+1)

**Example 21**

tert- butyl 4- (2- (4- (1 H- indol- 5- ylamino) quinazolin- 6- ylamino)- 2- oxoethylidene) piperidine- 1- carboxylate MS: 498 ($M^+$)

**Example 22**

tert- butyl 4- (2- (4- (3- ethynylphenylamino)- 7- methoxyquinazolin- 6- ylamino)- 2- oxoethylidene) piperidine- 1- carboxylate MS: 512 ($M^+$)

**Example 23**

tert- butyl 4- (2- (4- (3- chloro- 4- fluorophenylamino)- 7- methoxyquinazolin- 6- ylamino)- 2- oxoethylidene) piperidine- 1- carboxylate MS: 541 ($M^+$)

**Example 24**

tert- butyl 4- (2- (4- (1 H- indol- 5- ylamino)- 7- methoxyquinazolin- 6- ylamino)- 2- oxoethylidene) piperidine- 1- carboxylate MS: 528 ($M^+$)

**Example 25**

tert- butyl 4- (2- (4- (3- bromophenylamino)- 7- (3- methoxypropoxy) quinazolin- 6- ylamino)- 2- oxoethylidene) piperidine- 1- carboxylate MS: 626 ($M^+$)

**Example 26**

tert- butyl 4- (2- (4- (3- bromophenylamino)- 7- (3- morpholinopropoxy) quinazolin- 6- ylamino)- 2- oxoethylidene) piperidine- 1- carboxylate MS: 681 ($M^+$)

**Example 27**

tert- butyl 4- (2- (4- (3- ethynylphenylamino)- 7- (2- methoxyethoxy) quinazolin- 6- ylamino)- 2- oxoethylidene) piperidine- 1- carboxylate 27 MS: 557 (M+1)

**Example 28**

tert- butyl 4- (2- (4- (3- chloro- 4- fluorophenylamino)- 7- (2- methoxyethoxy) quinazolin- 6- ylamino)- 2- oxoethylidene) piperidine- 1- carboxylate MS: 585 ($M^+$)

**Example 29**

tert- butyl 4- (2- (4- (1 H- indol- 5- ylamino)- 7- (2- methoxyethoxy) quinazolin- 6- ylamino)- 2- oxoethylidene) piperidine- 1- carboxylate MS: 572 ($M^+$)

Following the procedure of Example 2, 3, the compounds of Example **30-50** were prepared.

**Example 30**

N- (4- (3- chlorophenylamino)- 7- fluoroquinazolin- 6- yl)- 2- (piperidin- 4- ylidene) acetamid e MS: 411 (M+1)

**Example 31**

N- (4- (phenylamino) quinazolin- 6- yl)- 2- (piperidin- 4- ylidene) acetamide MS: 359 (M+1)

**Example 32**

N- (4- (3- chloro- 4- fluorophenylamino) quinazolin- 6- yl)- 2- (piperidin- 4- ylidene) acetamid e MS: 410 ($M^+$)

**Example 33**

N- (4- (3- bromophenylamino) quinazolin- 6- yl)- 2- (piperidin- 4- ylidene) acetamide MS: 438 ($M^+$)

**Example 34**

(S)- N- (4- (1- phenylethylamino) quinazolin- 6- yl)- 2- (piperidin- 4- ylidene) acetamide MS: 387 (M+)

**Example 35**

(R)- N- (4- (1- phenylethylamino) quinazolin- 6- yl)- 2- (piperidin- 4- ylidene) acetamide MS: 387 (M+)

**Example 36**

N- (4- (3- chlorophenylamino) quinazolin- 6- yl)- 2- (piperidin- 4- ylidene) acetamide MS: 393 ($M^+$)

**Example 37**

N- (4- (3- bromophenylamino)- 7- (3- morpholinopropoxy) quinazolin- 6- yl)- 2- (piperidin- 4- ylidene) acetamide MS: 581 ($M^+$)

**Example 38**

N- (4- (3- bromophenylamino)- 7- methoxyquinazolin- 6- yl)- 2- (piperidin- 4- ylidene) acetamide MS: 467 ($M^+$)

**Example 39**

N- (4- (3- bromophenylamino)- 7- ethoxyquinazolin- 6- yl)- 2- (piperidin- 4- ylidene) acetami de MS: 482 ($M^+$)

**Example 40**

N- (4- (3- bromophenylamino)- 7- (2- methoxyethoxy) quinazolin- 6- yl)- 2- (piperidin- 4- ylide ne) acetamide MS: 512 (M+1)

**Example 41**

N- (4- (1 H- indol- 5- ylamino) quinazolin- 6- yl)- 2- (piperidin- 4- ylidene) acetamide MS: 398 (M+1)

**Example 42**

N- (4- (3- ethynylphenylamino)- 7- methoxyquinazolin- 6- yl)- 2- (piperidin- 4- ylidene) aceta mide MS: 413 ($M^+$)

**Example 43**

N- (4- (3- chloro- 4- fluorophenylamino)- 7- methoxyquinazolin- 6- yl)- 2- (piperidin- 4- yliden e) acetamide MS: 441 (M+1)

**Example 44**

N- (4- (1H- indol- 5- ylamino)- 7- methoxyquinazolin- 6- yl)- 2- (piperidin- 4- ylidene) acetamide MS: 428 (M+1)

**Example 45**

(S)- N- (7- methoxy- 4- (1- phenylethylamino) quinazolin- 6- yl)- 2- (piperidin- 4- ylidene) acet amide MS: 417 (M+)

**Example 46**

(R)- N- (7- methoxy- 4- (1- phenylethylamino) quinazolin- 6- yl)- 2- (piperidin- 4- ylidene) acet amide MS: 417 (M+)

**Example 47**

N- (4- (3- ethynylphenylamino)- 7- (2- methoxyethoxy) quinazolin- 6- yl)- 2- (piperidin- 4- ylid ene) acetamide MS: 457 ($M^+$)

**Example 48**

N- (4- (3- chloro- 4- fluorophenylamino)- 7- (2- methoxyethoxy) quinazolin- 6- yl)- 2- (piperidi n- 4- ylidene) acetamide MS: 485.5 ($M^+$)

**Example 49**

N- (4- (3- ethynylphenylamino)- 7- (3- morpholinopropoxy) quinazolin- 6- yl)- 2- (piperidin- 4- ylidene) acetamide MS: 526 (M+1)

**Example 50**

N- (4- (3- ethynylphenylamino)- 7- (3- methoxypropoxy) quinazolin- 6- yl)- 2- (piperidin- 4- yli dene) acetamide MS: 471 (M+1)

Following the procedure of Example **4,** the compounds of Example **51-83** were prepared.

**Example 51**

N- (4- (3- ethynylphenylamino)- 7- methoxyquinazolin- 6- yl)- 2- (1- methylpiperidin- 4- ylide ne) acetamide MS: 427 (M+1)

**Example 52**

N- (7- ethoxy- 4- (3- ethynylphenylamino) quinazolin- 6- yl)- 2- (1- ethylpiperidin- 4- ylidene) a cetamide MS: 455 ($M^+$)

**Example 53**

2- (1- ethylpiperidin- 4- ylidene)- N- (4- (3- ethynylphenylamino)- 7- methoxyquinazolin- 6- yl ) acetamide MS: 441 ($M^+$)

**Example 54**

N- (7- ethoxy- 4- (3- ethynylphenylamino) quinazolin- 6- yl)- 2- (1- methylpiperidin- 4- ylidene ) acetamide MS: 441 ($M^+$)

**Example 55**

N- (4- (3- bromophenylamino) quinazolin- 6- yl)- 2- (1- methylpiperidin- 4- ylidene) acetamid e MS: 452 ($M^+$)

**Example 56**

N- (4- (3- bromophenylamino) quinazolin- 6- yl)- 2- (1- ethylpiperidin- 4- ylidene) acetamide MS: 466 (M+1)

**Example 57**

N- (4- (3- bromophenylamino) quinazolin- 6- yl)- 2- (1- (2- methoxyethyl) piperidin- 4- ylidene ) acetamide MS: 496 (M+1)

**Example 58**

N- (4- (3- bromophenylamino)- 7- methoxyquinazolin- 6- yl)- 2- (1- methylpiperidin- 4- yliden e) acetamide MS: 482 (M+1)

**Example 59**

N- (4- (3- bromophenylamino)- 7- methoxyquinazolin- 6- yl)- 2- (1- ethylpiperidin- 4- ylidene) acetamide MS: 496 (M+1)

**Example 60**

N- (4- (3- bromophenylamino)- 7- methoxyquinazolin- 6- yl)- 2- (1- (2- methoxyethyl) piperidi n- 4- ylidene) acetamide MS: 526 ($M^+$)

**Example 61**

N- (4- (3- bromophenylamino)- 7- methoxyquinazolin- 6- yl)- 2- (1- (3- methoxypropyl) piperi din- 4- ylidene) acetamide MS: 540 ($M^+$)

**Example 62**

N- (4- (3- bromophenylamino)- 7- ethoxyquinazolin- 6- yl)- 2- (1- methylpiperidin- 4- ylidene) acetamide MS: 496 (M+1)

**Example 63**

N- (4- (3- bromophenylamino)- 7- ethoxyquinazolin- 6- yl)- 2- (1- ethylpiperidin- 4- ylidene) a cetamide MS: 510 (M+)

**Example 64**

N- (4- (3- bromophenylamino)- 7- ethoxyquinazolin- 6- yl)- 2- (1- (2- methoxyethyl) piperidin- 4- ylidene) acetamide MS: 540 (M+)

**Example 65**

N- (4- (3- bromophenylamino)- 7- (2- methoxyethoxy) quinazolin- 6- yl)- 2- (1- methylpiperidi n- 4- ylidene) acetamide MS: 526 ($M^+$)

**Example 66**

N- (4- (3- bromophenylamino)- 7- (3- methoxypropoxy) quinazolin- 6- yl)- 2- (1- methylpiperi din- 4- ylidene) acetamide MS: 540 ($M^+$)

**Example 67**

N- (4- (3- bromophenylamino)- 7- (3- (dimethylamino) propoxy) quinazolin- 6- yl)- 2- (1- meth ylpiperidin- 4- ylidene) acetamide MS: 552 ($M^+$)

**Example 68**

N- (4- (3- bromophenylamino)- 7- (3- morpholinopropoxy) quinazolin- 6- yl)- 2- (1- methylpip eridin- 4- ylidene)acetamide MS: 595 (M+1)

**Example 69**

N- (4- (3- chlorophenylamino)- 7- fluoroquinazolin- 6- yl)- 2- (1- methylpiperidin- 4- ylidene) a cetamide MS: 425 ($M^+$)

**Example 70**

N- (4- (3- chlorophenylamino)- 7- fluoroquinazolin- 6- yl)- 2- (1- ethylpiperidin- 4- ylidene) ac etamide MS: 439 ($M^+$)

**Example 71**

N- (4- (3- chlorophenylamino)- 7- fluoroquinazolin- 6- yl)- 2- (1- (2- methoxyethyl) piperidin- 4- ylidene) acetamide MS: 469 ($M^+$)

**Example 72**

N- (4- (3- chloro- 4- fluorophenylamino)- 7- methoxyquinazolin- 6- yl)- 2- (1- methylpiperidin- 4- ylidene) acetamide MS: 455 ($M^+$)

**Example 73**

N- (4- (3- chloro- 4- fluorophenylamino)- 7- methoxyquinazolin- 6- yl)- 2- (1- (2- methoxyethy l) piperidin- 4- ylidene) acetamide MS: 499 ($M^+$)

**Example 74**

N- (4- (3- chloro- 4- fluorophenylamino) quinazolin- 6- yl)- 2- (1- (2- methoxyethyl) piperidin- 4- ylidene) acetamide MS: 469 ($M^+$)

**Example 75**

N- (4- (1H- indol- 5- ylamino) quinazolin- 6- yl)- 2- (1- methylpiperidin- 4- ylidene) acetamide MS: 412 (M+1)

**Example 76**

N- (4- (1H- indol- 5- ylamino) quinazolin- 6- yl)- 2- (1- ethylpiperidin- 4- ylidene) acetamide MS: 426 (M+1)

**Example 77**

N- (4- (1H- indol- 5- ylamino) quinazolin- 6- yl)- 2- (l- (2- methoxyethyl) piperidin- 4- ylidene) a cetamide MS: 456 ($M^+$)

**Example 78**

(S)- 2- (1- methylpiperidin- 4- ylidene)- N- (4- (1- phenylethylamino) quinazolin- 6- yl) acetam ide MS: 401 (M+)

**Example 79**

(S)- 2- (1- ethylpiperidin- 4- ylidene)- N- (4- (1- phenylethylamino) quinazolin- 6- yl) acetamid e MS: 415 (M+)

**Example 80**

(S)- 2- (1- (2- methoxyethyl) piperidin- 4- ylidene)- N- (4- (1- phenylethylamino) quinazolin- 6- yl) acetamide MS: 445 (M+)

**Example 81**

(S)- N- (7- (2- methoxyethoxy)- 4- (1- phenylethylamino) quinazolin- 6- yl)- 2- (piperidin- 4- yli dene) acetamide MS: 537 (M+)

**Example 82**

N- (4- (1 H- indol- 5- ylamino)- 7- (2- methoxyethoxy) quinazolin- 6- yl)- 2- (piperidin- 4- yliden e) acetamide MS: 472 (M+1)

**Example 83**

N- (4- (3- bromophenylamino) quinazolin- 6- yl)- 2- (pyrrolidin- 3- ylidene) acetamide MS: 423 (M+1)

**Example 84**

Comparison compound **A:** N- (4- (3- bromophenylamino) quinazolin- 6- yl) propionamide [0243] *N*$^4$- (3- acetylenylphenyl) quinazoline- 4, 6- diamine (100 mg, 0.32 mmol) pyridine (0.3 ml) and DMAP (20 mg) were dissolved in 10 ml of anhydrous THF solution and added in a reaction flask dropwise. The solution was cooled to 5°C, propionyl chloride (33 mg, 0.35 mmol) added, ice bath removed, strired at room temperature and filtered. The filtrate was evaporated in vacuo to give a yellow solid which was dissolved with ethyl acetate, washed once separately with saturated $Na_2CO_3$, 10% acetic acid and saturated salt water. The organic phase was dried, filtered, spin- dried to give a crude product which was purified with TLC to give a whitish product.

**Example 85**

Comparison compound **B**: N- (4- (3- bromophenylamino) quinazolin- 6- yl) acrylamide [0245] Following the procedure of Example **84** except that propionyl chloride was substituted with acryloyl chloride, the title compound was prepared.

**Example 86**

Comparison compound **C:** N- (4- (3- bromophenylamino) quinazolin- 6- yl)- 3- methylbut- 2- enamide

Following the procedure of Example **84** except that propionyl chloride was substituted with 3- methyl- butyl- 2- en- acyl chloride, the title compound was prepared.

**Example 87**

N- (7- methoxy- 4- (2- phenylcyclopropylamino) quinazolin- 6- yl)- 2- (1- (2- methoxyethyl) pi peridin- 4- ylidene) acetamide

1) Preparation of 2- (1- (2- methoxyethyl) piperidin- 4- ylidene) acetyl chloride hydrochloride

2.4 g of 2- (1- (2- methoxyethyl) piperidin- 4- ylidene) acetic acid was dissolved in 20 ml of thionyl chloride, refluxed for 2 hours, evaporated under reduced pressure to remove thionyl chloride and give a solid product.

2) Preparation of N- (7- methoxy- 4- (2- phenylcyclopropylamino) quinazolin- 6- yl)- 2- (1- (2- methoxyethyl) piperidin- 4- ylidene) acetamide

Following the procedure of Example **84,** the title compound was prepard by contacting 2- (1- (2- methoxyethyl) piperidin- 4- ylidene) acetyl chloride hydrochloride with N$^4$- (7- methoxy- 4- (2- phenylcyclopropylamino) quinazolin- 4, 6- diamine.

**Example 88**

N$^1$- (4- (3- bromophenylamino) quinazolin- 6- yl)- N$^4$- (2- (2- (dimethylamino) ethoxy) ethyl) f umaramide

79 mg of N$^1$- (4- (3- bromophenyl) quinazolin- 4, 6- diamine, 39 mg of maleic anhydride and 15 ml of THF were added in a one- neck flask (50 ml), refluxed. After reaction, the solution was evaporated in vacuo and purified by thin layer chromatography. The pure product was dissolved with anhydrous THF, amino ethoxy alcohol added and cooled in ice bath. Subsequently, THF solution containg DCC was added dropwise, ice bath removed and heating reflux was followed for a day. After reation, the solution was cooled to room temperature, filtered, evaporated in vacuo to give a crude product. 105 mg of the crude product was dissolved with 20 ml of pyridine, 400 mg of p- methyl benzene sulfonic

chloride added with stirring at room temperature. After reaction, the solvent was removed and the residue dissolved with ethyl acetate, washed once separately with saturated $Na_2CO_3$, 1N HCl and saturated NaCl, dried over anhydrous magnesium sulfate, filtered, evaporated in vacuo to give a product. The product was dissolved with 10 ml of pyridine, dimethylamine added with stirring at room temparature. After reaction, the solvent was evaporated in vacuo and purified by thin layer chromatography, MS (electron impact) 528 $M^+$.

**Example 89**

N- (4- (3- bromophenylamino) quinazolin- 6- yl)- 2- (1- (2- (2- (2- hydroxyethoxy) ethylamino) acetyl) piperidin- 4- ylidene) acetamide

40 mg of N- (4- (3- bromophenylamino) quinazolin- 6- yl)- 2- (piperidine- 4- ylidene) acetamide and 10 ml of THF were added in a one- neck flask (50 ml), cooled in ice bath, 0.01 ml of chloroacetic chloride and 0.02 ml of triethylamine (dried over molecular sieve) added with stirring at room temperature. After reaction, the solvent was evaporated in vacuo, the residue was dissolved with ethyl acetate, washed thrice with water and once with saturated salt water, dried over anhydrous magnesium sulfate, filtered, evaporated in vacuo to give a crude product (30 mg) . The crude product was dissolved with 10 ml of acetonitrile, 7.3 mg (0.07 mmol) of amine ethoxyl alcohol and 0.02 ml of triethylamine (dried over molecular sieve) added with stirring at room temperature. After reaction, the solvent was evaporated in vacuo and purified by thin layer chromatography, MS (electron impact) 581 $M^+$.

Following the procedure of literatures, the compounds of Example **90-101** were prepared.

**Example 90**

N4- (4- (3- fluorobenzyloxy)- 3- chlorophenyl)- 7- (3- morpholinopropoxy) quinazoline- 4, 6- diamine

**Example 91**

$N^4$- (4- (3- fluorobenzyloxy)- 3- chlorophenyl)- 7- (3- methoxypropoxy) quinazoline- 4, 6- dia mine

**Example 92**

$N^4$- (3- chloro- 4- (pyridin- 2- ylmethoxy) phenyl)- 7- ethoxyquinazoline- 4, 6- diamine

**Example 93**

$N^4$- (3- chloro- 4- (pyridin- 2- ylmethoxy) phenyl)- 7- methoxyquinazoline- 4, 6- diamine

**Example 94**

N4- (3- chloro- 4- (pyridin- 2- ylmethoxy) phenyl)- 7- fluoroquinazoline- 4, 6- diamine

**Example 95**

N4- (3- chloro- 4- (pyridin- 2- ylmethoxy) phenyl) quinazoline- 4, 6- diamine

**Example 96**

7- methoxy- $N^4$- (3- methoxy- 4- phenoxyphenyl) quinazoline- 4, 6- diamine

**Example 97**

7- methoxy- $N^4$- (4- (3- methoxyphenoxy) phenyl) quinazoline- 4, 6- diamine

**Example 98**

$N^4$- (2- chloro- 4- (pyridin- 2- ylmethoxy) phenyl)- 7- methoxyquinazoline- 4, 6- diamine

**Example 99**

$N^4$- (4- (benzyloxy)- 3- chlorophenyl)- 7- methoxyquinazoline- 4, 6- diamine

**Example 100**

$N^4$- (4- (3- chlorobenzyloxy)- 3- fluorophenyl)- 7- methoxyquinazoline- 4, 6- diamine

**Example 101**

$N^4$- (4- (3- chloro- 4- fluorobenzyloxy) phenyl)- 7- methoxyquinazoline- 4, 6- diamine

**Example 102**

tert- butyl 4- (2- (4- (3- chloro- 4- (pyridin- 2- ylmethoxy) phenylamino) quinazolin- 6- ylamino)- 2- oxoethylidene) piperidine- 1- carboxylate

1 g of 2- (1- (tert- butyloxycarbonyl) piperidine- 4- ylidene) acetic acid, 20 ml of anhydrous THF were added in a one- neck flask (100 ml), dissolved with stirring and cooled with salt- ice bath, then 0.6 ml of isobutylchloroformat and 0.5 ml of N- methylmorpholine added, stirring for 20 mins. 1.046 g of $N^4$- (4- 3- chloro- (pyridin- 2- ylmethoxy) phenyl) quinazoline- 4, 6 diamine was dissolved with 10 ml of pyridine (dried over molecular sieve), 0.4 ml of N- methylmorpholine added, and the mixture was added to the flask under ice bath with stirring. After reaction, the solvent was evaporated in vacuo and partitioned with chloroform and water. The chloroform layer was washed once with saturated salt water, dried over anhydrous magnesium sulfate, filtered, evaporated in vacuo to give a crude product which was recrystallized from isopropanol, MS (electron impact) 601 M+.

**Example 103**

N- (4- (3- chloro- 4- (pyridin- 2- ylmethoxy) phenylamino) quinazolin- 6- yl)- 2- (piperidin- 4- yli dene) acetamide trifluoroacetate

tert- butyl- 4- ((4- (3- chloro- 4- (pyridin- 2- ylmethoxy) phenylamino) quinazolin- 6- yl- aminocarbonyl)- methylene) piperidine- 1- carboxylic ester (92 mg, 0.38 mmol) was dissovled with 10 ml of 20% anhydrous TFA/DCM solution with stirring at room temperature for 2 hours, evaporated in vacuo, vacuum dried to give a whitish foam solid, MS: 501 (M+1) .

**Example 104**

N- (4- (3- chloro- 4- (pyridin- 2- ylmethoxy) phenylamino) quinazolin- 6- yl)- 2- (piperidin- 4- yli dene) acetamide

N- (4- (3- chloro- 4- (pyridin- 2- ylmethoxy) phenylamino) quinazolin- 6- yl)- 2- (piperidin- 4- yli dene) acetamide trifluoroacetate was dissolved with ethyl acetate, washed once with saturated $Na_2CO_3$, and once with saturated salt water, dried over anhydrous magnesium sulfate, filtered, evaporated in vacuo to give a product, MS (electron impact) 501 (M+1) .

Following the procedure of Example **104**, the compounds of **105-115** were prepared.

**Example 105**

N- (4- (4- (3- fluorobenzyloxy)- 3- chlorophenylamino)- 7- methoxyquinazolin- 6- yl)- 2- (pipe ridin- 4- ylidene) acetamide MS: 548 (M+1)

**Example 106**

N- (4- (4- (3- fluorobenzyloxy)- 3- chlorophenylamino)- 7- (3- methoxypropoxy) quinazolin- 6- yl)- 2- (piperidin- 4- ylidene) acetamide MS: 605 (M+)

**Example 107**

N- (4- (3- chloro- 4- (pyridin- 2- ylmethoxy) phenylamino)- 7- ethoxyquinazolin- 6- yl)- 2- (pipe ridin- 4- ylidene) acetamide MS: 544 ($M^+$)

**Example 108**

N- (4- (3- chloro- 4- (pyridin- 2- ylmethoxy) phenylamino)- 7- methoxyquinazolin- 6- yl)- 2- (pi peridin- 4- ylidene) acetamide MS: 531 (M+1)

**Example 109**

N- (4- (3- chloro- 4- (pyridin- 2- ylmethoxy) phenylamino)- 7- fluoroquinazolin- 6- yl)- 2- (piper idin- 4- ylidene) acetamide MS: 519 (M+1)

**Example 110**

N- (7- methoxy- 4- (3- methoxy- 4- phenoxyphenylamino) quinazolin- 6- yl)- 2- (piperidin- 4- y lidene) acetamide MS: 512 (M+1)

**Example 111**

N- (7- methoxy- 4- (4- (3- methoxyphenoxy) phenylamino) quinazolin- 6- yl)- 2- (piperidin- 4- ylidene) acetamide MS: 511 ($M^+$)

**Example 112**

N- (4- (2- chloro- 4- (pyridin- 2- ylmethoxy) phenylamino)- 7- methoxyquinazolin- 6- yl)- 2- (pi peridin- 4- ylidene) acetamide MS: 531 (M+1)

**Example 113**

N- (4- (4- (benzyloxy)- 3- chlorophenylamino)- 7- methoxyquinazolin- 6- yl)- 2- (piperidin- 4- ylidene) acetamide MS: 531 (M+1)

**Example 114**

N- (4- (4- (3- chlorobenzyloxy)- 3- fluorophenylamino)- 7- methoxyquinazolin- 6- yl)- 2- (pipe ridin- 4- ylidene) acetamide MS: 547 ($M^+$)

**Example 115**

N- (4- (4- (3- chloro- 4- fluorobenzyloxy) phenylamino)- 7- methoxyquinazolin- 6- yl)- 2- (pipe ridin- 4- ylidene) acetamide MS: 548 (M+1)

**Example 116**

N- (4- (3- chloro- 4- (pyridin- 2- ylmethoxy) phenylamino) quinazolin- 6- yl)- 2- (1- methylpiper idin- 4- ylidene) acetamid

N- (4- (3- chloro- 4- (pyridin- 2- ylmethoxy) phenylamino) quinazolin- 6- yl)- 2- (1- piperidin- 4- ylidene) acetamide (20 mg, 0.046mmol), methyl iodide (8.0 mg, 0.056 mmol), anhydrous potassium carbonate (17 mg) and acetonitrile (5 ml) were added in a one- neck flask (50 ml), and stirred at room temperature for 24 hours. After reaction, the solution was filtered, evaporated in vacuo to give a solid. The solid was purified with TLC (silica gel plate with a thickness of 500 μm, developer: chloroform to methanol is 95: 5), MS: 515M+

Following the procedure of Example **116**, the compounds of Example **117-118** were prepared.

**Example 117**

N- (4- (3- chloro- 4- (pyridin- 2- ylmethoxy) phenylamino)- 7- methoxyquinazolin- 6- yl)- 2- (1- ethylpiperidin- 4- ylidene) acetamide MS: 558 ($M^+$)

**Example 118**

N- (4- (3- chloro- 4- (pyridin- 2- ylmethoxy) phenylamino)- 7- methoxyquinazolin- 6- yl)- 2- (1- (2- methoxyethyl) piperidin- 4- ylidene) acetamide MS: 588 ($M^+$)
Following the procedure of literatures, the compouds of Example **119-131** were preapred.

**Example 119**

6- amino- 4- (3- chlorophenylamino)- 7- methoxyquinoline- 3- carbonitrile

**Example 120**

6- amino- 4- (3- chloro- 4- fluorophenylamino)- 7- methoxyquinoline- 3- carbonitrile

**Example 121**

6- amino- 4- (3- ethynylphenylamino)- 7- methoxyquinoline- 3- carbonitrile

**Example 122**

6- amino- 4- (3- bromophenylamino)- 7- methoxyquinoline- 3- carbonitrile

**Example 123**

6- amino- 4- (3- chloro- 4- fluorophenylamino)- 7- ethoxyquinoline- 3- carbonitrile

**Example 124**

6- amino- 7- ethoxy- 4- (3- ethynylphenylamino) quinoline- 3- carbonitrile

**Example 125**

6- amino- 4- (3- bromophenylamino)- 7- ethoxyquinoline- 3- carbonitrile

**Example 126**

6- amino- 4- (3- chloro- 4- (pyridin- 2- ylmethoxy) phenylamino)- 7- methoxyquinoline- 3- car bonitrile

**Example 127**

6- amino- 4- (3- chloro- 4- (pyridin- 2- ylmethoxy) phenylamino)- 7- ethoxyquinoline- 3- carbo nitrile

**Example 128**

4- (4- (3- fluorobenzyloxy)- 3- chlorophenylamino)- 6- amino- 7- methoxyquinoline- 3- carb Onitrile

**Example 129**

4- (4- (3- fluorobenzyloxy)- 3- chlorophenylamino)- 6- amino- 7- ethoxyquinoline- 3- carboni trile

**Example 130**

6- amino- 4- (4- (3- fluorophenoxy)- 3- methoxyphenylamino)- 7- methoxyquinoline- 3- carb onitrile

**Example 131**

6- amino- 7- ethoxy- 4- (4- (3- fluorophenoxy)- 3- methoxyphenylamino) quinoline- 3- carbo nitrile
Following the procedure of Example **104,** the compounds of **132-145** were prepared.

**Example 132**

N- (4- (3- chloro- 4- (pyridin- 2- ylmethoxy) phenylamino)- 3- cyano- 7- methoxyquinolin- 6- yl )- 2- (piperidin- 4- ylidene) acetamide MS: 554 (M+1)

**Example 133**

N- (4- (3- chloro- 4- (pyridin- 2- ylmethoxy) phenylamino)- 3- cyano- 7- ethoxyquinolin- 6- yl)- 2- (piperidin- 4- ylidene) acetamide MS: 568 (M+1)

**Example 134**

N- (4- (4- (3- fluorobenzyloxy)- 3- chlorophenylamino)- 3- cyano- 7- methoxyquinolin- 6- yl)- 2- (piperidin- 4- ylidene) acetamide MS: 571 ($M^+$)

**Example 135**

N- (4- (4- (3- fluorobenzyloxy)- 3- chlorophenylamino)- 3- cyano- 7- ethoxyquinolin- 6- yl)- 2- (piperidin- 4- ylidene) acetamide MS: 586 (M+1)

**Example 136**

N- (4- (3- chloro- 4- (3- fluorophenoxy) phenylamino)- 3- cyano- 7- methoxyquinolin- 6- yl)- 2- (piperidin- 4- ylidene) acetamide MS: 558 (M+1)

**Example 137**

N- (4- (3- chlorophenylamino)- 3- cyano- 7- ethoxyquinolin- 6- yl)- 2- (piperidin- 4- ylidene) ac etamide MS: 461 ($M^+$)

**Example 138**

N- (4- (3- chlorophenylamino)- 3- cyano- 7- methoxyquinolin- 6- yl)- 2- (piperidin- 4- ylidene) acetamide MS: 448 (M+1)

**Example 139**

N- (4- (3- chloro- 4- fluorophenylamino)- 3- cyano- 7- (2- methoxyethoxy) quinolin- 6- yl)- 2- (p iperidin- 4- ylidene) acetamide MS: 510 (M+1)

**Example 140**

N- (4- (3- chloro- 4- fluorophenylamino)- 3- cyano- 7- ethoxyquinolin- 6- yl)- 2- (piperidin- 4- yli dene) acetamide MS: 480 (M+1)

**Example 141**

N- (4- (3- chloro- 4- fluorophenylamino)- 3- cyano- 7- methoxyquinolin- 6- yl)- 2- (piperidin- 4- ylidene) acetamide MS: 466 (M+1)

**Example 142**

N- (3- cyano- 7- ethoxy- 4- (3- ethynylphenylamino) quinolin- 6- yl)- 2- (piperidin- 4- ylidene) a cetamide MS: 452 (M+1)

**Example 143**

N- (3- cyano- 4- (3- ethynylphenylamino)- 7- methoxyquinolin- 6- yl)- 2- (piperidin- 4- ylidene ) acetamide MS:

438 (M+1)

**Example 144**

N- (4- (3- bromophenylamino)- 3- cyano- 7- ethoxyquinolin- 6- yl)- 2- (piperidin- 4- ylidene) ac etamide MS: 506 ($M^+$)

**Example 145**

N- (4- (3- bromophenylamino)- 3- cyano- 7- methoxyquinolin- 6- yl)- 2- (piperidin- 4- ylidene) acetamide MS: 493 (M+1)

Following the procedure of Example **116**, the compounds of **146-154** were prepraed.

**Example 146**

N- (4- (3- chloro- 4- fluorophenylamino)- 3- cyano- 7- ethoxyquinolin- 6- yl)- 2- (1- methylpipe ridin- 4- ylidene) acetamide MS: 494 (M+1)

**Example 147**

N- (4- (3- chloro- 4- fluorophenylamino)- 3- cyano- 7- ethoxyquinolin- 6- yl)- 2- (1- (2- methoxy ethyl) piperidin- 4- ylidene) acetamide MS: 537 ($M^+$)

**Example 148**

N- (3- cyano- 7- ethoxy- 4- (3- ethynylphenylamino) quinolin- 6- yl)- 2- (1- methylpiperidin- 4- ylidene) acetamide MS: 466 (M+1)

**Example 149**

N- (3- cyano- 7- ethoxy- 4- (3- ethynylphenylamino) quinolin- 6- yl)- 2- (1- (2- methoxyethyl) pi peridin- 4- ylidene) acetamide MS: 510 (M+1)

**Example 150**

N- (4- (3- chloro- 4- (pyridin- 2- ylmethoxy) phenylamino)- 3- cyano- 7- ethoxyquinolin- 6- yl)- 2- (1- methylpiperidin- 4- ylidene) acetamide MS: 582 (M+1)

**Example 151**

N- (4- (3- chloro- 4- (pyridin- 2- ylmethoxy) phenylamino)- 3- cyano- 7- ethoxyquinolin- 6- yl)- 2- (1- (2- methoxyethyl) piperidin- 4- ylidene) acetamide MS: 626 (M+1)

**Example 152**

N- (4- (4- (3- fluorobenzyloxy)- 3- chlorophenylamino)- 3- cyano- 7- ethoxyquinolin- 6- yl)- 2- (1- methylpiperidin- 4- ylidene) acetamide MS: 600 (M+1)

**Example 153**

N- (4- (4- (3- fluorobenzyloxy)- 3- chlorophenylamino)- 3- cyano- 7- ethoxyquinolin- 6- yl)- 2- (1- (2- methoxyethyl) piperidin- 4- ylidene) acetamide MS: 643 ($M^+$)

**Example 154**

N- (4- (3- ethynylphenylamino- )- 7- methoxyquinazolin- 6- yl)- 2- (1- (2- methoxyethyl) piperi din- 4- ylidene) acetamide MS: 471 ($M^+$)

## Claims

1. A compound of formula (I), or pharmaceutically acceptable salt or hydrate thereof,

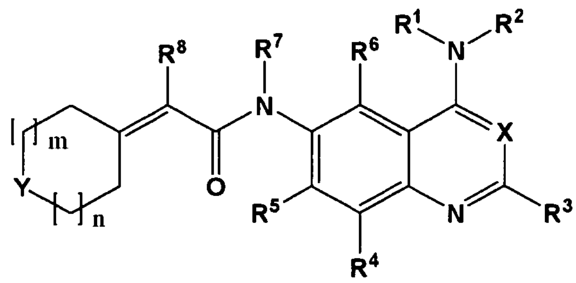

(I)

wherein
X represents N, C- CN, or CH;
Y represents $CH_2$, S, O, or N- $R^9$;
$R^1$, $R^3$, $R^7$ and $R^8$ independently represent H, $CF_3$, or $C_{1-6}$alkyl;
$R^2$ represents a group selected from formula (**II**), (**III**), (**IV**), (**V**), (**VI**), (**VII**), or (**VIII**) ;

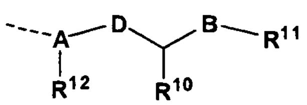

(**II**)

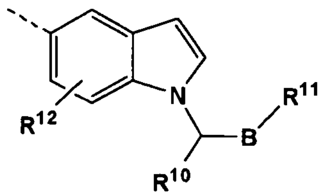

(**III**)

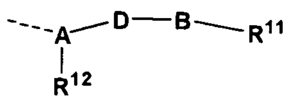

(**IV**)

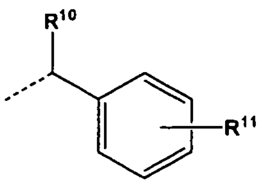

(**V**)

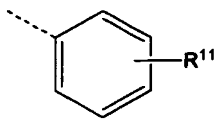

(**VI**)

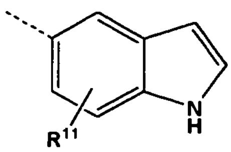

(**VII**)

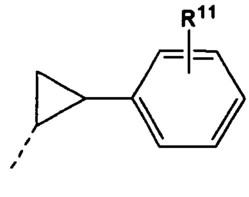

(**VIII**)

$R^4$ and $R^6$ independently represent H, $C_{1-6}$alkyl, $OC_{1-6}$alkyl, OH, F, Cl, Br, $OCF_3$, or trifluoromethyl;
$R^5$ at each occurrence is independently selected from H, F, $C_{1-6}$alkyl, OH, $OC_{1-6}$alkyl, $OCF_3$, $OCF_2CH_3$, $NH_2$, NH ($C_{1-6}$alkyl), N ($C_{1-6}$alkyl)$_2$, 1- pyrrolinyl, 1- piperidyl, 4- morpholinyl, Cl, Br, trifluoromethyl, O $(CH_2)_{2-4}OC_{1-6}$alkyl, O $(CH_2)_{2-4}OCF_3$, O $(CH_2)_{2-4}$NH ($C_{1-6}$alkyl), O $(CH_2)_{2-4}$N ($C_{1-6}$alkyl)$_2$, (1- pyrrolinyl) $(CH_2)_{2-4}$O, (1- piperidyl) $(CH_2)_{2-4}$O, (4- morpholinyl) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) $OC_{1-6}$alkyl), OC (O) NH ($C_{1-6}$alkyl), OC (O) N ($C_{1-6}$alkyl)$_2$, (1- piperidyl) $(CH_2)_{2-4}$OC (O), (4- morpholinyl) $(CH_2)_{2-4}$OC (O), (1- pyrrolinyl) $(CH_2)_{2-4}$OC (O), (1- imidazolyl) $(CH_2)_{2-4}$O, (pyrazolyl) $(CH_2)_{2-4}$O, (triazolyl) $(CH_2)_{2-4}$OC (O), or Ar $(CH_2)_{1-4}$O;
$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-6}$alkyl), $(CH_2)_{1-4}$N ($C_{1-6}$alkyl)$_2$, (1- pyrrolinyl) $(CH_2)_{1-4}$, (1- piperidyl) $(CH_2)_{1-4}$, (4- morpholinyl) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N ($C_{1-6}$alkyl)$_2$, (1- pyrrolinyl) $(CH_2)_{1-6}$C (O), (1- piperidyl) $(CH_2)_{1-6}$C (O), (4- morpholinyl) $(CH_2)_{1-4}$C (O), C (O) $OC_{1-6}$alkyl, C (O) O $(CH_2)_{2-4}OC_{1-6}$alkyl, C (O) O $(CH_2)_{2-4}$N ($C_{1-6}$alkyl)$_2$, (1- pyrrolinyl) $(CH_2)_{2-4}$OC (O), (1- piperidyl) $(CH_2)_{2-4}$OC (O), (4- morpholinyl) $(CH_2)_{2-4}$OC (O), $(CH_2)_{1-4}$C (O) $OC_{1-6}$alkyl, or Ar $(CH_2)_{1-4}$;
$R^{10}$ represents H, $C_{1-6}$alkyl, or one or two F;

$R^{11}$, $R^{12}$ independently represent H, or 1 to 4 same or different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH ($C_{1-4}$alkyl), N ($C_{1-4}$alkyl)$_2$, OC (O) $C_{1-4}$alkyl, NHC (O) H, NHC (O) $C_{1-4}$alkyl, N ($C_{1-4}$alkyl) C (O) $C_{1-4}$alkyl, C (O) $OC_{1-4}$alkyl, C (O) $NHC_{1-4}$alkyl, C (O) N ($C_{1-4}$alkyl)$_2$, COOH, C (O) $C_{1-4}$alkyl, S (O) $C_{1-4}$alkyl, $SO_2C_{1-4}$alkyl, $SO_2NHC_{1-4}$alkyl, or $SO_2N$ ($C_{1-4}$alkyl)$_2$;
A and B independently represent an aromatic ring;
Ar is a phenyl, a substituted phenyl or pyridyl;
D represents O, S, NH, or methylene; and
m and n independently represent an integer from 0 to 4.

**2.** The compound of formula (**I**) according to claim 1, wherein $R^1$, $R^3$, $R^4$, $R^6$ and $R^7$ independently represent H; and $R^8$ represents H, $CH_3$, or $CH_2CH_3$.

**3.** The compound of formula (**I**) according to claim 1, wherein $R^5$ at each occurrence is independently selected from H, F, $OCF_3$, $OC_{1-4}$alkyl, O $(CH_2)_{2-3}OC_{1-4}$alkyl, O $(CH_2)_{2-3}$N ($C_{1-4}$alkyl)$_2$, 2- (1- pyrrolinyl) ethoxyl, 2- (1- piperidyl) ethoxyl, 2- (4- morpholinyl) ethoxyl, 3- (1- pyrrolinyl) propyloxyl, 3- (1- piperidyl) propyloxyl, 3- (4- morpholinyl) propyloxyl, Ph $(CH_2)_{1-4}$O, or Py $(CH_2)_{1-4}$O.

**4.** The compound of formula (**I**) according to claim 1, wherein Y represents N- $R^9$, and $R^9$ at each occurrence is independently selected from H, $CF_3$, $C_{1-6}$alkyl, $(CH_2)_{2-4}$OH, $(CH_2)_{2-4}OC_{1-6}$alkyl, $(CH_2)_{2-4}$N ($C_{1-6}$alkyl)$_2$, (1- pyrrolinyl)- $(CH_2)_{2-4}$, (1- piperidyl)- $(CH_2)_{2-4}$, (4- morpholinyl)- $(CH_2)_{2-4}$, C (O) $OC_{1-6}$alkyl, $(CH_2)_{1-4}$ (O) $OC_{1-6}$alkyl, Ph $(CH_2)_{1-4}$, or Py $(CH_2)_{1-4}$.

**5.** The compound of formula (**I**) according to claim 1, wherein m and n independently represent 0, 1 or 2.

**6.** The compound of formula (**I**) according to claim 1, wherein said compound inhibits protein tyrosine kinase activity.

**7.** The compound of formula (**I**) according to claim 1, wherein said compound inhibits the growth of cancer cells.

**8.** The compound of formula (**I**) according to claim 6, wherein said protein tyrosine kinase represents EGF$\alpha$ tyrosine kinase or Her-2 tyrosine kinase.

**9.** The compound of formula (**I**) according to claim 7, wherein in said cancer cells EGFR is overexpressed or Her-2 is overexpressed.

**10.** A preparation comprising at least a compound of formula (**I**), or a pharmaceutically acceptable salt or hydrate thereof, wherein said compound or said pharmaceutically acceptable salt or hydrate thereof is used as medical active ingredient and processed with required and pharmaceutically acceptable excipients into a suitable dosage form, and said compound or said pharmaceutically acceptable salt or hydrate thereof used as medical active ingredient can treat or prevent diseases by oral administration, intravenous injection, intraperitoneal administration, subcutaneous injection, intramuscular injection, nasal drip, intraocular administration, inhalation, anal administration, vaginal administration, or epidermal administration,

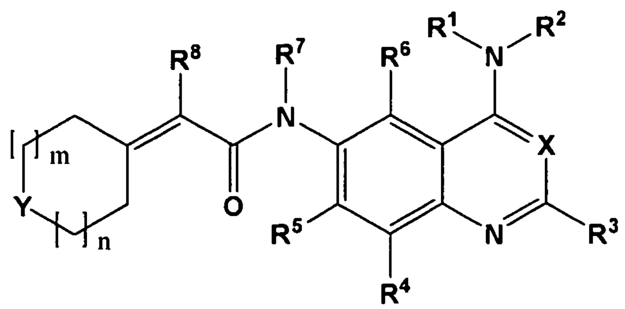

**(I)**

wherein
X represents N, C- CN, or CH;
Y represents $CH_2$, S, O, or N- $R^9$;

$R^1$, $R^3$, $R^7$ and $R^8$ independently represent H, $CF_3$, $C_{1-6}$alkyl;
$R^2$ represents a group selected from: formula (**II**), (**III**), (**IV**), (**V**), (**VI**), (**VII**), or (**VIII**) ;

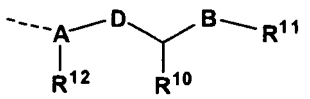

(**II**)

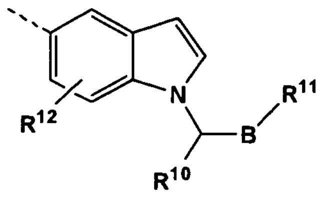

(**III**)

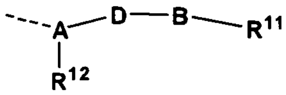

(**IV**)

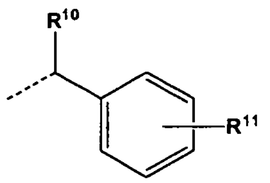

(**V**)

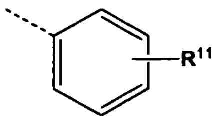

(**VI**)

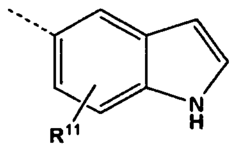

(**VII**)

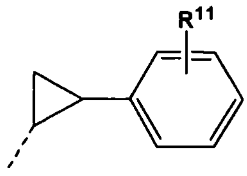

(**VIII**)

$R^4$ and $R^6$ independently represent H, $C_{1-6}$alkyl, $OC_{1-6}$alkyl, OH, F, Cl, Br, $OCF_3$, or trifluoromethyl;
$R^5$ at each occurrence is independently selected from H, F, $C_{1-6}$alkyl, OH, $OC_{1-6}$alkyl, $OCF_3$, $OCF_2CH_3$, $NH_2$, NH ($C_{1-6}$alkyl), N ($C_{1-6}$alkyl)$_2$, 1- pyrrolinyl, 1- piperidyl, 4- morpholinyl, Cl, Br, trifluoromethyl, O $(CH_2)_{2-4}OC_{1-6}$alkyl, O $(CH_2)_{2-4}OCF_3$, O $(CH_2)_{2-4}$NH ($C_{1-6}$alkyl), O $(CH_2)_{2-4}$N ($C_{1-6}$alkyl)$_2$, (1- pyrrolinyl) (CH2) 2- 40, (1- piperidyl) $(CH_2)_{2-4}$O, (4- morpholinyl) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) $OC_{1-6}$alkyl, OC (O) NH ($C_{1-6}$alkyl), OC (O) N ($C_{1-6}$alkyl)$_2$, (1- piperidyl) $(CH_2)_{2-4}$OC (O), (4- morpholinyl) $(CH_2)_{2-4}$OC (O), (1- pyrrolinyl) $(CH_2)_{2-4}$OC (O), (1- imidazolyl) $(CH_2)_{2-4}$O, (pyrazolyl) $(CH_2)_{2-4}$O, (triazolyl) $(CH_2)_{2-4}$OC (O), or Ar $(CH_2)_{1-4}$) O;
$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-6}$alkyl), $(CH_2)_{1-4}$N ($C_{1-6}$alkyl)$_2$, (1- pyrrolinyl) $(CH_2)_{1-4}$, (1- piperidyl) $(CH_2)_{1-4,}$ (4- morpholinyl) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N ($C_{1-6}$alkyl)$_2$, (1- pyrrolinyl) $(CH_2)_{1-6}$C (O), (1- piperidyl) $(CH_2)_{1-6}$C (O), (4- morpholinyl) $(CH_2)_{1-4}$C (O), C (O) $OC_{1-6}$alkyl, C (O) O $(CH_2)_{2-4}OC_1$-$_6$alkyl, C (O) O $(CH_2)_{2-4}$N ($C_{1-6}$alkyl)$_2$, (1- pyrrolinyl) $(CH_2)_{2-4}$OC (O), (1- piperidyl) $(CH_2)_{2-4}$OC (O), (4- morpholinyl) $(CH_2)_{2-4}$OC (O), $(CH_2)_{1-4}$C (O) $OC_{1-6}$alkyl, or Ar $(CH_2)_{1-4}$;
$R^{10}$ represents H, $C_{1-6}$alkyl, or one or two F;
$R^{11}$ and $R^{12}$ independently represent H, or 1 to 4 same or different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH ($C_{1-4}$alkyl), N ($C_{1-4}$alkyl)$_2$, OC (O) $C_{1-4}$alkyl, -NHC (O) H; NHC (O) $C_{1-4}$alkyl, -N ($C_{1-4}$alkyl) C (O) Calkyl, C (O) $OC_{1-4}$alkyl, C (O) $NHC_{1-4}$alkyl, C (O) N ($C_{1-4}$alkyl), COOH, C (O) $C_{1-4}$alkyl, S (O) $C_{1-4}$alkyl, $SO_2C_{1-4}$alkyl, $SO_2NHC_{1-4}$alkyl, or $SO_2$N ($C_{1-4}$alkyl) ;
A and B independently represent an aromatic ring;
Ar is phenyl, a substituted phenyl, or pyridyl;
D represents O, S, NH, or methylene; and
m and n independently represent an integer from 0 to 4.

**11.** The preparation according to claim 10 comprising at least a compound of formula (I), or pharmaceutically acceptable salt or hydrate thereof, wherein said preparation can be used to treat cancers.

**12.** The preparation according to claim 11, wherein said cancers comprise breast cancer, kidney cancer, bladder cancer, oral cancer, laryngeal cancer, esophagus cancer, gastric cancer, colorectal cancer, ovarian cancer, lung cancer and head and neck cancer.

**13.** The preparation according to claim 10 comprising at least a compound of formula (I), or pharmaceutically acceptable salts or hydrates thereof, wherein said preparation can be used to treat or prevent physiological disorder caused by inhibiting protein tyrosine kinase activity in mammals.

**14.** The preparation according to claim 13, wherein said physiological disorder comprises psoriasis, pneumonia, hepatitis, nephritis, pancreatitis, diabetes, or any kind of cancers.

**15.** A method of preparing a compound of formula (**I**) according to claims 1, or 10,

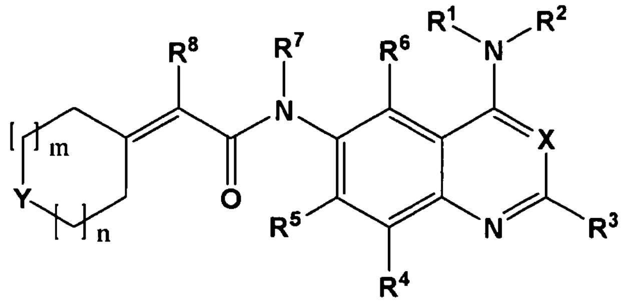

**(I)**

wherein
X represents N, C- CN, or CH;
Y represents $CH_2$, S, O, or N- $R^9$;
$R^1$, $R^3$, $R^7$ and $R^8$ independently represent H, $CF_3$, $C_{1-6}$alkyl;
$R^2$ represents a group selected from: formula (**II**), (**III**), (**IV**), (**V**), (**VI**), (**VII**), or (**VIII**) ;

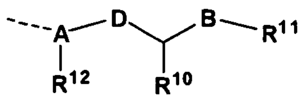

**(II)**

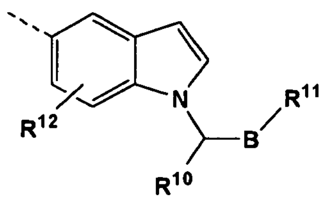

**(III)**

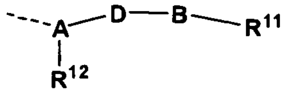

**(IV)**

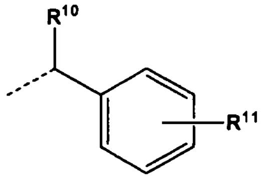

**(V)**

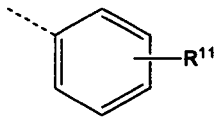

**(VI)**

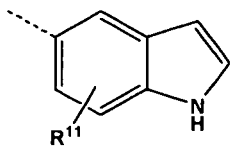

**(VII)**

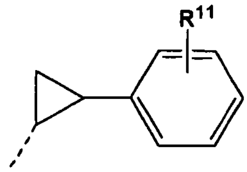

**(VIII)**

$R^4$ and $R^6$ independently represent H, $C_{1-6}$alkyl, $OC_{1-6}$alkyl, OH, F, Cl, Br, $OCF_3$, or trifluoromethyl;
$R^5$ at each occurrence is independently selected from H, F, $C_{1-6}$alkyl, OH, $OC_{1-6}$alkyl, $OCF_3$, $OCF_2CH_3$, $NH_2$, NH ($C_{1-6}$alkyl), N ($C_{1-6}$alkyl)$_2$, 1- pyrrolinyl, 1- piperidyl, 4- morpholin, Cl, Br, trifluoromethyl, O $(CH_2)_{2-4}OC_{1-6}$alkyl, O $(CH_2)_{2-4}OCF_3$, O $(CH_2)_{2-4}$NH ($C_{1-6}$alkyl), O $(CH_2)_{2-4}$N ($C_{1-6}$alkyl)$_2$, (1- pyrrolinyl) $(CH_2)_{2-4}$O, (1- piperidyl) $(CH_2)_{2-4}$O, (4- morpholinyl) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) O) $C_{1-6}$alkyl, OC (O) NH ($C_{1-6}$alkyl), OC (O) N ($C_{1-6}$alkyl)$_2$, (1- piperidyl) $(CH_2)_{2-4}$OC (O), (4- morpholinyl) $(CH_2)_{2-4}$0C (O), (1- pyrrolinyl) $(CH_2)_{2-4}$OC (O), (1- imidazolyl) $(CH_2)_{2-4}$O, (pyrazolyl) $(CH_2)_{2-4}$O, (triazolyl) $(CH_2)_{2-4}$OC (O), or Ar $(CH_2)_{1-4}$) O;
$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-8}$alkyl), $(CH_2)_{1-4}$N ($C_{1-8}$alkyl)$_2$, (1- pyrrolinyl) $(CH_2)_{1-4}$, (1- piperidyl) $(CH_2)_{1-4}$, (4- morpholinyl) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N ($C_{1-6}$alkyl)$_2$, (1- pyrrolinyl) $(CH_2)_{1-6}$C (O), (1- pipeddyl) $(CH_2)_{1-6}$C (O), (4- morpholinyl) $(CH_2)_{1-4}$C (O), C (O) $OC_{1-6}$alkyl, C (O) O $(CH_2)_{2-4}OC_{1-6}$alkyl, C (O) O $(CH_2)_{2-4}$N ($C_{1-6}$alkyl)$_2$, (1- pyrrolinyl) $(CH_2)_{2-4}$OC (O), (1- piperidyl) $(CH_2)_{2-4}$OC (O), (4- morpholinyl) $(CH_2)_{2-4}$OC (O), $(CH_2)_{1-4}$C (O) $OC_{1-6}$alkyl, or Ar $(CH_2)_{1-4}$;

$R^{10}$ represents H, $C_{1-6}$alkyl, or one or two F;
$R^{11}$ and $R^{12}$ independently represent H, or 1 to 4 same or different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH ($C_{1-4}$alkyl), N ($C_{1-4}$alkyl)$_2$, OC (O) $C_{1-4}$alkyl, NHC (O) H, NHC (O) $C_{1-4}$alkyl, N ($C_{1-4}$alky) C (O) $C_{1-4}$alkyl, C (O) $OC_{1-4}$alkyl, C (O) $NHC_{1-4}$alkyl, C (O) N ($C_{1-4}$alkyl), COOH, C (O) $C_{1-4}$alkyl, S (O) $C_{1-4}$alkyl, $SO_2C_{1-4}$alkyl, $SO_2NHC_{1-4}$alkyl, or $SO_2N$ ($C_{1-4}$alkyl) ;
A and B independently represent an aromatic ring;
Ar is phenyl, a substituted phenyl, or pyridyl;
D represents O, S, NH, or methylene; and
m and n independently represent an integer from 0 to 4;
the method comprising the steps of:

1) hydrolyzing the reaction product obtained by contacting the compound of formula (**XI**) with $R^{15}$ ($R^{13}$) P (O) CH ($R^8$) $COOR^{14}$ or $Ar_3P=CR^8CO_2R^{14}$ to obtain the compound of formula (**IX**), wherein $R^{13}$ and $R^{15}$ independently represent $C_{1-4}$alkyl, $OC_{1-4}$alkyl, phenyl, substituted phenyl, phenoxy, or substituted phenoxy; $R^{14}$ represents $C_{1-4}$alkyl, and the definition of Y is the same as that in claim 1 except that Y does not represents NH;

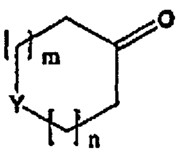

(**XI**)

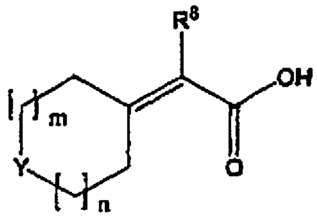

(**IX**)

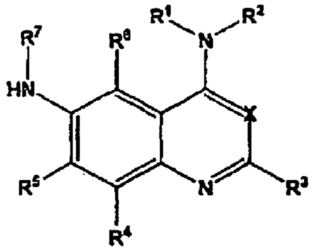

(**X**)

2) contacting the compound of formula (**X**), an acyl chloride, or a mixed anhydride thereof with the compound of formula (**IX**) in the presence of a condensing agent to obtain the compound of formula (**I**);
3) transforming the compound of formula (**I**) obtained in step 2) wherein Y represents N- $R^9$ and $R^9$ represents $(CH_3)_3O$ (C (O) into the compound of formula (**XII**) under acidic conditions, wherein the definitions of $R^1$, $R^2$, $R^4$, $R^5$, $R^6$, $R^7$, $R^8$, $R^{10}$, $R^{11}$, $R^{12}$, A, B, D, X, Y, m and n are the same as those in claim 1;

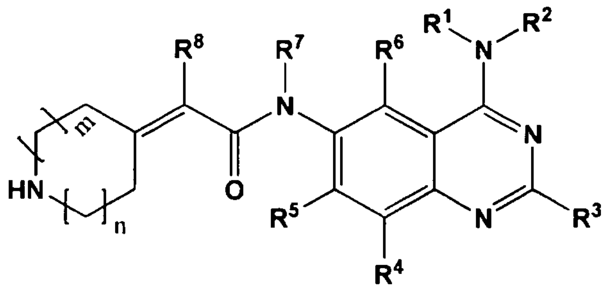

(**XII**)

4) contacting the compound of formula (**XII**) with $XR^9$ to obtain the compound of formula (**I**), wherein the definition of $R^9$ is the same as that for formula (**I**), and X represents Cl, Br, I, OMs, or OTs.

**16.** Use of compound of formula (**I**), or pharmaceutically acceptable salt or hydrate thereof,

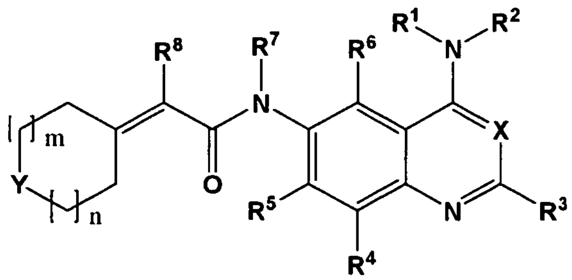

(I)

for the manufacture of a medicament for the treatment of psoriasis, pneumonia, hepatitis, nephritis, pancreatitis, diabetes, cancer, or any disorder which can be treated by inhibiting protein tyrosine kinase activity in mammals, wherein

X represents N, C- CN, or CH;

Y represents $CH_2$, S, O, or N- $R^9$;

$R^1$, $R^3$, $R^7$ and $R^8$ independently represent H, $CF_3$, or $C_{1-6}$alkyl;

$R^2$ represents a group selected from: formula (**II**), (**III**), (**IV**), (**V**), (**VI**), (**VII**), or (**VIII**) ;

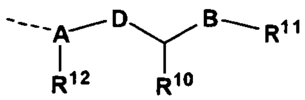

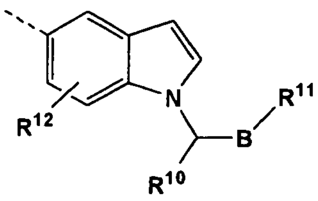

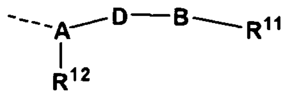

(**II**) (**III**) (**IV**)

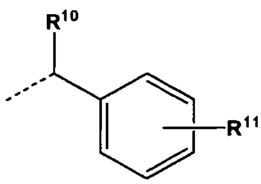

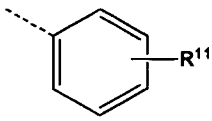

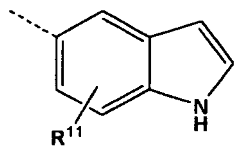

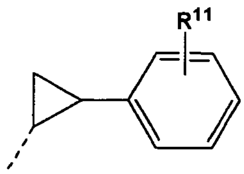

(**V**) (**VI**) (**VII**) (**VIII**)

$R^4$ and $R^6$ independently represent H, $C_{1-6}$alkyl, $OC_{1-6}$alkyl, OH, F, Cl, Br, $OCF_3$ or trifluoromethyl;

$R^5$ at each occurrence is independently selected from H, F, $C_{1-6}$alkyl, OH, $OC_{1-6}$alkyl, $OCF_3$, $OCF_2CH_3$, $NH_2$, NH ($C_{1-6}$alkyl), N ($C_{1-6}$alkyl)$_2$, 1- pyrrolinyl, 1- piperidyl, 4- morpholinyl, Cl, Br, trifluoromethyl, O $(CH_2)_{2-4}OC_{1-6}$alkyl, O $(CH_2)_{2-4}OCF_3$, O $(CH_2)_{2-4}$NH ($C_{1-6}$alkyl), O $(CH_2)_{2-4}$N ($C_{1-6}$alkyl)$_2$, (1- pyrrolinyl) $(CH_2)_{2-4}$O, (1- piperidyl) $(CH_2)_{2-4}$O, (4- morpholinyl) $(CH_2)_{2-4}$O, NHC (O) H, NHC (O) ($C_{1-6}$alkyl), N ($C_{1-6}$alkyl) C (O) ($C_{1-6}$alkyl), O $(CH_2)_{2-4}$OH, N ($C_{1-6}$alkyl) C (O) O ($C_{1-6}$alkyl), N ($C_{1-6}$alkyl) C (O) OH, NHC (O) $OC_{1-6}$alkyl), OC (O) NH ($C_{1-6}$alkyl), OC (O) N ($C_{1-6}$alkyl)$_2$, (1- piperidyl) $(CH_2)_{2-4}$OC (O), (4- morpholinyl) $(CH_2)_{2-4}$OC (O), (1- pyrrolinyl) $(CH_2)_{2-4}$OC (O), (1- imidazolyl) $(CH_2)_{2-4}$O, (pyrazolyl) $(CH_2)_{2-4}$O, (triazolyl) $(CH_2)_{2-4}$OC (O), or Ar $(CH_2)_{1-4}$O;

$R^9$ at each occurrence is independently selected from H, $C_{1-6}$alkyl, $CF_3$, $CF_2CH_3$, $(CH_2)_{2-4}$OH, $(CH_2)_{1-4}OC_{1-6}$alkyl, $(CH_2)_{1-4}$NH ($C_{1-6}$alkyl), $(CH_2)_{1-4}$N ($C_{1-6}$alkyl)$_2$, (1- pyrrolinyl) $(CH_2)_{1-4}$, (1- piperidyl) $(CH_2)_{1-4}$, (4- morpholinyl) $(CH_2)_{1-4}$, C (O) $C_{1-6}$alkyl, C (O) $(CH_2)_{1-4}$OH, C (O) $(CH_2)_{1-4}OC_{1-6}$alkyl, C (O) $(CH_2)_{1-4}$N ($C_{1-6}$alkyl)$_2$, (1- pyrrolinyl) $(CH_2)_{1-6}$C (O), (1- piperidyl) $(CH_2)_{1-6}$C (O), (4- morpholinyl) $(CH_2)_{1-4}$C (O), C (O) $OC_{1-6}$alkyl, C (O) O $(CH_{2-4}OC_{1-6}$alkyl, C (O) O $(CH_2)_{2-4}$N ($C_{1-6}$alkyl)$_2$, (1- pyrrolinyl) $(CH_2)_{2-4}$OC (O), (1- piperidyl) $(CH_2)_{2-4}$OC (O), (4- morpholinyl) $(CH_2)_{2-4}$OC (O), $(CH_2)_{1-4}$C (O) $OC_{1-6}$alkyl, or Ar $(CH_2)_{1-4}$;

$R^{10}$ represents H, $C_{1-6}$alkyl, or one or two F;

$R^{11}$, $R^{12}$ independently represent H, or 1 to 4 same or different groups selected from F, Cl, Br, I, CN, $NO_2$, $CF_3$, OH, $NH_2$, $C_{1-4}$alkyl, $OC_{1-4}$alkyl, $OCF_3$, $OCF_2CH_3$, NH ($C_{1-4}$alkyl), N ($C_{1-4}$alkyl)$_2$, OC (O) $C_{1-4}$alkyl, NHC (O) H, NHC (O) $C_{1-4}$alkyl, N ($C_{1-4}$alkyl) C (O) $C_{1-4}$alkyl, C (O) $OC_{1-4}$alkyl, C (O) $NHC_{1-4}$alkyl, C (O) N ($C_{1-4}$alkyl)$_2$, COOH, C (O)

$C_{1-4}$alkyl, S (O) $C_{1-4}$alkyl, $SO_2C_{1-4}$alkyl, $SO_2NHC_{1-4}$alkyl, or $SO_2N$ ($C_{1-4}$alkyl)$_2$;
A and B independently represent an aromatic ring;
Ar is a phenyl, a substituted phenyl, or pyridyl;
D represents O, S, NH, or methylene; and
m and n independently represent an integer from 0 to 4.

**Patentansprüche**

1. Verbindung der Formel (**I**) oder ein pharmazeutisch geeignetes Salz oder Hydrat davon,

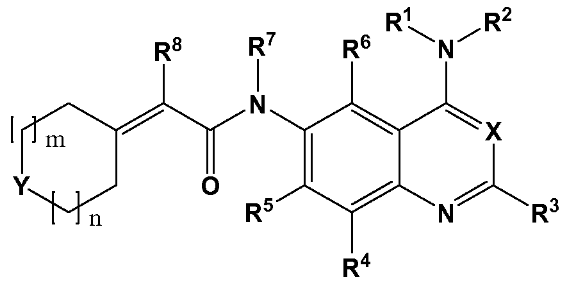

(**I**)

wobei
X für N, C- CN oder CH steht;
Y für $CH_2$, S, O oder N- $R^9$ steht;
$R^1$, $R^3$, $R^7$ und $R^8$ unabhängig für H, $CF_3$ oder $C_{1-6}$Alkyl stehen;
$R^2$ für eine Gruppe steht, die aus Formel (**II**), (**III**), (**IV**), (**V**), (**VI**), (**VII**) oder (**VIII**) ausgewählt ist;

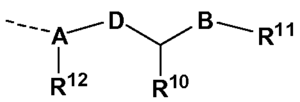

(**II**)

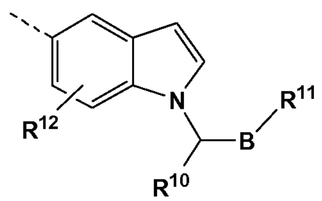

(**III**)

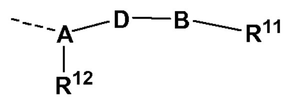

(**IV**)

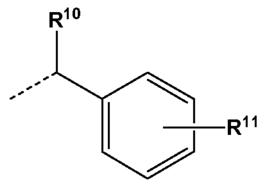

(**V**)

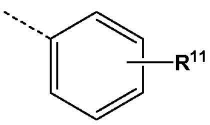

(**VI**)

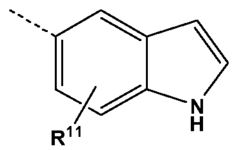

(**VII**)

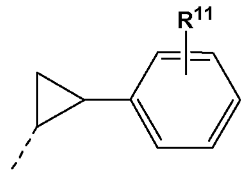

(**VIII**)

$R^4$ und $R^6$ unabhängig für H, $C_{1-6}$A1kyl, $OC_{1-6}$Alkyl, OH, F, Cl, Br, $OCF_3$ oder Trifluormethyl stehen;
$R^5$ bei jedem Auftreten unabhängig ausgewählt ist aus H, F, $C_{1-6}$Alkyl, OH, $OC_{1-6}$Alkyl, $OCF_3$, $OCF_2CH_3$, $NH_2$, NH ($C_{1-6}$Alkyl), N ($C_{1-6}$Alkyl)$_2$, 1- Pyrrolinyl, 1- Piperidyl, 4- Morpholinyl, Cl, Br, Trifluormethyl, O ($CH_2$)$_{2-4}$$OC_{1-6}$Alkyl, O ($CH_2$)$_{2-4}$$OCF_3$, O ($CH_2$)$_{2-4}$NH ($C_{1-6}$Alkyl), O ($CH_2$)$_{2-4}$N ($C_{1-6}$Alkyl)$_2$, (1- Pyrrolinyl) ($CH_2$)$_{2-4}$O, (1- Piperidyl) ($CH_2$)$_{2-4}$O, (4- Morpholinyl) ($CH_2$)$_{2-4}$O, NHC (O) H, NHC (O) ($C_{1-6}$Alkyl), N ($C_{1-6}$Alkyl) C (O) $C_{1-6}$alkyl), O ($CH_2$)$_{2-4}$OH, N ($C_{1-6}$Alkyl) C (O) O ($C_{1-6}$alkyl), N ($C_{1-6}$Alkyl) C (O) OH, NHC (O) $OC_{1-6}$Alkyl), OC (O) NH ($C_{1-6}$Alkyl), OC (O) N ($C_{1-6}$Alkyl)$_2$, (1- Piperidyl) ($CH_2$)$_{2-4}$OC (O), (4- Morpholinyl) ($CH_2$)$_{2-4}$OC (O), (1- Pyrrolinyl) ($CH_2$)$_{2-4}$OC (O), (1- Imidazolyl) ($CH_2$)$_{2-4}$O, (Pyrazolyl) ($CH_2$)$_{2-4}$O, (Triazolyl) ($CH_2$)$_{2-4}$OC (O) oder Ar ($CH_2$)$_{1-4}$O;

$R^9$ bei jedem Auftreten unabhängig ausgewählt ist aus H, $C_{1-6}$Alkyl, $CF_3$, $CF_2CH_3$, $(CH_2)_{2-4}OH$, $(CH_2)_{1-4}OC_{1-6}$Alkyl, $(CH_2)_{1-4}NH$ ($C_{1-6}$Alkyl), $(CH_2)_{1-4}N$ ($C_{1-6}$Alkyl)$_2$, (1- Pyrrolinyl) $(CH_2)_{1-4}$, (1- Piperidyl) $(CH_2)_{1-4}$, (4- Morpholinyl) $(CH_2)_{1-4}$, C (O) $C_{1-6}$Alkyl, C (O) $(CH_2)_{1-4}OH$, C (O) $(CH_2)_{1-4}OC_{1-6}$Alkyl, C (O) $(CH_2)_{1-4}N$ ($C_{1-6}$Alkyl)$_2$, (1- Pyrrolinyl) $(CH_2)_{1-6}$C (O), (1- Piperidyl) $(CH_2)_{1-6}$C (O), (4- Morpholinyl) $(CH_2)_{1-4}$C (O), C (O) $OC_{1-6}$Alkyl, C (O) O $(CH_2)_{2-4}OC_{1-6}$Alkyl, C (O) O $(CH_2)_{2-4}N$ ($C_{1-6}$Alkyl)$_2$, (1- Pyrrolinyl) $(CH_2)_{2-4}$OC (O), (1- Piperidyl) $(CH_2)_{2-4}$OC (O), (4- Morpholinyl) $(CH_2)_{2-4}$OC (O), $(CH_2)_{1-4}$C (O) $OC_{1-6}$Alkyl oder Ar $(CH_2)_{1-4}$;
$R^{10}$ für H, $C_{1-6}$Alkyl oder ein oder zwei F steht;
$R^{11}$, $R^{12}$ unabhängig für H oder 1 bis 4 gleiche oder unterschiedliche Gruppen stehen, die aus F, Cl, Br, I, CN, $NO_2$, $CF_3$, OH, $NH_2$, $C_{1-4}$Alkyl, $OC_{1-4}$Alkyl, $OCF_3$, $OCF_2CH_3$, NH ($C_{1-4}$Alkyl), N ($C_{1-4}$Alkyl)$_2$, OC (O) $C_{1-4}$Alkyl, NHC (O) H, NHC (O) $C_{1-4}$Alkyl, N ($C_{1-4}$Alkyl) C (O) $C_{1-4}$Alkyl, C (O) $OC_{1-4}$Alkyl, C (O) $NHC_{1-4}$Alkyl, C (O) N ($C_{1-4}$Alkyl)$_2$, COOH, C (O) $C_{1-4}$Alkyl, S (O) $C_{1-4}$Alkyl, $SO_2C_{1-4}$Alkyl, $SO_2NHC_{1-4}$Alkyl oder $SO_2N$ ($C_{1-4}$Alkyl)$_2$ ausgewählt sind;
A und B unabhängig für einen aromatischen Ring stehen;
Ar für ein Phenyl, ein substituiertes Phenyl oder Pyridyl steht;
D für O, S, NH oder Methylen steht; und
m und n unabhängig für eine Ganzzahl von 0 bis 4 stehen.

2. Verbindung der Formel (**I**) nach Anspruch 1, wobei $R^1$, $R^3$, $R^4$, $R^6$ und $R^7$ unabhängig für H; und $R^8$ represents H, $CH_3$ oder $CH_2CH_3$.

3. Verbindung der Formel (**I**) nach Anspruch 1, wobei $R^5$ bei jedem Auftreten unabhängig ausgewählt ist aus H, F, $OCF_3$, $OC_{1-4}$Alkyl, O $(CH_2)_{2-3}OC_{1-4}$Alkyl, O $(CH_2)_{2-3}N$ ($C_{1-4}$Alkyl)$_2$, 2- (1- Pyrrolinyl) ethoxyl, 2- (1- Piperidyl) ethoxyl, 2- (4- Morpholinyl) ethoxyl, 3- (1- Pyrrolinyl) propyloxyl, 3- (1- Piperidyl) propyloxyl, 3- (4- Morpholinyl) propyloxyl, Ph $(CH_2)_{1-4}$O oder Py $(CH_2)_{1-4}$O.

4. Verbindung der Formel (I) nach Anspruch 1, wobei Y für N- $R^9$ steht und $R^9$ bei jedem Auftreten unabhängig ausgewählt ist aus H, $CF_3$, $C_{1-6}$Alkyl, $(CH_2)_{2-4}OH$, $(CH_2)_{2-4}OC_{1-6}$Alkyl, $(CH_2)_{2-4}N$ ($C_{1-6}$Alkyl)$_2$, (1- Pyrrolinyl)- $(CH_2)_{2-4}$, (1- Piperidyl)- $(CH_2)_{2-4}$, (4- Morpholinyl)- $(CH_2)_{2-4}$, C (O) $OC_{1-6}$Alkyl, $(CH_2)_{1-4}$C (O) $OC_{1-6}$Alkyl, Ph $(CH_2)_{1-4}$ oder Py $(CH_2)_{1-4}$.

5. Verbindung der Formel (**I**) nach Anspruch 1, wobei m und n unabhängig für 0, 1 oder 2 stehen.

6. Verbindung der Formel (**I**) nach Anspruch 1, wobei die Verbindungen Proteintyrosinkinaseaktivität hemmen.

7. Verbindung der Formel (**I**) nach Anspruch 1, wobei die Verbindungen das Wachstum von Krebszellen hemmen.

8. Verbindung der Formel (**I**) nach Anspruch 6, wobei die Proteintyrosinkinase für EGF$\alpha$-Tyrosinkinase oder Her-2-Tyrosinkinase steht.

9. Verbindung der Formel (**I**) nach Anspruch 7, wobei in den Krebszellen EGFR überexprimiert ist oder Her-2 überexprimiert ist.

10. Zubereitung, welche mindestens eine Verbindung von Formel (**I**) oder ein pharmazeutisch geeignetes Salz oder Hydrat davon umfasst, wobei die Verbindung oder das pharmazeutisch geeignete Salz oder Hydrat davon als medizinischer Wirkstoff verwendet wird und mit erforderlichen und pharmazeutisch geeigneten Hilfsstoffen zu einer geeigneten Dosierungsform aufbereitet wird, und wobei die Verbindung oder das pharmazeutisch geeignete Salz oder Hydrat davon, die bzw. das als medizinischer Wirkstoff verwendet wird, Krankheiten durch orale Verabreichung, intravenöse Injektion, intraperitoneale Verabreichung, subkutane Injektion, intramuskuläre Injektion, Nasentropf, intraokuläre Verabreichung, Inhalierung, anale Verabreichung, vaginale Verabreichung oder epidermale Verabreichung behandeln oder verhindern kann,

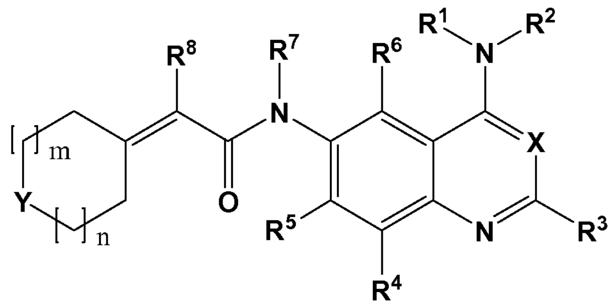

(I)

wobei

X für N, C- CN oder CH steht;

Y für $CH_2$, S, O oder N- $R^9$ steht;

$R^1$, $R^3$, $R^7$ und $R^8$ unabhängig für H, $CF_3$, $C_{1-6}$Alkyl stehen;

$R^2$ für eine Gruppe steht, die aus Formel (II), (III), (IV), (V), (VI), (VII) oder (VIII) ausgewählt ist;

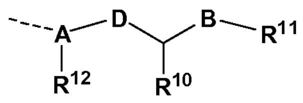

(II)

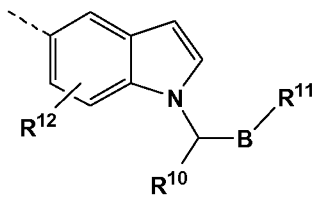

(III)

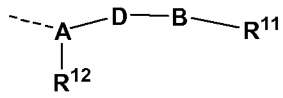

(IV)

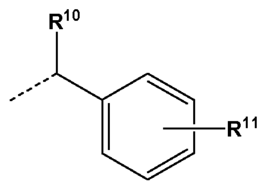

(V)

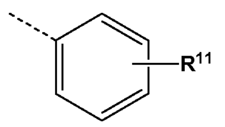

(VI)

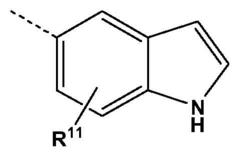

(VII)

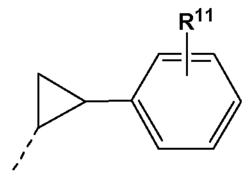

(VIII)

$R^4$ und $R^6$ unabhängig für H, $C_{1-6}$Alkyl, $OC_{1-6}$Alkyl, OH, F, Cl, Br, $OCF_3$ oder Trifluormethyl stehen;

$R^5$ bei jedem Auftreten unabhängig ausgewählt ist aus H, F, $C_{1-6}$Alkyl, OH, $OC_{1-6}$Alkyl, $OCF_3$, $OCF_2CH_3$, $NH_2$, NH ($C_{1-6}$Alkyl), N ($C_{1-6}$Alkyl)$_2$, 1- Pyrrolinyl, 1- Piperidyl, 4- Morpholinyl, Cl, Br, Trifluormethyl, O $(CH_2)_{2-4}OC_{1-6}$Alkyl, O $(CH_2)_{2-4}OCF_3$, O $(CH_2)_{2-4}$NH ($C_{1-6}$Alkyl), O $(CH_2)_{2-4}$N ($C_{1-6}$Alkyl)$_2$, (1- Pyrrolinyl) $(CH_2)_{2-4}$O, (1- Piperidyl) $(CH_2)_{2-4}$O, (4- Morpholinyl) $(CH_2)_{2-4}$O, NHC (O) H, NHC (O) ($C_{1-6}$Alkyl), N ($C_{1-6}$Alkyl) C (O) ($C_{1-6}$alkyl), O $(CH_2)_{2-4}$OH, N ($C_{1-6}$Alkyl) C (O) O ($C_{1-6}$alkyl), N ($C_{1-6}$Alkyl) C (O) OH, NHC (O) $OC_{1-6}$Alkyl, OC (O) NH ($C_{1-6}$Alkyl), OC (O) N ($C_{1-6}$Alkyl)$_2$, (1- Piperidyl) $(CH_2)_{2-4}$OC (O), (4- Morpholinyl) $(CH_2)_{2-4}$OC (O), (1- Pyrrolinyl) $(CH_2)_{2-4}$OC (O), (1- Imidazolyl) $(CH_2)_{2-4}$O, (Pyrazolyl) $(CH_2)_{2-4}$O, (Triazolyl) $(CH_2)_{2-4}$OC (O) oder Ar $(CH_2)_{1-4}$) O;

$R^9$ bei jedem Auftreten unabhängig ausgewählt ist aus H, $C_{1-6}$Alkyl, $CF_3$, $CF_2CH_3$, $(CH_2)_{2-4}$OH, $(CH_2)_{1-4}OC_{1-6}$Alkyl, $(CH_2)_{1-4}$NH ($C_{1-6}$Alkyl), $(CH_2)_{1-4}$N ($C_{1-6}$Alkyl)$_2$, (1- Pyrrolinyl) $(CH_2)_{1-4}$, (1- Piperidyl) $(CH_2)_{1-4}$, (4- Morpholinyl) $(CH_2)_{1-4}$, C (O) $C_{1-6}$Alkyl, C (O) $(CH_2)_{1-4}$OH, C (O) $(CH_2)_{1-4}OC_{1-6}$Alkyl, C (O) $(CH_2)_{1-4}$N ($C_{1-6}$Alkyl)$_2$, (1- Pyrrolinyl) $(CH_2)_{1-6}$C (O), (1- Piperidyl) $(CH_2)_{1-6}$C (O), (4- Morpholinyl) $(CH_2)_{1-4}$C (O), C (O) $OC_{1-6}$Alkyl, C (O) O $(CH_2)_{2-4}OC_{1-6}$Alkyl, C (O) O $(CH_2)_{2-4}$N ($C_{1-6}$Alkyl)$_2$, (1- Pyrrolinyl) $(CH_2)_{2-4}$OC (O), (1- Piperidyl) $(CH_2)_{2-4}$OC (O), (4- Morpholinyl) $(CH_2)_{2-4}$OC (O), $(CH_2)_{1-4}$C (O) $OC_{1-6}$Alkyl oder Ar $(CH_2)_{1-4}$;

$R^{10}$ für H, $C_{1-6}$Alkyl oder ein oder zwei F steht;

$R^{11}$ und $R^{12}$ unabhängig für H oder 1 bis 4 gleiche oder unterschiedliche Gruppen stehen, die aus F, Cl, Br, I, CN, $NO_2$, $CF_3$, OH, $NH_2$, $C_{1-4}$Alkyl, $OC_{1-4}$Alkyl, $OCF_3$, $OCF_2CH_3$, NH ($C_{1-4}$Alkyl), N ($C_{1-4}$Alkyl)$_2$, OC (O) $C_{1-4}$Alkyl, NHC

(O) H, NHC (O) $C_{1-4}$Alkyl, N ($C_{1-4}$Alkyl) C (O) $C_{1-4}$alkyl, C (O) $OC_{1-4}$Alkyl, C (O) $NHC_{1-4}$Alkyl, C (O) N ($C_{1-4}$Alkyl), COOH, C (O) $C_{1-4}$Alkyl, S (O) $C_{1-4}$Alkyl, $SO_2C_{1-4}$Alkyl, $SO_2NHC_{1-4}$Alkyl oder $SO_2N$ ($C_{1-4}$Alkyl) ausgewählt sind;
A und B unabhängig für einen aromatischen Ring stehen;
Ar für ein Phenyl, ein substituiertes Phenyl oder Pyridyl steht;
D für O, S, NH oder Methylen steht; und
m und n unabhängig für eine Ganzzahl von 0 bis 4 stehen.

**11.** Zubereitung nach Anspruch 10, die mindestens eine Verbindung der Formel (I) oder ein pharmazeutisch geeignetes Salz oder Hydrat davon umfasst, wobei die Zubereitung zum Behandeln von Krebserkrankungen verwendet werden kann.

**12.** Zubereitung nach Anspruch 11, wobei die Krebserkrankungen Brustkrebs, Nierenkrebs, Blasenkrebs, Mundkrebs, Larynxkrebs, Ösophaguskrebs, Magenkrebs, Kolorektalkrebs, Ovarialkrebs, Lungenkrebs und Kopf- und Halskrebs umfassen.

**13.** Zubereitung nach Anspruch 10, die mindestens eine Verbindung der Formel (I) oder pharmazeutisch geeignete Salze oder Hydrate davon umfasst, wobei die Zubereitung verwendet werden kann, um physiologische Störungen zu behandeln oder zu verhindern, die durch Hemmung von Proteintyroskinkinaseaktivität bei Säugetieren verursacht werden.

**14.** Zubereitung nach Anspruch 13, wobei die physiologische Störung Psoriasis, Pneumonie, Hepatitis, Nephritis, Pankreatitis, Diabetes oder eine beliebige Art von Krebserkrankung umfasst.

**15.** Verfahren des Zubereitens einer Verbindung der Formel (I) nach Anspruch 1 oder 10,

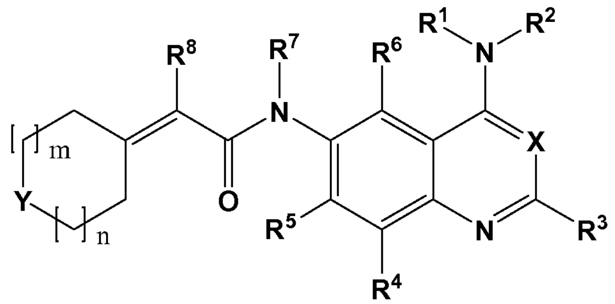

(**I**)

wobei
X für N, C- CN oder CH steht;
Y für $CH_2$, S, O oder N- $R^9$ steht;
$R^1$, $R^3$, $R^7$ und $R^8$ unabhängig für H, $CF_3$ oder $C_{1-6}$Alkyl stehen;
$R^2$ für eine Gruppe steht, die aus Formel (**II**), (**III**), (**IV**), (**V**), (**VI**), (**VII**) oder (**VIII**) ausgewählt ist;

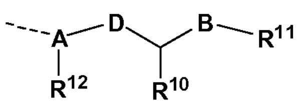

(**II**)

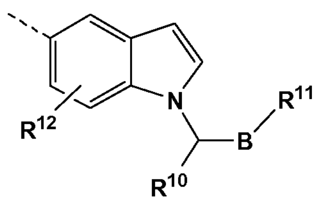

(**III**)

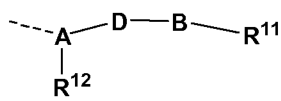

(**IV**)

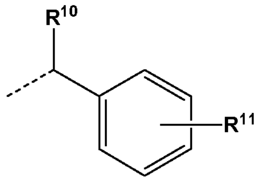
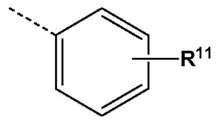
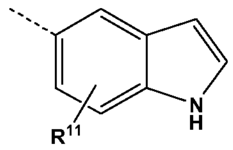
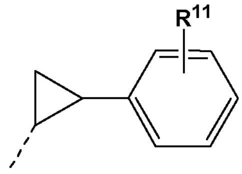

(**V**) (**VI**) (**VII**) (**VIII**)

$R^4$ und $R^6$ unabhängig für H, $C_{1-6}$Alkyl, $OC_{1-6}$Alkyl, OH, F, Cl, Br, $OCF_3$ oder Trifluormethyl stehen;
$R^5$ bei jedem Auftreten unabhängig ausgewählt ist aus H, F, $C_{1-6}$Alkyl, OH, $OC_{1-6}$Alkyl, $OCF_3$, $OCF_2CH_3$, $NH_2$, NH ($C_{1-6}$Alkyl), N ($C_{1-6}$Alkyl)$_2$, 1- Pyrrolinyl, 1- Piperidyl, 4- Morpholin, Cl, Br, Trifluormethyl, O $(CH_2)_{2-4}OC_{1-6}$Alkyl, O $(CH_2)_{2-4}OCF_3$, O $(CH_2)_{2-4}$NH ($C_{1-6}$Alkyl), O $(CH_2)_{2-4}$N ($C_{1-6}$Alkyl)$_2$, (1- Pyrrolinyl) $(CH_2)_{2-4}$O, (1- Piperidyl) $(CH_2)_{2-4}$O, (4- Morpholinyl) $(CH_2)_{2-4}$O, NHC (O) H, NHC (O) ($C_{1-6}$Alkyl), N ($C_{1-6}$Alkyl) C (O) ($C_{1-6}$alkyl), O $(CH_2)_{2-4}$OH, N ($C_{1-6}$Alkyl) C (O) O ($C_{1-6}$alkyl), N ($C_{1-6}$Alkyl) C (O) OH, NHC (O) O) $C_{1-6}$Alkyl), OC (O) NH ($C_{1-6}$Alkyl), OC (O) N ($C_{1-6}$Alkyl)$_2$, (1- Piperidyl) $(CH_2)_{2-4}$OC (O), (4- Morpholinyl) $(CH_2)_{2-4}$OC (O), (1- Pyrrolinyl) $(CH_2)_{2-4}$OC (O), (1- Imidazolyl) $(CH_2)_{2-4}$O, (Pyrazolyl) $(CH_2)_{2-4}$O, (Triazolyl) $(CH_2)_{2-4}$OC (O) oder Ar $(CH_2)_{1-4}$) O;
$R^9$ bei jedem Auftreten unabhängig ausgewählt ist aus H, $C_{1-6}$Alkyl, $CF_3$, $CF_2CH_3$, $(CH_2)_{2-4}$OH, $(CH_2)_{1-4}OC_{1-6}$Alkyl, $(CH_2)_{1-4}$NH ($C_{1-6}$Alkyl), $(CH_2)_{1-4}$N ($C_{1-6}$Alkyl)$_2$, (1- Pyrrolinyl) $(CH_2)_{1-4}$, (1- Piperidyl) $(CH_2)_{1-4}$, (4- Morpholinyl) $(CH_2)_{1-4}$, C (O) $C_{1-6}$Alkyl, C (O) $(CH_2)_{1-4}$OH, C (O) $(CH_2)_{1-4}OC_{1-6}$Alkyl, C (O) $(CH_2)_{1-4}$N ($C_{1-6}$Alkyl)$_2$, (1- Pyrrolinyl) $(CH_2)_{1-6}$C (O), (1- Piperidyl) $(CH_2)_{1-6}$C (O), (4- Morpholinyl) $(CH_2)_{1-4}$C (O), C (O) $OC_{1-6}$Alkyl, C (O) O $(CH_2)_{2-4}OC_{1-6}$Alkyl, C (O) O $(CH_2)_{2-4}$N ($C_{1-6}$Alkyl)$_2$, (1- Pyrrolinyl) $(CH_2)_{2-4}$OC (O), (1- Piperidyl) $(CH_2)_{2-4}$OC (O), (4- Morpholinyl) $(CH_2)_{2-4}$OC (O), $(CH_2)_{1-4}$C (O) $OC_{1-6}$Alkyl oder Ar $(CH_2)_{1-4}$;
$R^{10}$ für H, $C_{1-6}$Alkyl oder ein oder zwei F steht;
$R^{11}$ und $R^{12}$ unabhängig für H oder 1 bis 4 gleiche oder unterschiedliche Gruppen stehen, die aus F, Cl, Br, I, CN, $NO_2$, $CF_3$, OH, $NH_2$, $C_{1-4}$Alkyl, $OC_{1-4}$Alkyl, $OCF_3$, $OCF_2CH_3$, NH ($C_{1-4}$Alkyl), N ($C_{1-4}$Alkyl)$_2$, OC (O) $C_{1-4}$Alkyl, NHC (O) H, NHC (O) $C_{1-4}$Alkyl, N ($C_{1-4}$Alkyl) C (O) $C_{1-4}$alkyl, C (O) $OC_{1-4}$Alkyl, C (O) $NHC_{1-4}$Alkyl, C (O) N ($C_{1-4}$Alkyl), COOH, C (O) $C_{1-4}$Alkyl, S (O) $C_{1-4}$Alkyl, $SO_2C_{1-4}$Alkyl, $SO_2NHC_{1-4}$Alkyl oder $SO_2$N ($C_{1-4}$Alkyl) ausgewählt sind;
A und B unabhängig für einen aromatischen Ring stehen;
Ar für ein Phenyl, ein substituiertes Phenyl oder Pyridyl steht;
D für O, S, NH oder Methylen steht; und
m und n unabhängig für eine Ganzzahl von 0 bis 4 stehen;
wobei das Verfahren die folgenden Schritte umfasst:

1) Hydrolysieren des Reaktionsproduktes, das erhalten wird, indem die Verbindung der Formel (**XI**) mit $R^{15}$ ($R^{13}$) P (O) CH ($R^8$) $COOR^{14}$ oder $Ar_3P=CR^8CO_2R^{14}$ umgesetzt wird, um die Verbindung der Formel (**IX**) zu erhalten, wobei $R^{13}$ und $R^{15}$ unabhängig für $C_{1-4}$Alkyl, $OC_{1-4}$Alkyl, Phenyl, substituiertes Phenyl, Phenoxy oder substituiertes Phenoxy stehen; $R^{14}$ für $C_{1-4}$Alkyl steht und die Definition von Y gleich ist wie die in Anspruch 1, außer, dass Y nicht für NH steht;

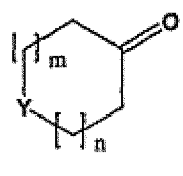
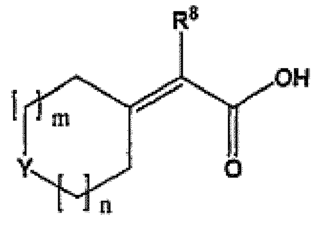
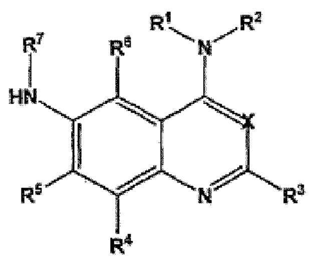

(**XI**) (**IX**) (**X**)

2) Inkontaktbringen der Verbindung der Formel (**X**), eines Acylchlorids oder eines gemischten Anhydrids davon mit der Verbindung der Formel (**IX**) in der Gegenwart eines Kondensationsmittels, um die Verbindung der Formel (**I**) zu erhalten;
3) Transformieren der in Schritt 2) erhaltenen Verbindung der Formel (**I**), wobei Y für N- $R^9$ steht und $R^9$ für $(CH_3)_3$O (C (O) steht, zu der Verbindung der Formel (**XII**) unter sauren Bedingungen, wobei die Definitionen

von $R^1$, $R^2$, $R^3$, $R^4$, $R^5$, $R^6$, $R^7$, $R^8$, $R^{10}$, $R^{11}$, $R^{12}$, A, B, D, X, Y, m und n gleich sind wie die in Anspruch 1;

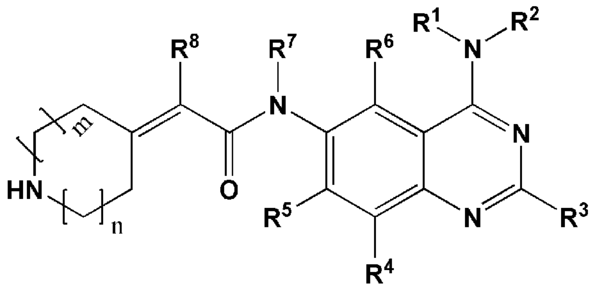

(**XII**)

4) Inkontaktbringen der Verbindung der Formel (**XII**) mit $XR^9$, um die Verbindung der Formel (**I**) zu erhalten, wobei die Definition von $R^9$ gleich ist wie die für Formel (**I**) und X für Cl, Br, I, OMs oder OTs steht.

**16.** Verwendung der Verbindung der Formel (**I**) oder eines pharmazeutisch geeigneten Salzes oder Hydrats davon,

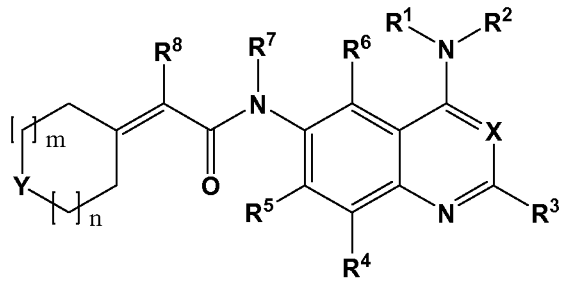

(**I**)

für die Herstellung eines Medikaments für die Behandlung von Psoriasis, Pneumonie, Hepatitis, Nephritis, Pankreatitis, Diabetes, Krebs oder eine beliebige Störung, die durch Hemmen von Proteintyrosinkinaseaktivität bei Säugetieren behandelt werden kann,
wobei
X für N, C- CN oder CH steht;
Y für $CH_2$, S, O oder N- $R^9$ steht;
$R^1$, $R^3$, $R^7$ und $R^8$ unabhängig für H, $CF_3$ oder $C_{1-6}$Alkyl stehen;
$R^2$ für eine Gruppe steht, die aus Formel (**II**), (**III**), (**IV**), (**V**), (**VI**), (**VII**) oder (**VIII**) ausgewählt ist;

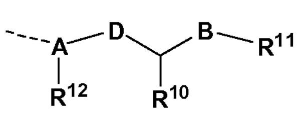

(**II**)

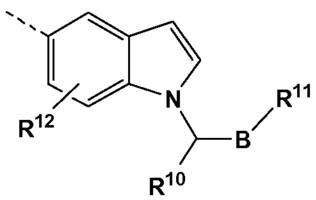

(**III**)

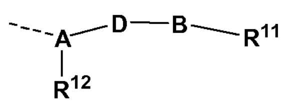

(**IV**)

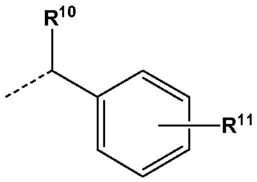
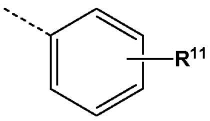
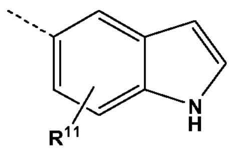
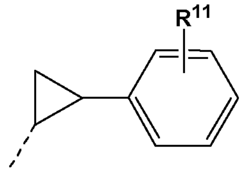

(**V**) (**VI**) (**VII**) (**VIII**)

$R^4$ und $R^6$ unabhängig für H, $C_{1-6}$Alkyl, $OC_{1-6}$Alkyl, OH, F, Cl, Br, $OCF_3$ oder Trifluormethyl stehen;
$R^5$ bei jedem Auftreten unabhängig ausgewählt ist aus H, F, $C_{1-6}$Alkyl, OH, $OC_{1-6}$Alkyl, $OCF_3$, $OCF_2CH_3$, $NH_2$, NH ($C_{1-6}$Alkyl), N ($C_{1-6}$Alkyl)$_2$, 1- Pyrrolinyl, 1- Piperidyl, 4- Morpholinyl, Cl, Br, Trifluormethyl, O $(CH_2)_{2-4}OC_{1-6}$Alkyl, O $(CH_2)_{2-4}OCF_3$, O $(CH_2)_{2-4}$NH ($C_{1-6}$Alkyl), O $(CH_2)_{2-4}$N ($C_{1-6}$Alkyl)$_2$, (1- Pyrrolinyl) $(CH_2)_{2-4}$O, (1- Piperidyl) $(CH_2)_{2-4}$O, (4- Morpholinyl) $(CH_2)_{2-4}$O, NHC (O) H, NHC (O) ($C_{1-6}$Alkyl), N ($C_{1-6}$Alkyl) C (O) ($C_{1-6}$alkyl), O $(CH_2)_{2-4}$OH, N ($C_{1-6}$Alkyl) C (O) O ($C_{1-6}$alkyl), N ($C_{1-6}$Alkyl) C (O) OH, NHC (O) $OC_{1-6}$Alkyl), OC (O) NH ($C_{1-6}$Alkyl), OC (O) N ($C_{1-6}$Alkyl)$_2$, (1- Piperidyl) $(CH_2)_{2-4}$OC (O), (4- Morpholinyl) $(CH_2)_{2-4}$OC (O), (1- Pyrrolinyl) $(CH_2)_{2-4}$OC (O), (1- Imidazolyl) $(CH_2)_{2-4}$O, (Pyrazolyl) $(CH_2)_{2-4}$O, (Triazolyl) $(CH_2)_{2-4}$OC (O) oder Ar $(CH_2)_{1-4}$O;
$R^9$ bei jedem Auftreten unabhängig ausgewählt ist aus H, $C_{1-6}$Alkyl, $CF_3$, $CF_2CH_3$, $(CH_2)_{2-4}$OH, $(CH_2)_{1-4}OC_{1-6}$Alkyl, $(CH_2)_{1-4}$NH ($C_{1-6}$Alkyl), $(CH_2)_{1-4}$N ($C_{1-6}$Alkyl)$_2$, (1- Pyrrolinyl) $(CH_2)_{1-4}$, (1- Piperidyl) $(CH_2)_{1-4}$, (4- Morpholinyl) $(CH_2)_{1-4}$, C (O) $C_{1-6}$Alkyl, C (O) $(CH_2)_{1-4}$OH, C (O) $(CH_2)_{1-4}OC_{1-6}$Alkyl, C (O) $(CH_2)_{1-4}$N ($C_{1-6}$Alkyl)$_2$, (1- Pyrrolinyl) $(CH_2)_{1-6}$C (O), (1- Piperidyl) $(CH_2)_{1-6}$C (O), (4- Morpholinyl) $(CH_2)_{1-4}$C (O), C (O) $OC_{1-6}$Alkyl, C (O) O $(CH_2)_{2-4}OC_{1-6}$Alkyl, C (O) O $(CH_2)_{2-4}$N ($C_{1-6}$Alkyl)$_2$, (1- Pyrrolinyl) $(CH_2)_{2-4}$OC (O), (1- Piperidyl) $(CH_2)_{2-4}$OC (O), (4- Morpholinyl) $(CH_2)_{2-4}$OC (O), $(CH_2)_{1-4}$C (O) $OC_{1-6}$Alkyl oder Ar $(CH_2)_{1-4}$;
$R^{10}$ für H, $C_{1-6}$Alkyl oder ein oder zwei F steht;
$R^{11}$, $R^{12}$ unabhängig für H oder 1 bis 4 gleiche oder unterschiedliche Gruppen stehen, die aus F, Cl, Br, I, CN, $NO_2$, $CF_3$, OH, $NH_2$, $C_{1-4}$Alkyl, $OC_{1-4}$Alkyl, $OCF_3$, $OCF_2CH_3$, NH ($C_{1-4}$Alkyl), N ($C_{1-4}$Alkyl)$_2$, OC (O) $C_{1-4}$Alkyl, NHC (O) H, NHC (O) $C_{1-4}$Alkyl, N ($C_{1-4}$Alkyl) C (O) $C_{1-4}$alkyl, C (O) $OC_{1-4}$Alkyl, C (O) $NHC_{1-4}$Alkyl, C (O) N ($C_{1-4}$Alkyl)$_2$, COOH, C (O) $C_{1-4}$Alkyl, S (O) $C_{1-4}$Alkyl, $SO_2C_{1-4}$Alkyl, $SO_2NHC_{1-4}$Alkyl oder $SO_2$N ($C_{1-4}$Alkyl)$_2$ ausgewählt sind;
A und B unabhängig für einen aromatischen Ring stehen;
Ar für ein Phenyl, ein substituiertes Phenyl oder Pyridyl steht;
D für O, S, NH oder Methylen steht; und
m und n unabhängig für eine Ganzzahl von 0 bis 4 stehen.

## Revendications

**1.** Composé de formule (**I**), ou un sel ou un hydrate pharmaceutiquement acceptable de celui- ci,

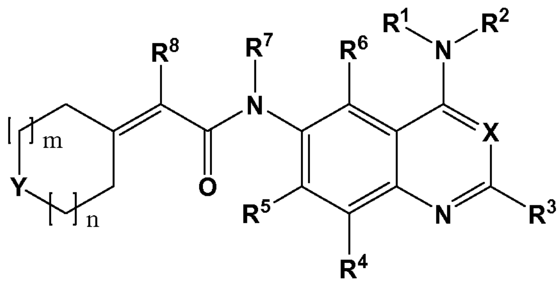

(**I**)

dans laquelle
X représente N, C- CN, ou CH ;
Y représente $CH_2$, S, O, ou N- $R^9$ ;
$R^1$, $R^3$, $R^7$ et $R^8$ représentent indépendamment H, $CF_3$, ou alkyle en $C_{1-6}$ ;
$R^2$ représente un groupe sélectionné parmi la formule (**II**), (**III**), (**IV**), (**V**), (**VI**), (**VII**), ou (**VIII**) ;

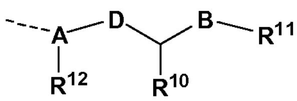

(II)

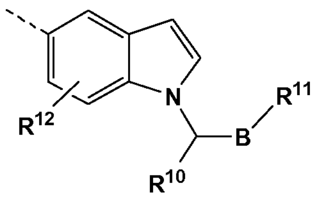

(III)

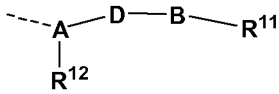

(IV)

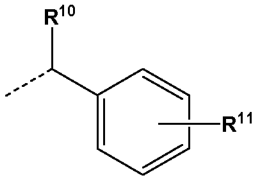

(V)

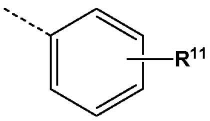

(VI)

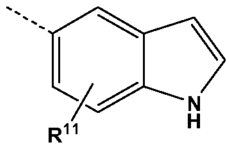

(VII)

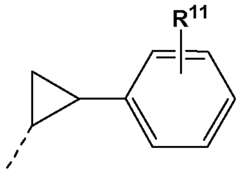

(VIII)

$R^4$ et $R^6$ représentent indépendamment H, alkyle en $C_{1-6}$, O- alkyle en $C_{1-6}$, OH, F, Cl, Br, $OCF_3$, ou trifluorométhyle ;
$R^5$ à chaque occurrence est indépendamment sélectionné parmi H, F, alkyle en $C_{1-6}$, OH, O- alkyle en $C_{1-6}$, $OCF_3$, $OCF_2CH_3$, $NH_2$, NH ($C_{1-6}$alkyle), N ($C_{1-6}$alkyle)$_2$, 1- pyrrolinyle, 1- pipéridyle, 4- morpholinyle, Cl, Br, trifluorométhyle, O ($CH_2$)$_{2-4}$O$C_{1-6}$alkyle, O ($CH_2$)$_{2-4}$$OCF_3$, O ($CH_2$)$_{2-4}$NH ($C_{1-6}$alkyle), O ($CH_2$)$_{2-4}$N ($C_{1-6}$alkyle)$_2$, (1- pyrrolinyle) ($CH_2$)$_{2-4}$O, (1- pipéridyle) ($CH_2$)$_{2-4}$O, (4- morpholinyle) ($CH_2$)$_{2-4}$O, NHC (O) H, NHC (O) ($C_{1-6}$alkyle), N ($C_{1-6}$alkyle) C (O) ($C_{1-6}$alkyle), O ($CH_2$)$_{2-4}$OH, N ($C_{1-6}$alkyle) C (O) O ($C_{1-6}$alkyle), N ($C_{1-6}$alkyle) C (O) OH, NHC (O) O$C_{1-6}$alkyle, OC (O) NH ($C_{1-6}$alkyle), OC (O) N ($C_{1-6}$alkyle)$_2$, (1- pipéridyle) ($CH_2$)$_{2-4}$OC (O), (4- morpholinyle) ($CH_2$)$_{2-4}$OC (O), (1- pyrrolinyle) ($CH_2$)$_{2-4}$OC (O), (1- imidazolyle) ($CH_2$)$_{2-4}$O, (pyrazolyle) ($CH_2$)$_{2-4}$O, (triazolyle) ($CH_2$)$_{2-4}$OC (O), ou Ar ($CH_2$)$_{1-4}$O ;
$R^9$ à chaque occurrence est indépendamment sélectionné parmi H, $C_{1-6}$alkyle, $CF_3$, $CF_2CH_3$, ($CH_2$)$_{2-4}$OH, ($CH_2$)$_{1-4}$O$C_{1-6}$alkyle, ($CH_2$)$_{1-4}$NH ($C_{1-6}$alkyle), ($CH_2$)$_{1-4}$N ($C_{1-6}$alkyle)$_2$, (1- pyrrolinyle) ($CH_2$)$_{1-4}$, (1- pipéridyle) ($CH_2$)$_{1-4}$, (4- morpholinyle) ($CH_2$)$_{1-4}$, C (O) $C_{1-6}$alkyle, C (O) ($CH_2$)$_{1-4}$OH, C (O) ($CH_2$)$_{1-4}$O$C_{1-6}$alkyle, C (O) ($CH_2$)$_{1-4}$N ($C_{1-6}$alkyle)$_2$, (1- pyrrolinyle) ($CH_2$)$_{1-6}$C (O), (1- pipéridyle) ($CH_2$)$_{1-6}$C (O), (4- morpholinyle) ($CH_2$)$_{1-4}$C (O), C (O) O$C_{1-6}$alkyle, C (O) O ($CH_2$)$_{2-4}$O$C_{1-6}$alkyle, C (O) O ($CH_2$)$_{2-4}$N ($C_{1-6}$alkyle)$_2$, (1- pyrrolinyle) ($CH_2$)$_{2-4}$OC (O), (1- pipéridyle) ($CH_2$)$_{2-4}$OC (O), (4- morpholinyle) ($CH_2$)$_{2-4}$OC (O), ($CH_2$)$_{1-4}$ (O) O$C_{1-6}$alkyle, ou Ar ($CH_2$)$_{1-4}$;
$R^{10}$ représente H, alkyle en $C_{1-6}$, ou un ou deux F ;
$R^{11}$, $R^{12}$ représentent indépendamment H, ou 1 à 4 groupes identiques ou différents sélectionnés parmi F, Cl, Br, I, CN, $NO_2$, $CF_3$, OH, $NH_2$, alkyle en $C_{1-4}$, O- alkyle en $C_{1-4}$, $OCF_3$, $OCF_2CH_3$, NH ($C_{1-4}$alkyle), N ($C_{1-4}$alkyle)$_2$, OC (O) $C_{1-4}$alkyle, NHC (O) H, NHC (O) $C_{1-4}$alkyle, N ($C_{1-4}$alkyle) C (O) $C_{1-4}$alkyle, C (O) O$C_{1-4}$alkyle, C (O) NH$C_{1-4}$alkyle, C (O) N ($C_{1-4}$alkyle)$_2$, COOH, C (O) $C_{1-4}$alkyle, S (O) $C_{1-4}$alkyle, $SO_2C_{1-4}$alkyle, $SO_2NHC_{1-4}$alkyle, ou $SO_2$N ($C_{1-4}$alkyle)$_2$ ;
A et B représentent indépendamment un noyau aromatique ;
Ar est un phényle, un phényle substitué ou un pyridyle ;
D représente O, S, NH, ou méthylène ; et
m et n représentent indépendamment un entier de 0 à 4.

2. Composé de formule (**I**) selon la revendication 1, dans laquelle $R^1$, $R^3$, $R^4$, $R^6$ et $R^7$ représentent indépendamment H ; et $R^8$ représente H, $CH_3$, ou $CH_2CH_3$.

3. Composé de formule (**I**) selon la revendication 1, dans laquelle $R^5$ à chaque occurrence est sélectionné indépendamment parmi H, F, $OCF_3$, O$C_{1-4}$alkyle, O ($CH_2$)$_{2-3}$O$C_{1-4}$alkyle, O ($CH_2$)$_{2-3}$N ($C_{1-4}$alkyle)$_2$, 2- (1- pyrrolinyl) éthoxyle, 2- (1- pipéridyl) éthoxyle, 2- (4- morpholinyl) éthoxyle, 3- (1- pyrrolinyle) propyloxyle, 3- (1- pipéridyle) propyloxyle, 3- (4- morpholinyle) propyloxyle, Ph ($CH_2$)$_{1-4}$O, ou Py ($CH_2$)$_{1-4}$O.

4. Composé de formule (**I**) selon la revendication 1, dans laquelle Y représente N- $R^9$, et $R^9$ à chaque occurrence est indépendamment sélectionné parmi H, $CF_3$, $C_{1-6}$alkyle, ($CH_2$)$_{2-4}$OH, ($CH_2$)$_{2-4}$O$C_{1-6}$alkyle, ($CH_2$)$_{2-4}$N ($C_{1-6}$alkyle)$_2$, (1- pyrrolinyle)- ($CH_2$)$_{2-4}$, (1- pipéridyle)- ($CH_2$)$_{2-4}$, (4- morpholinyle)- ($CH_2$)$_{2-4}$, C (O) O$C_{1-6}$alkyle, ($CH_2$)$_{1-4}$ (O) O$C_{1-6}$alkyle, Ph ($CH_2$)$_{1-4}$, ou Py ($CH_2$)$_{1-4}$.

**5.** Composé de formule (**I**) selon la revendication 1, dans laquelle m et n représentent indépendamment 0, 1 ou 2.

**6.** Composé de formule (**I**) selon la revendication 1, dans laquelle ledit composé inhibe l'activité de la protéine tyrosine kinase.

**7.** Composé de formule (**I**) selon la revendication 1, dans laquelle ledit composé inhibe la croissance des cellules cancéreuses.

**8.** Composé de formule (**I**) selon la revendication 6, dans laquelle ladite protéine tyrosine kinase représente la tyrosine kinase EGF$\alpha$ ou la tyrosine kinase Her-2.

**9.** Composé de formule (**I**) selon la revendication 7, dans laquelle dans lesdites cellules cancéreuses EGFR est surexprimée ou Her-2 est surexprimée.

**10.** Proéparation comprenant au moins un composé de formule (**I**), ou un sel ou un hydrate pharmaceutiquement acceptable de celui- ci, dans laquelle ledit composé ou ledit sel ou hydrate pharmaceutiquement acceptable de celui- ci est utilisé comme ingrédient actif médical et traité avec les excipients requis et pharmaceutiquement acceptables en une forme de dosage appropriée, et ledit composé ou ledit sel ou hydrate pharmaceutiquement acceptable de celui- ci utilisé comme ingrédient actif médical peut traiter ou prévenir des maladies par administration orale, injection intraveineuse, administration intrapéritonéale, injection sous- cutanée, injection intramusculaire, goutte- à- goutte nasal, administration intraoculaire, inhalation, administration anale, administration vaginale, ou administration épidermique,

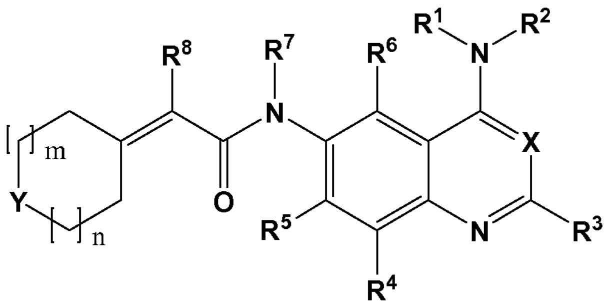

(**I**)

dans laquelle
X représente N, C- CN, ou CH ;
Y représente $CH_2$, S, O, ou N- $R^9$ ;
$R^1$, $R^3$, $R^7$ et $R^8$ représentent indépendamment H, $CF_3$, alkyle en $C_{1-6}$ ;
$R^2$ représente un groupe sélectionné parmi : la formule (**II**), (**III**), (**IV**), (**V**), (**VI**), (**VII**), ou (**VIII**) ;

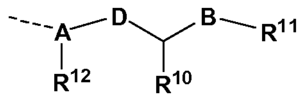

(**II**)

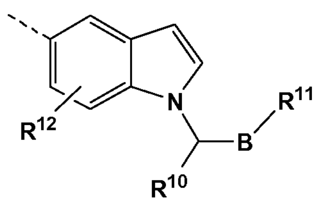

(**III**)

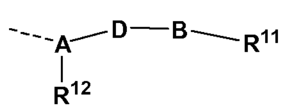

(**IV**)

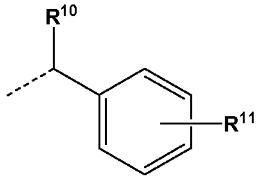 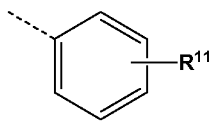 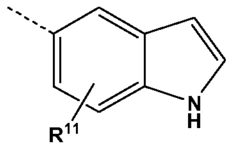 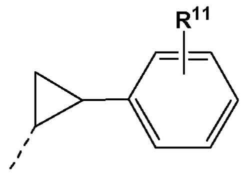

(V) (VI) (VII) (VIII)

$R^4$ et $R^6$ représentent indépendamment H, alkyle en $C_{1-6}$, O- alkyle en $C_{1-6}$, OH, F, Cl, Br, $OCF_3$, ou trifluorométhyle ;
$R^5$ à chaque occurrence est indépendamment sélectionné parmi H, F, alkyle en $C_{1-6}$, OH, O- alkyle en $C_{1-6}$, $OCF_3$, $OCF_2CH_3$, $NH_2$, NH ($C_{1-6}$alkyle), N ($C_{1-6}$alkyle)$_2$, 1- pyrrolinyle, 1- pipéridyle, 4- morpholinyle, Cl, Br, trifluorométhyle, O $(CH_2)_{2-4}OC_{1-6}$alkyle, O $(CH_2)_{2-4}OCF_3$, O $(CH_2)_{2-4}$NH ($C_{1-6}$alkyle), O $(CH_2)_{2-4}$N ($C_{1-6}$alkyle)$_2$, (1- pyrrolinyle) $(CH_2)_{2-4}$O, (1- pipéridyle) $(CH_2)_{2-4}$O, (4- morpholinyle) $(CH_2)_{2-4}$O, NHC (O) H, NHC (O) ($C_{1-6}$alkyle), N ($C_{1-6}$alkyle) C (O) ($C_{1-6}$alkyle), O $(CH_2)_{2-4}$OH, N ($C_{1-6}$alkyle) C (O) O ($C_{1-6}$alkyle), N ($C_{1-6}$alkyle) C (O) OH, NHC (O) $OC_{1-6}$alkyle, OC (O) NH ($C_{1-6}$alkyle), OC (O) N ($C_{1-6}$alkyle)$_2$, (1- pipéridyle) $(CH_2)_{2-4}$OC (O), (4- morpholinyle) $(CH_2)_{2-4}$OC (O), (1- pyrrolinyle) $(CH_2)_{2-4}$OC (O), (1- imidazolyle) $(CH_2)_{2-4}$O, (pyrazolyle) $(CH_2)_{2-4}$O, (triazolyle) $(CH_2)_{2-4}$OC (O), ou Ar $(CH_2)_{1-4}$) O ;
$R^9$ à chaque occurrence est indépendamment sélectionné parmi H, $C_{1-6}$alkyle, $CF_3$, $CF_2CH_3$, $(CH_2)_{2-4}$OH, $(CH_2)_{1-4}OC_{1-6}$alkyle, $(CH_2)_{1-4}$NH ($C_{1-6}$alkyle), $(CH_2)_{1-4}$N ($C_{1-6}$alkyle)$_2$, (1- pyrrolinyle) $(CH_2)_{1-4}$, (1- pipéridyle) $(CH_2)_{1-4}$, (4- morpholinyle) $(CH_2)_{1-4}$, C (O) $C_{1-6}$alkyle, C (O) $(CH_2)_{1-4}$OH, C (O) $(CH_2)_{1-4}OC_{1-6}$alkyle, C (O) $(CH_2)_{1-4}$N ($C_{1-6}$alkyle)$_2$, (1- pyrrolinyle) $(CH_2)_{1-6}$C (O), (1- pipéridyle) $(CH_2)_{1-6}$C (O), (4- morpholinyle) $(CH_2)_{1-4}$C (O), C (O) $OC_{1-6}$alkyle, C (O) O $(CH_2)_{2-4}OC_{1-6}$alkyle, C (O) O $(CH_2)_{2-4}$N ($C_{1-6}$alkyle)$_2$, (1- pyrrolinyle) $(CH_2)_{2-4}$OC (O), (1- pipéridyle) $(CH_2)_{2-4}$OC (O), (4- morpholinyle) $(CH_2)_{2-4}$OC (O), $(CH_2)_{1-4}$C (O) $OC_{1-6}$alkyle, ou Ar $(CH_2)_{1-4}$;
$R^{10}$ représente H, alkyle en $C_{1-6}$, ou un ou deux F ;
$R^{11}$ et $R^{12}$ représentent indépendamment H, ou 1 à 4 groupes identiques ou différents sélectionnés parmi F, Cl, Br, I, CN, $NO_2$, $CF_3$, OH, $NH_2$, $C_{1-4}$alkyle, $OC_{1-4}$alkyle, $OCF_3$, $OCF_2CH_3$, NH ($C_{1-4}$alkyle), N ($C_{1-4}$alkyle)$_2$, OC (O) $C_{1-4}$alkyle, NHC (O) H, NHC (O) $C_{1-4}$alkyle, N ($C_{1-4}$alkyle) C (O) $C_{1-4}$alkyle, C (O) $OC_{1-4}$alkyle, C (O) $NHC_{1-4}$alkyle, C (O) N ($C_{1-4}$alkyle), COOH, C (O) $C_{1-4}$alkyle, S (O) $C_{1-4}$alkyle, $SO_2C_{1-4}$alkyle, $SO_2NHC_{1-4}$alkyle, ou $SO_2$N ($C_{1-4}$alkyle) ;
A et B représentent indépendamment un noyau aromatique ;
Ar est un phényle, un phényle substitué, ou un pyridyle ;
D représente O, S, NH, ou méthylène ; et
m et n représentent indépendamment un entier de 0 à 4.

**11.** Préparation selon la revendication 10 comprenant au moins un composé de formule (**I**), ou un sel ou un hydrate pharmaceutiquement acceptable de celui-ci, dans laquelle ladite préparation peut être utilisée pour traiter des cancers.

**12.** Préparation selon la revendication 11, dans laquelle lesdits cancers comprennent le cancer du sein, le cancer du rein, le cancer de la vessie, le cancer de la bouche, le cancer du larynx, le cancer de l'oesophage, le cancer de l'estomac, le cancer colorectal, le cancer de l'ovaire, le cancer du poumon et le cancer de la tête et du cou.

**13.** Préparation selon la revendication 10 comprenant au moins un composé de formule (**I**), ou des sels ou des hydrates pharmaceutiquement acceptables de celui-ci, dans laquelle ladite préparation peut être utilisée pour traiter ou prévenir un trouble physiologique provoqué par l'inhibition de l'activité de la protéine tyrosine kinase chez des mammifères.

**14.** Préparation selon la revendication 13, dans laquelle ledit trouble physiologique comprend le psoriasis, la pneumonie, l'hépatite, la néphrite, la pancréatite, le diabète, ou tout type de cancers.

**15.** Procédé de préparation d'un composé selon la formule (**I**) selon la revendication 1 ou la revendication 10,

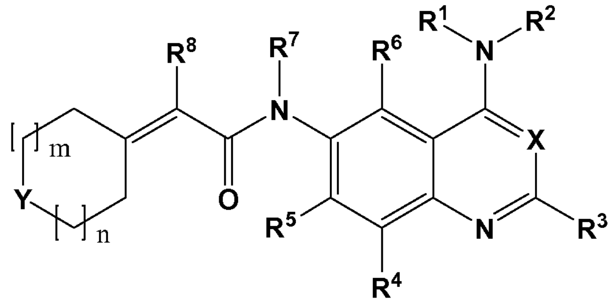

(I)

dans laquelle
X représente N, C- CN, ou CH ;
Y représente $CH_2$, S, O, ou N- $R^9$ ;
$R^1$, $R^3$, $R^7$ et $R^8$ représentent indépendamment H, $CF_3$, alkyle en $C_{1-6}$ ;
$R^2$ représente un groupe sélectionné parmi : la formule (**II**), (**III**), (**IV**), (**V**), (**VI**), (**VII**), ou (**VIII**) ;

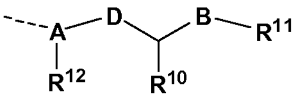 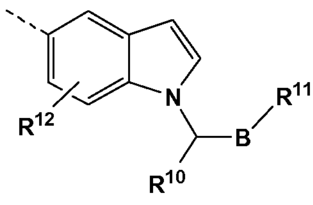 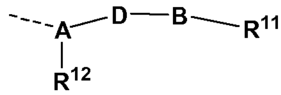

(**II**) (**III**) (**IV**)

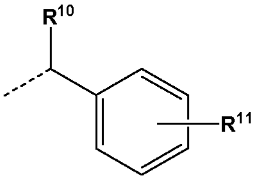 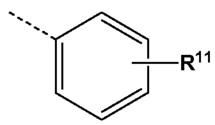 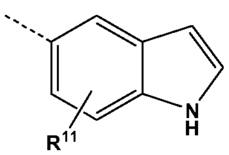 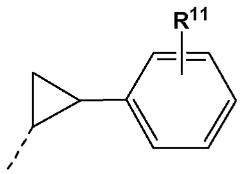

(**V**) (**VI**) (**VII**) (**VIII**)

$R^4$ et $R^6$ représentent indépendamment H, alkyle en $C_{1-6}$, O- alkyle en $C_{1-6}$, OH, F, Cl, Br, $OCF_3$ ou trifluorométhyle ;
$R^5$ à chaque occurrence est indépendamment sélectionné parmi H, F, alkyle en $C_{1-6}$, OH, O- alkyl en $C_{1-6}$, $OCF_3$, $OCF_2CH_3$, $NH_2$, NH ($C_{1-6}$alkyle), N ($C_{1-6}$alkyle)$_2$, 1- pyrrolinyle, 1- pipéridyle, 4- morpholine, Cl, Br, trifluorométhyle, O $(CH_2)_{2-4}OC_{1-6}$alkyle, O $(CH_2)_{2-4}OCF_3$, O $(CH_2)_{2-4}$NH ($C_{1-6}$alkyle), O $(CH_2)_{2-4}$N ($C_{1-6}$alkyle)$_2$, (1- pyrrolinyle) $(CH_2)_{2-4}$O, (1- pipéridyle) $(CH_2)_{2-4}$O, (4- morpholinyle) $(CH_2)_{2-4}$O, NHC (O) H, NHC (O) ($C_{1-6}$alkyle), N ($C_{1-6}$alkyle) C (O) ($C_{1-6}$alkyle), O $(CH_2)_{2-4}$OH, N ($C_{1-6}$alkyle) C (O) O ($C_{1-6}$alkyle), N ($C_{1-6}$alkyle) C (O) OH, NHC (O) O) $C_{1-6}$alkyle), OC (O) NH ($C_{1-6}$alkyle), OC (O) N ($C_{1-6}$alkyle)$_2$, (1- pipéridyle) $(CH_2)_{2-4}$OC (O), (4- morpholinyle) $(CH_2)_{2-4}$OC (O), (1- pyrrolinyle) $(CH_2)_{2-4}$OC (O), (1- imidazolyle) $(CH_2)_{2-4}$O, (pyrazolyle) $(CH_2)_{2-4}$O, (triazolyle) $(CH_2)_{2-4}$OC (O), ou Ar $(CH_2)_{1-4}$) O ;
$R^9$ à chaque occurrence est indépendamment sélectionné parmi H, $C_{1-6}$alkyle, $CF_3$, $CF_2CH_3$, $(CH_2)_{2-4}OH$, $(CH_2)_{1-4}OC_{1-6}$alkyle, $(CH_2)_{1-4}$NH ($C_{1-6}$alkyle), $(CH_2)_{1-4}$N ($C_{1-6}$alkyle)$_2$, (1- pyrrolinyle) $(CH_2)_{1-4}$, (1- pipéridyle) $(CH_2)_{1-4}$, (4- morpholinyle) $(CH_2)_{1-4}$, C (O) $C_{1-6}$alkyle, C (O) $(CH_2)_{1-4}$OH, C (O) $(CH_2)_{1-4}OC_{1-6}$alkyle, C (O) $(CH_2)_{1-4}$N ($C_{1-6}$alkyle)$_2$, (1- pyrrolinyle) $(CH_2)_{1-6}$C (O), (1- pipéridyle) $(CH_2)_{1-6}$C (O), (4- morpholinyle) $(CH_2)_{1-4}$C (O), C (O) $OC_{1-6}$alkyle, C (O) O $(CH_2)_{2-4}OC_{1-6}$alkyle, C (O) O $(CH_2)_{2-4}$N ($C_{1-6}$alkyle)$_2$, (1- pyrrolinyle) $(CH_2)_{2-4}$OC (O), (1-pipéridyle) $(CH_2)_{2-4}$OC (O), (4- morpholinyle) $(CH_2)_{2-4}$OC (O), $(CH_2)_{1-4}$C (O) $OC_{1-6}$alkyle, ou Ar $(CH_2)_{1-4}$;
$R^{10}$ représente H, alkyle en $C_{1-6}$, ou un ou deux F ;
$R^{11}$ et $R^{12}$ représentent indépendamment H, ou 1 à 4 groupes identiques ou différents sélectionnés parmi F, Cl, Br, I, CN, $NO_2$, $CF_3$, OH, $NH_2$, $C_{1-4}$alkyle, $OC_{1-4}$alkyle, $OCF_3$, $OCF_2CH_3$, NH ($C_{1-4}$alkyle), N ($C_{1-4}$alkyle)$_2$, OC (O) $C_{1-4}$alkyle, NHC (O) H, NHC (O) $C_{1-4}$alkyle, N ($C_{1-4}$alkyle) C (O) $C_{1-4}$alkyle, C (O) $OC_{1-4}$alkyle, C (O) $NHC_{1-4}$alkyle,

C (O) N ($C_{1-4}$alkyle), COOH, C (O) $C_{1-4}$alkyle, S (O) $C_{1-4}$alkyle, $SO_2C_{1-4}$alkyle, $SO_2NHC_{1-4}$alkyle, ou $SO_2N$ ($C_{1-4}$alkyle) ;
A et B représentent indépendamment un noyau aromatique ;
Ar est un phényle, un phényle substitué, ou un pyridyle ;
D représente O, S, NH, ou méthylène ; et
m et n représentent indépendamment un entier de 0 à 4 ;
le procédé comprenant les étapes consistant à :

1) hydrolyser le produit de réaction obtenu en mettant en contact le composé de formule (**XI**) avec $R^{15}$ ($R^{13}$) P (O) CH ($R^8$) $COOR^{14}$ ou $Ar_3P=CR^8CO_2R^{14}$ pour obtenir le composé de formule (**IX**), dans laquelle $R^{13}$ et $R^{15}$ représentent indépendamment alkyle en $C_{1-4}$, O- alkyle en $C_{1-4}$, phényle, phényle substitué, phénoxy, ou phénoxy substitué ; $R^{14}$ représente alkyle en $C_{1-4}$, et la définition d'Y est la même que celle de la revendication 1 sauf qu'Y ne représente pas NH ;

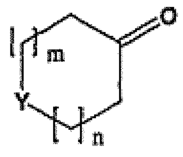

(**XI**)

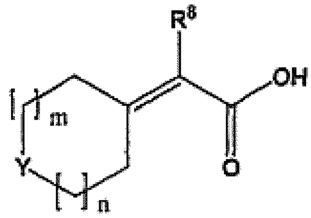

(**IX**)

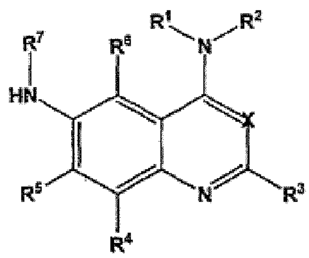

(**X**)

2) mettre en contact le composé de formule (**X**), un chlorure d'acyle, ou un anhydride mélangé de celui-ci avec le composé de formule (**IX**) en présence d'un agent de condensation pour obtenir le composé de formule (**I**) ;
3) tranformer le composé de formule (**I**) obtenu à l'étape 2) dans laquelle Y représente N- $R^9$ et $R^9$ représente $(CH_3)_3O$ (C (O) en le composé de formule (**XII**) dans des conditions acides, dans laquelle les définitions de $R^1$, $R^2$, $R^3$, $R^4$, $R^5$, $R^6$, $R^7$, $R^8$, $R^{10}$, $R^{11}$, $R^{12}$, A, B, D, X, Y, m et n sont les mêmes que celles dans la revendication 1 ;

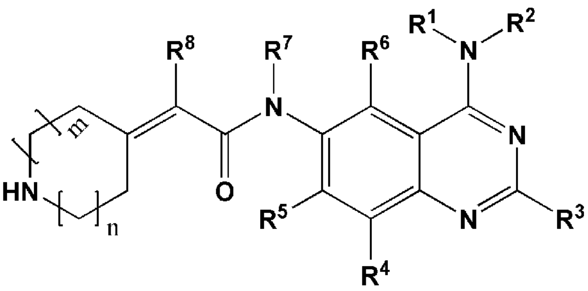

(**XII**)

4) mettre en contact le composé de formule (**XII**) avec $XR^9$ pour obtenir le composé de formule (**I**), dans laquelle la définition de $R^9$ est la même que celle pour la formule (**I**), et X représente Cl, Br, I, OMs, ou OTs.

**16.** Utilisation d'un composé de formule (**I**), ou d'un sel ou d'un hydrate pharmaceutiquement acceptable de celui- ci,

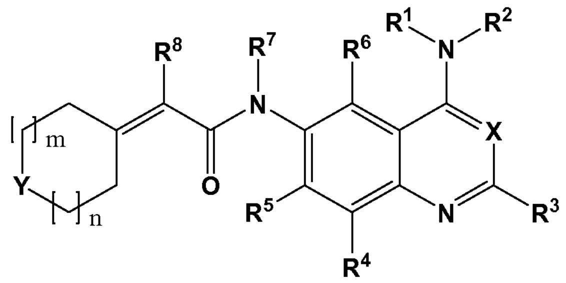

(**I**)

pour la fabrication d'un médicament pour le traitement du psoriasis, de la pneumonie, de l'hépatite, de la néphrite, de la pancréatite, du diabète, du cancer, ou de tout trouble qui peut être traité en inhibant l'activité de la protéine tyrosine kinase chez des mammifères,

dans laquelle

X représente N, C- CN, ou CH ;

Y représente $CH_2$, S, O, ou N- $R^9$ ;

$R^1$, $R^3$, $R^7$ et $R^8$ représentent indépendamment H, $CF_3$, ou alkyle en $C_{1-6}$ ;

$R^2$ représente un groupe sélectionné parmi la formule (**II**), (**III**), (**IV**), (**V**), (**VI**), (**VII**), ou (**VIII**) ;

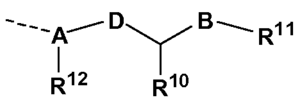

(**II**)

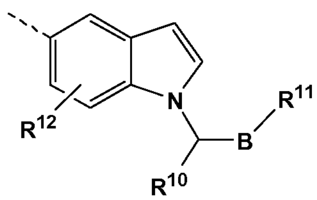

(**III**)

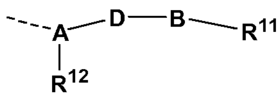

(**IV**)

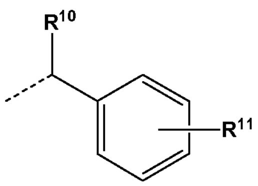

(**V**)

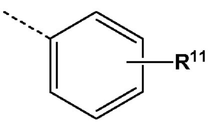

(**VI**)

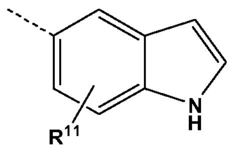

(**VII**)

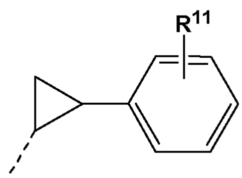

(**VIII**)

$R^4$ et $R^6$ représentent indépendamment H, alkyle en $C_{1-6}$, O- alkyle en $C_{1-6}$, OH, F, Cl, Br, $OCF_3$, ou trifluorométhyle ;

$R^5$ à chaque occurrence est indépendamment sélectionné parmi H, F, alkyle en $C_{1-6}$, OH, O- alkyle en $C_{1-6}$, $OCF_3$, $OCF_2CH_3$, $NH_2$, NH ($C_{1-6}$alkyle), N ($C_{1-6}$alkyle)$_2$, 1- pyrrolinyle, 1- pipéridyle, 4- morpholinyle, Cl, Br, trifluorométhyle, O $(CH_2)_{2-4O}C_{1-6}$alkyle, O $(CH_2)_{2-4}OCF_3$, O $(CH_2)_{2-4}$NH ($C_{1-6}$alkyle), O $(CH_2)_{2-4}$N ($C_{1-6}$alkyle)$_2$, (1- pyrrolinyle) $(CH_2)_{2-4}$O, (1- pipéridyle) $(CH_2)_{2-4}$O, (4- morpholinyle) $(CH_2)_{2-4}$O, NHC (O) H, NHC (O) ($C_{1-6}$alkyle), N ($C_{1-6}$alkyle) C (O) ($C_{1-6}$alkyle), O $(CH_2)_{2-4}$OH, N ($C_{1-6}$alkyle) C (O) O ($C_{1-6}$alkyle), N ($C_{1-6}$alkyle) C (O) OH, NHC (O) $OC_{1-6}$alkyle), OC (O) NH ($C_{1-6}$alkyle), OC (O) N ($C_{1-6}$alkyle)$_2$, (1- pipéridyle) $(CH_2)_{2-4}$OC (O), (4- morpholinyle) $(CH_2)_{2-4}$OC (O), (1- pyrrolinyle) $(CH_2)_{2-4}$OC (O), (1- imidazolyle) $(CH_2)_{2-4}$O, (pyrazolyle) $(CH_2)_2$O, (triazolyle) $(CH_2)_{2-4}$OC (O), ou Ar $(CH_2)_{1-4}$O;

$R^9$ à chaque occurrence est indépendamment sélectionné parmi H, $C_{1-6}$alkyle, $CF_3$, $CF_2CH_3$, $(CH_2)_{2-4}$OH, $(CH_2)_{1-4}OC_{1-6}$alkyle, $(CH_2)_{1-4}$NH ($C_{1-6}$alkyle), $(CH_2)_{1-4}$N ($C_{1-6}$alkyle)$_2$, (1- pyrrolinyle) $(CH_2)_{1-4}$, (1- pipéridyle) $(CH_2)_{1-4}$, (4- morpholinyle) $(CH_2)_{1-4}$, C (O) $C_{1-6}$alkyle, C (O) $(CH_2)_{1-4}$OH, C (O) $(CH_2)_{1-4}OC_{1-6}$alkyle, C (O) $(CH_2)_{1-4}$N ($C_{1-6}$alkyle)$_2$, (1- pyrrolinyle) $(CH_2)_{1-6}$C (O), (1- pipéridyle) $(CH_2)_{1-6}$C (O), (4- morpholinyle) $(CH_2)_{1-4}$C (O), C (O) $OC_{1-6}$alkyle, C (O) O $(CH_2)_{2-4}OC_{1-6}$alkyle, C (O) O $(CH_2)_{2-4}$N ($C_{1-6}$alkyle)$_2$, (1- pyrrolinyle) $(CH_2)_{2-4}$OC (O), (1- pipéridyle) $(CH_2)_{2-4}$OC (O), (4- morpholinyle) $(CH_2)_{2-4}$OC (O), $(CH_2)_{1-4}$ (O) $OC_{1-6}$alkyle, ou Ar $(CH_2)_{1-4}$;

$R^{10}$ représente H, alkyle en $C_{1-6}$, ou un ou deux F ;

$R^{11}$, $R^{12}$ représentent indépendamment H, ou 1 à 4 groupes identiques ou différents sélectionnés parmi F, Cl, Br, I, CN, $NO_2$, $CF_3$, OH, $NH_2$, alkyle en $C_{1-4}$, O- alkyle en $C_{1-4}$, $OCF_3$, $OCF_2CH_3$, NH ($C_{1-4}$alkyle), N ($C_{1-4}$alkyle)$_2$, OC (O) $C_{1-4}$alkyle, NHC (O) H, NHC (O) $C_{1-4}$alkyle, N ($C_{1-4}$alkyle) C (O) $C_{1-4}$alkyle, C (O) O$C_{1-4}$alkyle, C (O) NH$C_{1-4}$alkyle, C (O) N ($C_{1-4}$alkyle)$_2$, COOH, C (O) $C_{1-4}$alkyle, S (O) $C_{1-4}$alkyle, $SO_2C_{1-4}$alkyle, $SO_2NHC_{1-4}$alkyle, ou $SO_2N$ ($C_{1-4}$alkyle)$_2$ ;
A et B représentent indépendamment un noyau aromatique ;
Ar est un phényle, un phényle substitué, ou un pyridyle ;
D représente O, S, NH, ou méthylène ; et
m et n représentent indépendamment un entier de 0 à 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 92078844 A
- WO 9214716 A
- WO 9220642 A
- EP 520722 A1
- EP 566226 A1
- EP 635498 A1
- EP 602851 A1
- WO 9519774 A
- WO 9515758 A
- US 5760041 A
- EP 0787722 A


### Non-patent literature cited in the description


- **ROYCHAUDHURY et al.** *Pathology*
- **KORC et al.** *Gut,* 1994, vol. 35, 1468
- **FRIESS et al.** *Ann.Surg.,* 1994, vol. 220, 183
- *Taga Nippon Sanka Fujinka Gakkai Zasshi,* 1992, vol. 44, 939
- **BROWN et al.** *Endocrinology,* 1989, vol. 124, 2882
- **DAS et al.** *Development,* 1994, vol. 120, 1071
- **ADAMSON.** *Mol. reprod. Dev.,* 1990, vol. 27, 16
- **TSUTSUMI et al.** *J.Endocrinlogy,* 1993, vol. 138, 437
- **JUDIT ANIDO.** *Clinical Cancer Research,* 2003, vol. 9, 1274-1283